(19) Europäisches Patentamt
European Patent Office
Office européen des brevets

(11) EP 0 750 508 B1

(12) FASCICULE DE BREVET EUROPEEN

(45) Date de publication et mention de la délivrance du brevet: **16.04.2003 Bulletin 2003/16**

(21) Numéro de dépôt: **95912306.8**

(22) Date de dépôt: **13.03.1995**

(51) Int Cl.[7]: **A61K 38/17**, A61K 39/395, C07K 16/18, C07K 16/42, G01N 33/564, G01N 33/577, G01N 33/68, C07K 5/02, C07K 7/02 // C07K14/47

(86) Numéro de dépôt international: **PCT/FR95/00292**

(87) Numéro de publication internationale: **WO 95/024916 (21.09.1995 Gazette 1995/40)**

# (54) RETROPEPTIDES, ANTICORPS DIRIGES CONTRE CES DERNIERS, ET LEURS UTILISATIONS POUR LA VACCINATION ET LE DIAGNOSTIC $i(IN VITRO)

RETROPEPTIDE, ANTIKÖRPER DAGEGEN UND DEREN VERWENDUNGEN IN DER IMPFUNG UND IN VITRO DIAGNOSTIK

RETROPEPTIDES, ANTIBODIES THERETO, AND USES THEREOF FOR VACCINATION AND $i(IN VITRO) DIAGNOSIS

(84) Etats contractants désignés: **AT BE CH DE DK ES FR GB GR IE IT LI LU MC NL PT SE**

(30) Priorité: **14.03.1994 FR 9402950**

(43) Date de publication de la demande: **02.01.1997 Bulletin 1997/01**

(73) Titulaire: **BIOMERIEUX 69280 Marcy-L'Etoile (FR)**

(72) Inventeurs:
- **GUICHARD, Gilles 67000 Strasbourg (FR)**
- **MULLER, Sylviane 67000 Strasbourg (FR)**
- **BRIAND, Jean-Paul 67000 Strasbourg (FR)**
- **REGENMORTEL, Marc 67000 Strasbourg (FR)**

(74) Mandataire: **Grosset-Fournier, Chantal Catherine et al Grosset-Fournier & Demachy, 54,rue Saint-Lazare 75009 Paris (FR)**

(56) Documents cités:
**WO-A-90/15813**

- **THE JOURNAL OF BIOLOGICAL CHEMISTRY, vol. 268, no. 35, 15 Décembre 1993 BALTIMORE MD, TATS UNIS, pages 26279-26285, N. BENKIRANE ET AL. 'Antigenicity and immunogenicity of modified synthetic peptides containing D-amino acid residues. Antibodies to a D-enantiomer do recognize the parent L-hexapeptide and reciprocally.' cité dans la demande**
- **MOLECULAR IMMUNOLOGY, vol. 30, no. 6, Avril 1993 OXFORD, GRANDE BRETAGNE, pages 539-547, M. FRIEDE ET AL. 'Induction of immune response against a short synthetic peptide antigen coupled to small neutral liposome containing monophosphoryl lipid A.' cité dans la demande**
- **PROCEEDINGS OF THE NATIONAL ACADEMY OF SCIENCES OF THE USA, vol. 91, no. 21, Octobre 1994 WASHINGTON DC, TATS UNIS, pages 9765-9769, G. GUICHARD ET AL. 'Antigenic mimicry of natural L-peptides with retro-inverso-peptidomimetics.'**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 750 508 B1

## Description

La présente invention a pour objet des rétropeptides, ainsi que des anticorps dirigés contre ces derniers, et leurs utilisations, principalement dans le domaine de la préparation de compositions pharmaceutiques, notamment de vaccins, et pour le diagnostic *in vitro* de diverses pathologies.

Le développement des neuropeptides, des hormones peptidiques et des antibiotiques à base de peptides, ou des vaccins synthétiques à base de peptides, est fortement perturbé par la grande sensibilité des peptides vis à vis de la protéolyse qui limite, entre autres, l'administration orale et parentérale.

Pendant de nombreuses années, on s'est attaché à la synthèse d'analogues peptidiques, afin de rechercher des peptides mimant les peptides ou protéines naturels et présentant une activité et une demie vie biologique accrues par rapport à ces derniers. A titre d'exemple, des analogues peptidiques ont été obtenus par remplacement des acides aminés L du peptide naturel par les acides aminés D correspondants, ou par des résidus non naturels (par exemple sarcosine et β-alanine), ou encore par modification des liaisons peptidiques du peptide naturel (Chorev, M. & Goodman, M. (1993), Acc. Chem. Res. **26,** 266-273; Marraud et al., (1993), Biopolymers, **33**, 1135-1148).

Ces modifications donnent des pseudopeptides ou des peptides mimant les peptides ou protéines naturels (on parlera encore de peptidomimétiques) avec une stabilité métabolique supérieure à celle de ces derniers, puisque la plupart des protéases naturelles ne peuvent pas cliver les aminoacides D, et les liaisons non peptidiques.

Le problème majeur que l'on rencontre avec de tels pseudopeptides, est celui de la conservation de leur activité biologique par rapport à celle du peptide naturel, ou de la protéine naturelle, qu'ils sont supposés mimer.

Récemment, la forme D de la protéase HIV-1 a été synthétisée (De L. Milton et al., (1992), Science, **256**, 1445-1448). Comme on pouvait s'y attendre, la protéine énantiomère montrait une spécificité chirale réciproque de sorte que l'enzyme était incapable de cliver le substrat normal L, mais hydrolysait son énantiomère D.

Au contraire, Wen et Laursen (Wen, D. & Laursen, R.A., (1993), FEBS Lett., **317,** 31-34) ont montré qu'à la fois la forme D et L d'un polypeptide anti-givrant α-hélicoïdal se lient aussi bien au même substrat de glace achirale, tandis que Wade et al. (Wade et al., (1990), Proc. Natl. Acad. Sci., USA, **87**, 4761-4765) ont trouvé que les énantiomères L et D de plusieurs antibiotiques formant des canaux étaient tout aussi actifs l'un que l'autre.

Les peptides modifiés pourraient être utiles comme vaccins potentiels synthétiques s'ils pouvaient induire la formation d'anticorps qui reconnaissent les structures antigéniques non modifiées du pathogène correspondant et neutralisent son caractère infectieux.

Toutefois, en comparaison avec le travail considérable effectué jusqu'à ce jour dans le domaine de la production d'anticorps dirigés contre des protéines naturelles, ou contre des peptides synthétiques issus de ces dernières, et de l'étude des réactions croisées entre de tels anticorps et ces protéines ou peptides naturels, on en connaît peu sur la réponse immunitaire contre des analogues peptidiques, en particulier contre les peptides D et les peptides contenant des liaisons modifiées.

Plusieurs auteurs ont affirmé que les pseudopeptides possèdent probablement très peu ou pas d'immunogénicité, puisqu'ils ne pouvaient pas être transformés et présentés aux molécules du complexe majeur d'histocompatibilité (CMH) pour être reconnus par les cellules T auxiliaires ou par les lymphocytes T cytotoxiques. C'est ainsi que Dintzis et al. (8) ont récemment décrit que l'énantiomère L de la rubrexodine induit une forte réponse immunitaire par production d'immunoglobulines d'isotype G (IgG), tandis que la protéine correspondante constituée d'acides aminés tous de configuration D, n'induit pas de réponse immunitaire.

La présente invention a pour but de fournir des compositions pharmaceutiques, et plus particulièrement des vaccins, comprenant des analogues peptidiques présentant une demi vie nettement supérieure à celle des protéines naturelles, ou à celle des peptides de synthèse issus ou non de ces protéines naturelles (ces protéines naturelles, ou peptides issus ou non de ces dernières, étant encore désignés dans ce qui suit par l'expression "protéines ou peptides parents"), dont ils sont les analogues, tout en présentant une activité biologique, et plus particulièrement immunologique, comparable, voire même supérieure, à celle des protéines ou peptides parents susmentionnés.

La présente invention a également pour but de fournir des méthodes de diagnostic *in vitro* de pathologies associées à la présence dans l'organisme d'un individu de protéines endogènes ou exogènes, ces méthodes étant réalisées à l'aide d'analogues peptidiques tels que définis ci-dessus, et présentant l'avantage d'être plus performants que les méthodes de diagnostic actuelles réalisées à l'aide des peptides ou protéines parents. La présente invention a donc notamment pour but de fournir de nouveaux kits pour la mise en oeuvre de telles méthodes de diagnostic.

La présente invention a principalement pour objet l'utilisation

* de composés du type peptidique (encore désignés analogues peptidiques, ou pseudopeptides, ou peptidomimétiques),

  dont l'une au moins des liaisons -CO-NH-, et avantageusement toutes les liaisons -CO-NH-, de la chaîne peptidique du peptide parent correspondant, (ne comportant pas de liaison -NH-CO- dans sa chaîne peptidique), est (sont) remplacée(s) par une (des) liaison(s) -NH-CO-, la chiralité de chaque résidu aminoacyle, qu'il soit impliqué ou non dans une ou plusieurs liaisons -NH-CO- susmentionnées, étant soit conservée, soit inversée par rapport aux résidus aminoacyles correspondants constituant le dit peptide parent,
ces composés du type peptidique étant encore désignés peptides « rétro-inverso », les dits peptides rétro-inverso dérivant des peptides parents qui répondent à la formule (I) suivante:

$$X\text{-}AA1\text{-}(AA2\text{-----}AAn\text{-}1)_i\text{-}AAn\text{-}Y \qquad (I)$$

dans laquelle:

- AA1 représente un résidu aminoacyle pouvant être désaminé et dont la fonction amine en $\alpha$, si elle existe, peut être protégée par un groupement X, X représentant P-, R- ou RCO-
- i = 0 ou 1,
- n représente 2 lorsque i = 0, et n représente un nombre entier allant de 3 à 1000, et de préférence de 5 à 100, lorsque i = 1, étant entendu que lorsque n = 3, le peptide rétro-inverso correspondant répond à la formule AA1-AA2-AA3,
- AAn représente un résidu aminoacyle pouvant être décarboxylé dont la fonction acide en $\alpha$, si elle existe, est protégée éventuellement par un groupement Y, Y étant de type ester: -OR ou amide $-NH_2$, ou -NRR',

les groupements R, R' pouvant représenter des atomes d'hydrogène, des radicaux alkyle de 1 à 25 atomes de carbone, des radicaux contenant un groupe allyle et ayant de 3 à 25 atomes de carbone ou des radicaux contenant un groupe aryle et ayant de 6 à 25 atomes de carbone, et notamment $-CH_3$ (méthyl), $-CH_2CH_3$ (éthyl),$-CH(CH_3)_2$ (isopropyl), $-C(CH_3)_3$ (tertio-butyl), $-\Phi$ (phényl), $-CH_2\Phi$ (benzyl), $-CH_2\text{-}CH_2\Phi$ (2-phényl-éthyl), $-CH_2CHCH_2$ (allyl), méthyl-fluorényl, $-CH_2CONH_2$ (glycolamide), $-CH_2CON\Phi_2$ (benzhydrylglycolamide), cette liste n'étant pas limitative,

le groupement P étant de type uréthane (Boc (tertbutyloxycarbonyl), Fmoc (fluorénylméthyloxycarbonyl), Z (benzyloxycarbonyl), $CH_2CHCH_2OCO$-(allyloxycarbonyl) ou autre),

et dans ces peptides parents de formule (I), l'une au moins des liaisons entre deux résidus aminoacyles de la formule I étant une liaison -NH-CO-, et le cas échéant, l'un au moins des résidus aminoacyles AA1 à AAn étant de chiralité opposée à celle du résidu aminoacyle correspondant dans le peptide parent et notamment les peptides retro invero répondant à la formule (II) suivante:

$$A\text{-}CH(R_i)\text{-}NH\text{-}[CO\text{-}CH(R_k)\text{-}NH]_{j\text{-}i}\text{-}CO\text{-}CH(R_{j+1})\text{-}B \qquad (II)$$

dans laquelle:

- $R_i$, $R_k$, $R_{j+1}$ représentent les chaînes latérales des résidus impliqués dans une ou plusieurs liaisons -NH-CO,
- le nombre total de résidus de la séquence est fixé à n où n est un nombre entier supérieur à 1, et de préférence de 3 à 1000, de préférence de 3 à 100,
- i, j, k, sont des paramètres entiers définis de la façon suivante:
  $1 \leq 1 < j \leq n$.
  k = 0 si i = j et k prend les valeurs i+1 à j,

quatre cas de figure pouvant se présenter concernant la nature des blocs A et B:

<u>1/ i = 1 et j+1 = n:</u>

A = H-, $H_2N$-, P-HN-, RR'N-, $H_2NCO$-, RR'NCO-, RCO-
B = H-, -COOH, -COOR, $-CONH_2$, -CONRR', -NHCOR.

<u>2/ i = 1 et j+1 $\neq$n:</u>

A = H-, $H_2N$-, P-HN-, RR'N-, $H_2NCO$-, RR'NCO-, RCO-
B = $-CO\text{-}NH\text{-}CH(R_{j+2})\text{-}CO\text{-}...\text{-}NH\text{-}CH(R_n)\text{-}CO\text{-}Y$
avec Y = -OH, -OR, $-NH_2$, -NRR'.

<u>3/ i $\neq$ 1 et j+1 = n:</u>

A = X-HN-CH($R_1$)-CO-...-NH-CH($R_{i-1}$)CO-NH-
avec X = H-, P-, R-, RCO-
B = H-, -COOH, -COOR, -CONH$_2$, -CONRR', -NHCOR.

4/ i ≠ 1 et j+1 ≠ n:

A = X-HN-CH($R_1$)-CO-...-NH-CH($R_{i-1}$)CO-NH-
avec X = H-, P-, R-, RCO-
B = -CO-NH-CH($R_{j+2}$)-CO-...-NH-CH($R_n$)-CO-Y
avec Y = -OH, -OR, -NH$_2$, -NRR',

les groupements R, R' pouvant représenter des atomes d'hydrogène, des radicaux alkyle de 1 à 25 atomes de carbone, des radicaux contenant un groupe allyle et ayant de 3 à 25 atomes de carbone ou des radicaux contenant un groupe aryle et ayant 6 à 25 atomes de carbone, et notamment -CH$_3$ (méthyl), -CH$_2$CH$_3$ (éthyl), -CH(CH$_3$)$_2$ (isopropyl), -C(CH$_3$)$_3$ (tertio-butyl), -Φ (phényl), -CH$_2$Φ (benzyl), -CH$_2$-CH$_2$Φ (2-phényl-éthyl), -CH$_2$CHCH$_2$ (allyl), méthyl-fluorényl, -CH$_2$CONH$_2$ (glycolamide), -CH$_2$CONΦ$_2$ (benzhydrylglycolamide), cette liste n'étant pas limitative,

le groupement P étant de type uréthane (Boc (tertbutyloxycarbonyl), Fmoc (fluorénylméthyloxycarbonyl), Z (benzyloxycarbonyl), CH$_2$CHCH$_2$OCO-(allyloxycarbonyl) ou autre), la chiralité de chaque résidu, qu'il soit impliqué ou non dans une ou plusieurs liaisons -NH-CO-, étant soit conservée, soit inversée par rapport aux résidus aminoacyles correspondants constituant le peptide parent.

* ou d'anticorps dirigés contre les susdits peptides rétro-inverso (anticorps anti- rétro-inverso),

  lesdits peptides rétro-inverso étant susceptibles de former un complexe avec les susdits anticorps anti-rétro-inverso ainsi qu'avec les anticorps dirigés contre les peptides ou protéines parents (désignés anticorps anti-parents), pour la préparation:

  - d'un médicament destiné à la prévention ou au traitement de pathologies d'origine virale ou bactérienne, ou de pathologies auto immunes ou de maladies neuro dégénératives, ou
  - d'un médicament destiné à la prévention ou au traitement de pathologies impliquant les molécules du complexe majeur d'histocompatibilité et/ou les récepteurs des cellules T,

ou pour la mise en oeuvre d'une méthode de diagnostic *in vitro* des pathologies susmentionnées.

Comme nous l'avons déjà vu précédemment, il faut entendre par l'expression "peptide parent" susmentionnée,

- soit un peptide existant tel quel à l'état naturel, notamment dans un microorganisme ou dans un organisme supérieur (notamment dans l'organisme humain),
- soit tout peptide d'intérêt immunologique obtenu par synthèse peptidique,
- soit un peptide issu d'une protéine telle qu'elle existe à l'état naturel dans les organismes susmentionnés, notamment par fragmentation de ladite protéine (notamment à l'aide de protéases appropriées, puis purification du peptide en question), ou par synthèse peptidique (selon les méthodes classiquement utilisées dans ce domaine)
- soit un peptide issu d'une protéine telle qu'elle existe à l'état naturel mais dont l'activité immunologique a été modifiée, conservée ou optimisée par remplacement de certains amino acides de la séquence naturelle, par exemple à la suite d'un criblage d'une librairie de peptides analogues obtenue par synthèse peptidique.

Il va de soi que les liaisons -CO-NH- et -NH-CO- doivent être prises en compte dans ce qui précède et ce qui suit, dans le sens de la chaîne peptidique parente allant de l'extrémité aminoterminale (N-terminale) vers l'extrémité carboxyterminale (C-terminale).

Les peptides rétro-inverso utilisés dans l'invention peuvent être linéaires ou cycliques ou branchés.

Les peptides rétro-inverso utilisés dans le cadre de la présente invention sont des composés constitués d'une chaîne peptidique dans laquelle l'un au moins des résidus, ce résidu étant, le cas échéant, de chiralité opposée à celle du résidu aminoacyle lui correspondant dans la chaîne peptidique du peptide parent, est lié à au moins un de ses résidus voisins par une liaison -NH-CO-, ladite chaîne peptidique comportant, le cas échéant, un ou plusieurs aminoacyles de chiralité opposée à celle du résidu aminoacyle lui correspondant dans la chaîne peptidique du peptide parent, les extrémités amino et carboxyterminales pouvant être, indépendamment l'une de l'autre, soit identiques aux extrémités N et C-terminales du peptide parent correspondant, soit différentes de ces dernières extrémités.

Par résidu, on entend un groupe de formule -X-CH(R)-Y-, dans laquelle X et Y sont identiques ou différents l'un de l'autre et sont choisis parmi -NH- ou CO-. Par résidu, on désigne donc soit un résidu aminoacyle, soit un dérivé

d'aminoacyle dans lequel les extrémités terminales ne sont pas celles d'un résidu aminoacyle.

Dans les peptides utilisés dans le cadre de la présente invention, les liaisons - NH-CO- qui remplacent les liaisons -CO-NH- du peptide parent correspondant peuvent être à n'importe quel endroit du peptide, et peuvent remplacer soit un -CO-NH- correspondant à un site de coupure aux protéases, soit un -CO-NH- ne correspondant pas à un tel site.

Les liaisons -NH-CO- qui remplacent les liaisons -CO-NH- sont avantageusement celles qui interagissent avec le récepteur du peptide (anticorps, MHC, récepteur T).

Lorsque i = 1 et j+1 = n, toutes les liaisons sont des liaisons -NH-CO-.

Lorsque i = 1 et j+1 ≠ n, les liaisons -NH-CO- sont du côté N-terminal.

Lorsque i ≠ 1 et j+1 ≠ n, les liaisons -NH-CO- sont du côté C-terminal.

Lorsque i ≠ 1 et j+1 ≠ n, les liaisons -NH-CO- ne sont ni du côté N-terminal, ni du côté C-terminal.

Le nombre de liaisons -NH-CO- est égal à j-i+1.

De façon avantageuse, le nombre de liaisons -NH-CO- est au moins égal à 2.

Par peptides rétro-inverso, il faut entendre tout peptide et analogue peptidique répondant à la définition donnée ci-dessus des peptides utilisés dans le cadre de la présente invention, ledit peptide étant plus particulièrement constitué d'une chaîne peptidique dans laquelle l'un au moins des résidus d'une part est lié à au moins un résidu voisin par une liaison -NH-CO-, et d'autre part s'il s'agit d'un résidu aminoacyle, est de chiralité opposée à celle de ce même résidu aminoacyle dans la chaîne peptidique du peptide parent.

Les peptides rétro-inverso utilisés dans le cadre de la présente invention sont plus particulièrement ceux tels que définis ci-dessus, et répondant à la formule (II) susmentionnée, dans laquelle l'un au moins des résidus est lié à au moins un de ses résidus voisins par une liaison -NH-CO-, et s'il s'agit d'un résidu aminoacyle est de chiralité opposée à celle du résidu aminoacyle correspondant dans le peptide parent.

Ces peptides rétro-inverso susmentionnés sont:

* soit des peptides partiellement rétro-inverso, à savoir:

  a) soit des peptides partiellement rétro-totalement inverso, c'est-à-dire dont seulement une ou plusieurs, mais non la totalité, des liaisons entre les résidus, est (sont) une (des) liaison(s) -NH-CO-, et dont la totalité des résidus aminoacyles lié(s) à au moins un résidu voisin par une liaison -NH-CO- est (sont) de chiralité opposée à celle du résidu aminoacyle correspondant dans le peptide parent,

  b) soit des peptides partiellement rétro-partiellement inverso, c'est-à-dire dont seulement une ou plusieurs, mais non la totalité, des liaisons entre les résidus, est (sont) une (des) liaison(s) NH-CO-, et dont au moins un, mais non la totalité, des résidus aminoacyles lié(s) à au moins un résidu voisin par une liaison -NH-CO- est (sont) de chiralité opposée à celle du résidu aminoacyle correspondant dans le peptide parent,

  ces deux types de peptides partiellement rétro-inverso étant représentés par la formule (II) et plus particulièrement décrits par les cas de figure 2, 3, 4 qui s'y rapportent,

* soit des peptides totalement rétro-partiellement inverso, c'est-à-dire dont la totalité des liaisons entre les résidus, est (sont) une (des) liaison(s) -NH-CO-, et dont au moins un, mais pas la totalité des résidus aminoacyles lié(s) à au moins un résidu voisin par une liaison -NH-CO- est (sont) de chiralité opposée à celle du résidu aminoacyle correspondant dans le peptide parent,
* soit des peptides totalement rétro-inverso, c'est-à-dire dont la totalité des liaisons entre les résidus, sont des liaisons -NH-CO-, et dont la totalité des résidus aminoacyles de la chaîne peptidique de ces peptides rétro-inverso, sont de chiralité opposée à celle de leurs résidus aminoacyles correspondants dans le peptide parent.

Ces deux derniers types de peptides rétro-inverso sont représentés par la formule (II) et sont plus particulièrement décrits par le cas de figure 1 qui s'y rapporte.

Avantageusement, les peptides rétro-inverso utilisés dans le cadre de la présente invention sont totalement rétro-inverso.

Une autre classe avantageuse de peptides rétro-inverso utilisés dans le cadre de l'invention ont au moins deux liaisons "rétro-inverso" consécutives.

Les pathologies susceptibles d'être diagnostiquées ou traitées à l'aide de compositions pharmaceutiques à base de peptide rétro-inverso dans le cadre de la présente invention, sont principalement des pathologies d'origine virale ou bactérienne, ou sont des pathologies auto-immunes, ou encore des maladies neurodégénératives.

Parmi les maladies d'origine virale susceptibles d'être diagnostiquées ou traitées dans le cadre de la présente invention, on peut citer:

- le SIDA provoqué par le virus de l'immuno-déficience humaine HIV1 et HIV2,

- la paraplégie associée à HTVL-1, ou la leucémie à cellules T de l'adulte, provoquée par le virus de la leucémie humaine à cellules T (virus HTLV),
- infections provoquées par le virus respiratoire syncitial,
- infections provoquées par le virus coxsakie, par exemple méningites aiguës lymphocytaires,
- infections provoquées par le virus d'Epstein-Barr, par exemple la mononucléose infectieuse,
- infections provoquées par le cytomégalovirus, par exemple maladie des inclusions cytomégaliques,
- herpès provoqué par le virus de l'herpès humain,
- herpès provoqué par le virus 6 de l'herpès simplex,
- infections provoquées par le parvovirus B19 humain, par exemple gastro-entérites infectieuses,
- l'hépatite B provoquée par le virus de l'hépatite B,
- l'hépatite C provoquée par le virus de l'hépatite C,
- la grippe provoquée par le virus influenza,
- la rubéole provoquée par le virus de la rubéole,
- infections provoquées par le virus de la Dengue, par exemple arboviroses,
- rhumes, rhinites, coryza provoqués par les rhinovirus
- la fièvre aphteuse provoquée par le virus de la fièvre aphteuse.

Parmi les principales maladies autoinmmunes susceptibles d'être traitées dans le cadre de la présente invention, on peut citer celles rassemblées dans le Tableau A qui suit.

Tableau A

| Principales maladies autoimmunes (en allant de haut en bas, des maladies autoimmunes spécifiques d'organes aux maladies autoimmunes non spécifiques d'organes). | |
|---|---|
| Maladies | Autoantigènes cibles |
| Thyroïdite de Hashimoto | Thyroglobuline, microsomes |
| Maladie de Basedow | Récepteur TSH |
| Maladie d'Addison | Corticosurrénale |
| Insuffisance hypophysaire | Hypophyse |
| Gastrite de Biermer | Cellule pariétale de l'estomac |
| | Facteur intrinsèque |
| Certaines stérilités | Spermatozoïdes, ovaires |
| Diabète juvénile de type 1 | Ilots Langerhans, insuline |
| Syndrome de Goodpasture | Membrane basale glomérulaire |
| Myasthénie | Muscle strié, récepteur acétyl-choline |
| Rhumatisme articulaire aigu | Myocarde (streptocoques) |
| Pemphigus | Ponts intercellaires épiderme |
| Pemphigoïde bulleuse | Membrane basale cutanée |
| Dermatite herpétiforme | Gliadine, réticuline |
| Vitiligo | Mélanocytes |
| Pelade | Follicule pileux |
| Psoriasis | |
| Ophtalmie sympathique | Uvée |
| Uvéite | Chambre antérieure de l'oeil |
| Syndrome de Guillain-Baré | |
| Sclérose en plaques | Myéline |
| Anémie hémolytique | Hématies |
| Purpura thrombopénique idiopathique | Plaquettes |
| Leucopénie idiopathique | Granulocytes |
| Cirrhose biliaire primitive | Mitochondries |
| Hépatite chronique active | Muscle lisse, noyaux |
| Rectocolite hémorragique | |
| Iléite de Crohn | Côlon (*E. coli*) |
| Syndrome de Gougerot-Sjögren | Noyaux: SS-A, SS-B |

Tableau A (suite)

| Principales maladies autoimmunes (en allant de haut en bas, des maladies autoimmunes spécifiques d'organes aux maladies autoimmunes non spécifiques d'organes). | |
|---|---|
| Maladies | Autoantigènes cibles |
| Polyarthrite rhumatoïde | IgG, noyaux |
| Dermatopolymyosite | Noyaux: Jol, muscles |
| Sclérodermie | Noyaux: Scl-70 |
| Connectivite mixte | Noyaux: RNP |
| Lupus érythémateux discoïde | Noyaux: |
| Lupus érythémateux disséminé | Noyaux: ADN, antigène Sm |
| | Facteurs de coagulation Cardiolipine, etc... |

L'invention a également pour objet les anticorps dirigés contre les peptides rétro-inverso selon l'invention, encore désignés anticorps anti-peptides rétro-inverso

Les anticorps anti-rétro-inverso selon l'invention sont des anticorps polyclonaux ou monoclonaux.

Les anticorps polyclonaux susmentionnés sont obtenus par immunisation d'un animal avec au moins un peptide rétro-inverso selon l'invention, suivie de la récupération des anticorps recherchés sous forme purifiée, par prélèvement du sérum dudit animal, et séparation desdits anticorps des autres constituants du sérum, notamment par chromatographie d'affinité sur une colonne sur laquelle est fixée un antigène spécifiquement reconnu par les anticorps, notamment un peptide rétro-inverso selon l'invention.

Les anticorps monoclonaux selon l'invention peuvent être obtenus par la technique des hybridomes dont le principe général est rappelé ci-après.

Dans un premier temps, on immunise un animal, généralement une souris, (ou des cellules en culture dans le cadre d'immunisations *in vitro*) avec un peptide rétro-inverso selon l'invention, dont les lymphocytes B sont alors capables de produire des anticorps contre le peptide rétro-inverso et/ou contre cette protéine et/ou contre le peptide parent. Ces lymphocytes producteurs d'anticorps sont ensuite fusionnés avec des cellules myélomateuses "immortelles" (mutines dans l'exemple) pour donner lieu à des hybridomes. A partir du mélange hétérogène des cellules ainsi obtenu, on effectue alors une sélection des cellules capables de produire un anticorps particulier et de se multiplier indéfiniment. Chaque hybridome est multiplié sous la forme de clone, chacun conduisant à la production d'un anticorps monoclonal dont les propriétés de reconnaissance vis-à-vis du peptide rétro-inverso de l'invention pourront être testées par exemple en ELISA, par immunotransfert en une ou deux dimensions, en immunofluorescence, ou à l'aide d'un biocapteur. Les anticorps monoclonaux ainsi sélectionnés, sont par la suite purifiés notamment selon la technique de chromatographie d'affinité décrite ci-dessus.

Les anticorps selon l'invention, sont plus particulièrement caractérisés en ce qu'ils sont susceptibles de former un complexe avec des peptides rétro-inverso

, et/ou avec les peptides ou protéines parents correspondant à ces derniers.

A ce titre, l'invention vise plus particulièrement les anticorps tels que définis ci-dessus, et caractérisés en ce qu'ils reconnaissent les peptides rétro-inverso, et/ou les peptides ou protéines parents correspondants, notamment les peptides ou protéines parents de configuration L.

Les anticorps selon l'invention sont plus particulièrement caractérisés encore, en ce qu'ils sont des anticorps protecteurs, à savoir des anticorps capables, lorsqu'ils sont administrés dans l'organisme d'un individu porteur d'une protéine endogène ou exogène pathogène, ou lorsque leur formation est induite par administration dans l'organisme de l'individu d'un peptide rétro-inverso selon l'invention susceptible d'être reconnu par de tels anticorps, de se lier à ladite protéine endogène ou exogène dans des conditions telles que le caractère pathogène associé à cette protéine soit ainsi neutralisé.

Les anticorps anti-peptide rétro-inverso de l'invention reconnaissent le peptide parent ou la protéine parente avec une affinité au moins égale à celle présentée par les anticorps anti-peptide parent ou anti-protéine parente vis à vis du peptide parent ou de la protéine parente.

Les anticorps anti-peptides ou anti-protéines parentes reconnaissent les peptides rétro-inverso utilisés dans le cadre de l'invention avec une affinité au moins égale à celle présentée par ces mêmes susdits anticorps vis à vis des peptides parents ou protéines parents correspondant à ces susdits peptides rétro-inverso. L'affinité dont il est question ci-dessus peut se mesurer par la constante d'affinité à l'équilibre Ka des complexes impliquant l'un des susdits anticorps avec l'un des sus-dits antigènes.

L'invention concerne également les anti-idiotypes susceptibles de former un complexe avec les anticorps selon l'invention, ces anti-idiotypes étant obtenus par immunisation d'un animal avec lesdits anticorps tels que définis

ci-dessus selon l'invention.

L'invention a également plus particulièrement pour objet les complexes antigène-anticorps formés entre les peptides rétro-inverso selon l'invention, ou leur peptide parent ou protéine parente et les anticorps tels que définis ci-dessus.

A ce titre, l'invention vise plus particulièrement les complexes suivants:

| | |
|---|---|
| - protéine parente | anticorps dirigés contre un peptide rétro-inverso correspondant à un peptide parent issu de la protéine parente |
| - peptide parent | - anticorps dirigé contre un peptide rétro-inverso correspondant au peptide parent |
| - peptide rétro-inverso correspondant à un peptide parent | - anticorps dirigé contre le peptide parent ou la protéine parente |
| - peptide rétro-inverso correspondant à un peptide parent | - anticorps dirigé contre un peptide rétro-inverso correspondant au peptide parent |

Par peptide parent issu de la protéine parente, on désigne une séquence de la protéine parente.

Il faut noter que l'ensemble des complexes définis ci-dessus sont tels qu'ils présentent une stabilité au moins égale à celle de ces complexes, dans lesquels, lorsqu'intervient un peptide rétro-inverso, il est remplacé par un peptide parent ou une protéine parente, et lorsqu'intervient un anticorps anti-peptide rétro-inverso, il est remplacé par un anticorps anti-peptide parent ou anti-protéine parent.

En particulier, les complexes peptides parents/protéines parentes-anticorps anti-peptides rétro-inverso sont au moins aussi stables que les complexes peptides parents/protéines parentes-anticorps dirigés contre le peptide ou la protéine parente correspondant à ces susdits peptides rétro-inverso.

Il faut également noter que les complexes peptides rétro-inverso-anticorps dirigés contre le peptide parent ou la protéine parente sont au moins aussi stables que les complexes correspondants dans lesquels les peptides rétro-inverso sont remplacés par le peptide parent ou la protéine parente.

De façon avantageuse, l'invention a pour objet des complexes antigène-anticorps, dans lesquels l'antigène est un peptide rétro-inverso et/ou l'anticorps est dirigé contre un peptide rétro-inverso, ayant une constante d'affinité supérieure à la constante d'affinité présentée par les complexes antigène parent (c'est-à-dire peptide parent ou protéine parente) anticorps vis à vis des antigènes parents (c'est-à-dire anticorps dirigés contre le peptide parent ou la protéine parente).

Selon un mode de réalisation avantageux, l'invention concerne les complexes antigène-anticorps dans lesquels l'antigène est un un rétro-inverso et l'anticorps est dirigé contre le peptide parent ou la protéine parente (correspondant au susdit rétro-inverso), ayant une constante d'affinité supérieure à la constante d'affinité présentée par le complexe antigène-anticorps dans lequel l'antigène est le peptide parent ou la protéine parente et l'anticorps est dirigé contre le peptide parent ou la protéine parente.

Afin de fixer les idées, l'ordre de grandeur selon lequel "la constante d'affinité est supérieure" peut varier d'environ 5 à environ 1000, en particulier d'environ 7 à environ 1000, avantageusement d'environ 10 à 1000, et notamment d'environ 10 à environ 100.

L'invention concerne également les complexes entre un peptide rétro-inverso tel que défini ci-dessus, et une molécule du complexe majeur d'histocompatibilité (encore désigné complexe CMH-rétro-inverso).

En effet, la réponse immunitaire met en jeu la reconnaissance d'un antigène endogène ou exogène par des cellules spécialisées. Pour être reconnu, l'antigène doit être initialement présenté de façon adéquate par des cellules présentatrices d'antigène (CPA). Alors que les lymphocytes B reconnaissent des épitopes portés par les antigènes intacts non modifiés, la présentation de l'antigène aux lymphocytes T est plus complexe dans la mesure où l'antigène est d'abord internalisé par la cellule présentatrice, protéolysé, puis éventuellement réexprimé à sa surface sous forme de fragments peptidiques en association avec les protéines du complexe majeur d'histocompatibilité (CMH). Le lymphocyte T qui ne reconnaît pas l'antigène natif, reconnaît un fragment peptidique associé à une molécule CMH.

Ces molécules CMH appartiennent à deux classes: I et II.

Les molécules de classe I sont des glycoprotéines transmembranaires constituées d'une chaîne lourde $\alpha$ polymorphe associée de façon non covalente à une chaîne légère non glycosylée $\beta$2m. Leur structure cristallographique a été résolue (Bjorkman et al. (1987), Nature, 329: 506-512), et montre la présence d'un sillon formant le site de présentation du peptide dont le fond est composé de huit feuillets $\beta$ et les bords de deux hélices $\alpha$. Ces molécules sont présentées à la surface de la quasi totalité des cellules.

Les molécules de classe II sont également des glycoprotéines membranaires constituées de deux chaînes polymorphes $\alpha$ et $\beta$ liées de façon non covalentes pour former, comme le montre la structure cristallographique récemment élucidée (Brown et al. (1993), Nature, 364: 33-39), une plateforme β plissée supportant deux hélices α. Le sillon formé est le site de présentation du peptide. Ces molécules ne sont exprimées qu'à la surface de certaines cellules parmi lesquelles les macrophages et les cellules B.

Les lymphocytes T cytotoxiques (cellules possédant des marqueurs CD8) reconnaissent des fragments protéolytiques de protéines virales associées aux molécules CMH de classe I et provoquent la lyse des cellules présentant l'antigène.

Les lymphocytes T auxiliaires (cellules portant des marqueurs CD4) reconnaissent des fragments de protéines exogènes capturées par endocytose présentés en association avec les molécules CMH de classe II et induisent la stimulation cellulaire de la réponse immunitaire.

L'invention a également pour objet les complexes entre un peptide rétro-inverso selon l'invention, et un récepteur de cellules T.

L'invention vise également les complexes entre une molécule du complexe majeur d'histocompatibilité, un peptide rétro-inverso tel que défini ci-dessus, et un récepteur de cellules T (encore désigné complexe CMH-peptide rétro-inverso-récepteur T).

L'invention a également pour objet l'utilisation des peptides rétro-inverso tels que définis ci-dessus, pour la mise en oeuvre de méthodes de diagnostic *in vitro* de pathologies, telles que celles mentionnées ci-dessus, associées à la présence dans l'organisme d'un individu, d'une ou plusieurs protéine(s) exogène(s) ou endogène(s) susceptible (s), d'une part d'être directement ou indirectement impliquée(s) dans le processus d'apparition et/ou de développement de ces pathologies, et, d'autre part, d'être reconnue(s) soit par les anticorps anti-peptides rétro-inverso selon l'invention, soit par les peptides rétro-inverso selon l'invention, par exemple dans le cas de la détection d'anticorps chez le patient.

A ce titre, l'invention a plus particulièrement pour objet toute méthode de diagnostic *in vitro* telle que définie ci-dessus et comprenant:

- la mise en contact d'un échantillon biologique provenant d'un patient susceptible d'être porteur d'anticorps dirigés contre lesdites protéines endogènes ou exogènes, avec un peptide rétro-inverso selon l'invention dans des conditions permettant la réaction entre les anticorps dirigés contre lesdites protéines et susceptibles d'être présents dans l'échantillon biologique, et le susdit peptide rétro-inverso ;
- la détection *in vitro* du complexe antigène - anticorps tel que défini ci-dessus selon l'invention, susceptible d'être formé à l'étape précédente, ou
- la détection *in vitro* d'anticorps chez le patient par un test de compétition en utilisant un anticorps anti-peptide rétro-inverso.

Avantageusement, le peptide rétro-inverso utilisé dans la méthode de diagnostic *in vitro* susmentionnée, est un peptide rétro-inverso correspondant à tout ou partie desdites protéines endogènes ou exogènes, ou correspondant à un peptide susceptible d'être reconnu par des anticorps reconnaissant eux-mêmes les protéines exogènes ou endogènes.

L'invention concerne également toute méthode de diagnostic *in vitro* telle que définie ci-dessus et comprenant:

- la mise en contact d'un échantillon biologique provenant d'un individu susceptible d'être porteur desdites protéines endogènes ou exogènes, avec l'un au moins des anticorps tels que définis ci-dessus, dirigés contre un selon l'invention, dans des conditions permettant la réaction entre lesdites protéines susceptibles d'être présentes dans l'échantillon biologique, et les susdits anticorps dirigés contre le susdit peptide rétro-inverso;
- la détection *in vitro* du complexe antigène - anticorps tel que défini ci-dessus, susceptible d'être formé à l'étape précédente, ou
- la détection d'antigènes circulants dans des tests de compétition en utilisant l'un des susdits peptides rétro-inverso.

Avantageusement, les anticorps dirigés contre le peptide rétro-inverso, utilisés dans la méthode de diagnostic in vitro susmentionnée, sont ceux décrits ci-dessus selon l'invention, dirigés contre un peptide rétro-inverso correspondant à tout ou partie desdites protéines endogènes ou exogènes.

Selon un autre mode préféré de réalisation de la méthode de diagnostic *in vitro* susmentionnée, les anticorps dirigés contre le peptide rétro-inverso, sont ceux tels que définis ci-dessus selon l'invention, dirigés contre un peptide rétro correspondant à tout ou partie desdites protéines endogènes ou exogènes.

Les méthodes de diagnostic susmentionnées de l'invention sont avantageusement réalisées de la façon suivante:

- incubation de l'échantillon biologique susceptible de contenir des antigènes (protéine ou peptide exogène ou endogène), ou des anticorps dirigés contre ces antigènes, ces antigènes ou anticorps étant associés à une pathologie ou une famille de pathologies déterminées, notamment aux pathologies décrites ci-dessus, avec respectivement

des anticorps anti-rétro-inverso selon l'invention, susceptibles de reconnaître lesdits antigènes, ou des peptides rétro-inverso selon l'invention, susceptibles d'être reconnus par lesdits anticorps dirigés contre ces antigènes, lesdits antigènes ou anticorps dirigés contre ces derniers étant fixés sur un support solide, notamment à l'intérieur de puits de plaques de microtitration de type de celles habituellement utilisées pour la mise en oeuvre de techniques de détection ou dosage bien connues sous le nom d'ELISA (Enzyme Linked Immuno Sorbent Assay),
- rinçage du support solide,
- incubation des éléments restés fixés sur le support solide après l'étape de rinçage précédente:

  . soit avec un milieu contenant des anticorps, notamment des anticorps anti-rétro-inverso selon l'invention, marqués (notamment de manière radioactive, enzymatique ou fluorescente), ou susceptibles d'être reconnus à leur tour par un réactif marqué, les dits anticorps marqués étant susceptibles de reconnaître les antigènes présents dans l'échantillon biologique, qui sont restés liés, après l'étape de rinçage précédente, aux anticorps anti-rétro-inverso selon l'invention initialement fixés sur le support solide,
  . soit avec un milieu contenant des antigènes, notamment des peptides rétro-inverso selon l'invention, marqués (notamment de manière radioactive, enzymatique ou fluorescente), ou susceptibles d'être reconnus à leur tour par un réactif marqué, lesdits antigènes marqués étant susceptibles de reconnaître les anticorps présents dans l'échantillon biologique qui sont restés liés, après l'étape de rinçage précédente, aux peptides rétro-inverso selon l'invention initialement fixés sur le support solide,

- rinçage du support solide,
- détection des antigènes ou anticorps marqués restés respectivement liés aux anticorps ou antigènes de l'échantillon biologique lors de l'étape d'incubation précédente.

Avantageusement, les méthodes de diagnostic *in vitro* de l'invention sont réalisées à l'aide de peptides totalement rétro-inverso, ou encore d'anticorps dirigés contre ces peptides totalement rétro-inverso.
L'invention a également pour objet les nécessaires ou kits pour la mise en oeuvre de méthodes de diagnostic *in vitro* telles que décrites ci-dessus, comprenant:

- un peptide rétro-inverso selon l'invention choisi parmi un peptide rétro-inverso correspondant à tout ou partie desdites protéines endogènes ou exogènes, ou correspondant à un peptide susceptible d'être reconnu par des anticorps reconnaissant eux-mêmes les protéines exogènes ou endogènes, ou
- des anticorps anti-rétro-inverso selon l'invention, dirigés contre ce peptide rétro-inverso;
- des réactifs pour rendre un milieu apte à la formation d'une réaction immunologique;
- des réactifs permettant de détecter le complexe antigène - anticorps, appartenant à la liste des composés définie ci-dessus, qui a été produit à l'issue de la réaction immunologique, lesdits réactifs contenant éventuellement un marqueur ou étant susceptibles d'être reconnus à leur tour par un réactif marqué, plus particulièrement dans le cas où le peptide rétro-inverso ou les anticorps anti-rétro-inverso susmentionnés ne sont pas marqués.

L'invention concerne également l'utilisation d'au moins un peptide rétro-inverso tel que défini ci-dessus, pour la préparation d'un médicament destiné à la prévention ou au traitement de pathologies associées à la présence dans l'organisme d'un individu, d'une ou plusieurs protéine(s) exogène(s) ou endogéne(s) susceptible(s) d'être directement ou indirectement impliquée(s) dans le processus d'apparition et/ou de développement de ces pathologies.
Les pathologies susmentionnées susceptibles d'être traitées dans le cadre de la présente invention, sont principalement soit des maladies d'origine virale, bactérienne ou parasitaire, lorsqu'elles sont associées à la présence du microorganisme lui-même, d'une protéine ou d'un peptide exogène provenant d'une particule virale, bactérienne ou parasitaire, soit des maladies autoimmunes lorsqu'elles sont associées à la présence de protéines ou peptides endogènes perturbant le fonctionnement physiologique normal d'un organisme lorsque ces derniers jouent directement un rôle d'anticorps ou induisent la formation d'anticorps reconnaissant et altérant des sites particuliers de l'organisme (par exemple en formant des dépôts de complexes anticorps-antigène, en provoquant des états inflammatoires etc...).
Les pathologies susmentionnées peuvent également être des maladies neurodégénératives lorsqu'elles sont associées à la présence dans l'organisme de protéines exogènes ayant pour effet de provoquer des lésions neurologiques.
Les peptides rétro-inverso utilisés pour la préparation des compositions pharmaceutiques ou des vaccins dans ce qui précède et ce qui suit, sont avantageusement des peptides totalement rétro-inverso.
L'invention vise plus particulièrement l'utilisation d'au moins un peptide rétro-inverso tel que défini ci-dessus, pour la préparation d'un vaccin dans le cadre de la prévention de pathologies associées à la présence dans l'organisme d'un individu, d'une ou plusieurs protéine(s) exogène(s) ou endogène(s) susceptible(s) d'être reconnue(s) par les anticorps dirigés contre les peptides rétro-inverso ou dirigés contre les anti-idiotypes selon l'invention.

L'invention concerne également les compositions pharmaceutiques, notamment les vaccins, comprenant au moins un anti-idiotype tel que défini ci-dessus, en association avec un véhicule physiologiquement acceptable.

Avantageusement, les anti-idiotypes utilisés dans les compositions pharmaceutiques susmentionnées sont obtenus à l'aide de peptides totalement rétro-inverso.

L'invention vise également toute composition pharmaceutique comprenant au moins un peptide rétro-inverso tel que défini ci-dessus, ou au moins un anti-idiotype susmentionné, associés à une molécule porteuse protéique ou non, pouvant induire *in vivo* la production d'anticorps neutralisant lesdites protéines exogènes ou endogènes responsables de la pathologie, ou induire *in vivo* une réponse immune cellulaire cytotoxique, comme décrit ci-dessus.

A titre d'illustration dans le domaine de la vaccination, l'invention a plus particulièrement pour objet les deux peptides totalement rétro-partiellement inverso de formule suivante:

HO-m(R,S)Leu-DGln-DArg-DAla-DVal-DArg-DX1-DAla-DLeu-DSer-Gly-DX2-DAsp-Gly-DArg-DVal-Gly-DSer-Gly-[Cys-$NH_2$]

X1 = Ser ou Phe
X2 = Leu ou Pro

correspondant aux peptides [A] et [USA] issus du déterminant antigénique majeur situé sur la protéine VP1 du virus de la fièvre aphteuse, et répondant aux formules suivantes:

[A] [H-Cys]-Gly-Ser-Gly-Val-Arg-Gly-Asp-Ser-Gly-Ser-Ala-Leu-Arg-Val-Ala-Arg-Gln-Leu-OH

[USA] [H-Cys]-Gly-Ser-Gly-Val-Arg-Gly-Asp-Phe-Gly-Ser-Ala-Pro-Arg-Val-Ala-Arg-Gln-Leu-OH

L'invention a également plus particulièrement pour objet le peptide cyclique totalement rétro-partiellement inverso de formule suivante:

[$CH_3$CO-Cys-Gly-Gly]-DX-DPhe-DAsp-DSer-Gly-DPro-Gly-DArg-DLys-Ser

X = acide D, $\alpha$, $\beta$, diamino-propionique,
correspondant aux résidus 139-147 du site A de l'hémagglutinine du virus de la grippe (Influenza souche X31), et répondant à la formule suivante:

Ser-Lys-Arg-Gly-Pro-Gly-Ser-Asp-Phe-Asp-Gly-Gly-Cys-$NH_2$

L'invention a également pour objet toute composition pharmaceutique comprenant des anticorps anti-rétro-inverso selon l'invention, en association ou non avec un véhicule physiologiquement acceptable.

Cette dernière catégorie de composition pharmaceutique est plus particulièrement destinée au traitement de pathologies associées à la présence dans l'organisme d'un individu, de protéines exogènes ou endogènes susceptibles d'être reconnues par les anticorps anti-rétro-inverso selon l'invention, ces derniers agissant en tant qu'anticorps protecteurs neutralisant lesdites protéines exogènes ou endogènes.

L'invention a également pour objet les peptides rétro-inverso tels que définis ci-dessus, et correspondant à des épitopes T cytotoxiques ou auxiliaires, ou encore à des peptides reconnus par les molécules CMH de type I ou II

pouvant être utilisés, soit dans la préparation de vaccins synthétiques, soit dans la prévention ou le traitement de maladies autoimmunes.

S'agissant de leur utilisation en tant que vaccins, l'invention a plus particulièrement pour objet les peptides rétro-inverso partiellement rétro-inverso d'un épitope T cytotoxique (épitope minimum 56-68: M56-68) de la matrice du virus de la grippe et d'un épitope T auxiliaire (séquence parente 830-844: TT830-844) de la toxine du tétanos dont les séquences sont représentées ci-après:

1/ Séquence de M56-68:

H-Gly-Ile-Leu-Gly-Phe-Val-Phe-Thr-Leu-OH

Séquence du rétro-analogue:

HO-m(R,S)Leu(D)-Thr(D)Phe-(D)Val-(D)Phe-(D)Phe-Gly-(D)Leu-gIle-Gly-H

2/ Séquence de TT830-844:

H-Gln-Tyr-Ile-Lys-Ala-Asn-Ser-Lys-Phe-Ile-Gly-Ile-Thr-Glu-Leu-OH

Séquence du rétro-analogue

HO-m(R,S)Leu-(D)Thr-Gly-(D)Ile-(D)Phe(D)Lys-(D)Ser-(D)Asn-(D)Ala-(D)Lys-(D)Ile-gTyr-Gln-H

L'analogue rétro-inverso de M56-68 interagit avec les molécules CMH I et est présenté aux lymphocytes T cytotoxiques afin d'induire une réponse cytotoxique. L'analogue rétro-inverso de TT830-844 interagit avec les molécules CMH II, et est présenté aux lymphocytes T auxiliaires afin de stimuler la prolifération de cellules T.

S'agissant de l'utilisation des peptides rétro-inverso dans le cadre de la préparation de médicaments destinés au traitement des maladies autoimmunes, il convient de rappeler que la pathogénèse de nombreuses maladies autoimmunes implique la présentation d'autoantigènes (liés aux molécules CMH) au récepteur de cellules T (TCR) autoréactives qui ont d'une façon ou d'une autre échappé au processus de tolérance du soi. Aussi, le développement de nouvelles stratégies pour moduler la réponse des cellules T autoréactives pourrait conduire à des approches thérapeutiques capables de traiter certaines maladies autoimmunes.

Certaines maladies autoimmunes sont associées à des allèles CMH I ou II spécifiques. Ainsi, l'utilisation de peptides bloquants capables d'interagir avec une molécule CMH donnée (par exemple une molécule CMH de classe II associée à une maladie autoimmune particulière) mais ne pouvant pas activer la réponse cellulaire T pathogénique est prometteuse. Cependant, la dégradation rapide des peptides étudiés dans les milieux biologiques rend leur utilisation difficile. Dans ce cas, les peptides rétro-inverso de par leur stabilité seraient très avantageux.

Récemment, le phénomène d'antagonisme TCR par des peptides analogues à des épitopes T a été montré et l'utilisation d'antagonistes spécifiques du TCR a été décrite (De Magistris, M.T., Alexander, J., Coggeshall, M., Altmon, A., Gaeta F.C.A., Grey, H.M. and Sette, A. (1992), Cell **68:** 625). Ces peptides sont capables d'inhiber la prolifération des cellules T induites par un antigène. De tels peptides antagonistes sont obtenus par substitution d'un acide aminé parmi les résidus du peptide antigène en contact avec la cellule T, ou bien par incorporation de résidus en contact avec le TCR dans une séquence de poly-alanines. La présente invention a précisément pour objet l'utilisation de peptides rétro-inverso correspondant à des peptides antagonistes des TCR, pour l'obtention de médicaments destinés au traitement de maladies autoimmunes telles que celles décrites ci-dessus.

Les peptides rétro-inverso peuvent être préparés comme indiqué ci-après:

A titre d'illustration, des méthodes générales de synthèse de peptides totalement ou partiellement rétro-inverso, avec modification ou non des extrémités N- et C-terminales par rapport aux extrémités des peptides parents, sont indiquées ci-après.

Le procédé de synthèse de peptides dont la totalité des liaisons -CO-NH- sont remplacées par des liaisons -NH-CO-, en comparaison aux peptides parents, sans modification des extrémités, est réalisé en reliant entre eux les différents résidus aminoacyles constituant le peptide parent, dans le sens inverse de celui indiqué pour le peptide

parent. Ainsi, si le peptide parent est représenté par une séquence du type

H-AA1-AA2-........-AAn-1AAn-OH,

le rétro analogue correspondant, synthétisé selon les techniques classiques de synthèse peptidique en phase liquide ou solide, aura la formule suivante:

H-AAn-AAn-1..........-AA2-AA1-OH

A titre d'illustration, la méthode de synthèse en phase liquide consiste à condenser successivement deux à deux les aminoacyles dans l'ordre requis ou à condenser des aminoacyles et des fragments préalablement formés et contenant déjà plusieurs aminoacyles dans l'ordre approprié, ou encore plusieurs fragments préalablement ainsi préparés, étant entendu que l'on aura eu soin de protéger au préalable toutes les fonctions réactives portées par ces aminoacyles ou fragments, à l'exception des fonctions amines de l'un et carboxyles de l'autre ou vice-versa, qui doivent normalement intervenir dans la formation des liaisons peptidiques, notamment après activation de la fonction carboxyle, selon les méthodes bien connues dans la synthèse des peptides.

On pourra par exemple utiliser des groupements protecteurs de type uréthane (Boc, Fmoc benzyloxycarbonyl ou allyloxycarbonyl) pour protéger les extrémités N-terminales des acides aminés et des groupements de type ester (méthylique, éthylique, benzylique, tertio-butylique, allylique ou encore benzhydrilglycolamidique) pour protéger les extrémités C-terminales des acides aminés.

Une telle synthèse peut être effectuée en condensant tout d'abord le résidu aminoacyle AA1 dont la fonction COOH est protégée avec le résidu aminoacyle AA2 dont la fonction $NH_2$ est protégée. La fonction amine du résidu AA2 dans le fragment AA2-AA1 ainsi obtenu, est alors déprotégée, pour condenser par la suite ledit fragment avec le résidu aminoacyle AA3 dont la fonction amine est protégée. Les étapes précédentes sont répétées autant de fois qu'il y a de résidus aminoacyles à introduire dans la chaîne des rétro analogues à synthétiser.

Selon une autre technique préférée de l'invention, on a recours à celle décrite par RD. Merrifield dans l'article intitulé "Solid phase peptide synthesis" (J. Am. Chem. Soc. (1963), **85,** 2149-2154).

Pour fabriquer une chaîne peptidique selon le procédé de Merrifield, on a recours à une résine polymère très poreuse, sur laquelle on fixe le premier acide aminé C-terminal (en l'occurrence AA1-OH) de la chaîne. Cet acide aminé est fixé sur la résine par l'intermédiaire de son groupe carboxylique et sa fonction amine est protégée, par exemple par le groupe t-butyloxycarbonyle.

Lorsque le premier acide aminé C-terminal est ainsi fixé sur la résine, on enlève le groupe protecteur de la fonction amine en lavant la résine avec un acide.

On fixe ainsi, les uns après les autres, les acides aminés qui vont constituer la chaîne peptidique sur le groupe amine chaque fois déprotégé au préalable de la portion de la chaîne peptidique déjà formée, et qui est rattachée à la résine.

Lorsque la totalité de la chaîne peptidique désirée est formée, on élimine les groupes protecteurs des différents acides aminés constituant la chaîne peptidique et on détache le peptide de la résine par exemple à l'aide d'acide fluorhydrique.

L'obtention de rétropeptides cycliques est avantageusement réalisée selon A. Kates et al., Tetrabedron Lett., **34**, 4709, (1993).

A l'inverse, si l'ensemble de ces résidus AA1 à AAn sont de chiralité opposée à celle de ces mêmes résidus dans le peptide parent, alors le rétro analogue obtenu sera un peptide rétro-inverso.

Si un ou plusieurs, mais pas la totalité de ces résidus, sont de chiralité opposée à celle de ces mêmes résidus dans le peptide parent, alors le rétro analogue obtenu sera un peptide totalement rétro-partiellement inverso.

L'obtention de peptides partiellement rétro-inverso, mais aussi de peptides totalement rétro-inverso avec modification des extrémités est avantageusement réalisée à l'aide de la technique classique utilisant les résidus *gem*-diaminoalkyle et les dérivés C-2 substitués de l'acide malonique, décrite notamment dans l'article de Pallai et Goodman (Pallai, P.V., et Goodman, M., (1982), J. Chem. Soc. Chem. Commun., 280-281), et dans le chapitre de Cope et al. ((1957), Org. React., **9**, 107), respectivement.

Dans cette dernière référence, la préparation de dérivés C-2 substitués de l'acide malonique est réalisée par l'alkylation d'un diester de l'acide malonique.

Le monoester C-2 substitué de l'acide malonique peut aussi être obtenu par alcoolyse de dérivés de l'acide de Meldrum (Junek et al., (1976), Synthesis, 333-334; Chorev et al., (1983); J. Med. Chem. 26, 129-135), ces dérivés étant eux-mêmes obtenus par alkylation réductive de l'acide de Meldrum conformément à la méthode décrite par Hrubowchak, D.M., et Smith, F.X. (1983), Tetrahedron Lett., (24), 4951-4954).

S'agissant de la synthèse des dérivés gem-diaminoalkyl décrite par Pallai et Goodman, les résidus gem-diaminoalkyl mono-N-acylés optiquement purs sont obtenus à partir de peptides amides N-protégés, ou de dérivés aminoacyles carboxamides, via un réarrangement d'Hoffman en utilisant un réactif moyennement oxydant: iodobenzène bis(trilluoroacétate) appelé IBTFA ou TIB. Par ailleurs, cette méthode est utilisable en phase solide selon la technique décrite par Pessi et al. (1983), J. Chem. Commun., 195-197.

A titre d'illustration, l'obtention d'un rétropeptide de formule:

...-NH-CH(R1)-CO-NH-CH(R2)-CO-NH-CH(R3)-NH-CO-CH(R4)-CO-NH-CH(R5)-CO-NH-CH(R6)-CO-...

peut être réalisé par condensation du dérivé d'acide aminé P-HN-CH(R2)-COOH, P représentant un groupement protecteur de type uréthane, avec le dérivé carboxamide $H_2N$-CH(R3)-CO-$NH_2$, suivie d'un traitement du dipeptide amide obtenu par IBTFA ce qui conduit à l'obtention d'un résidu gem-diaminoalkyl mono-N-acylé de formule P-HN-CH(R2) -CO-NH-CH(R3)-$NH_2$ puis condensation de ce dérivé avec un monoester C2-substitué de l'acide malonique de formule HOOC-CH(R4)-COOR, -R représentant un groupement protecteur tel que -$CH_3$, -$CH_2CH_3$, -$C(CH_3)_3$ ou -benzyl, ce qui conduit à l'obtention d'un fragment de formule:

P-HN-CH(R2)-CO-NH-CH(R3)-NH-CO-CH(R4)-COOR,

suivie de la déprotection sélective du groupement R, et condensation du fragment dont la fonction acide C-terminale est ainsi déprotégée avec un fragment de formule $H_2N$-CH(R5)-CO-NH-CH(R6)-CO..., obtenu par condensation d'un dérivé d'acide aminé P'-NH-CH(R5)-COOH, P' représentant une protection de type uréthane et un fragment de formule $H_2N$-CH(R6)-CO.., dont l'extrémité C-terminale est protégée, et déprotection sélective de P' ce qui conduit à l'obtention d'un fragment de formule:

P-HN-CH(R2)-CO-NH-CH(R3)-NH-CO-CH(R4)-CO-NH-CH(R5)-CO-NH-

CH(R6)-CO-...,

suivie de la déprotection sélective du groupe protecteur P, et condensation du fragment dont la fonction amine N-terminale est ainsi déprotégée avec un fragment ou un dérivé d'acide aminé de formule ...-NH-CH(R1)-COOH dont l'extrémité N-terminale est protégée, ce qui conduit à l'obtention du dérivé souhaité de formule:

...-NH-CH(R1)-CO-NH-CH(R2)-CO-NH-CH(R3)-NH-CO-CH(R4)-CO-NH-CH(R5)-CO-NH-CH(R6)-CO-...

contenant un résidu gem-diaminoalkyl portant R3 et un dérivé de l'acide malonique portant R4.

Si l'on souhaite obtenir plusieurs liaisons -NH-CO- successives au sein d'un même peptide partiellement rétro-inverso, il suffit d'incorporer selon les techniques classiques en synthèse peptidique, un ou plusieurs résidu(s) aminoacyle(s) de chiralité identique ou opposée à ce(s) résidu(s) aminoacyle(s) dans le peptide parent, entre un résidu gem-diaminoalkyl et un dérivé de l'acide malonique tel que décrits ci-dessus.

A titre d'exemple, le fragment de formule:

P-HN-CH(R2)-CO-NH-CH(R3)-$NH_2$,

dont l'obtention par traitement du dipeptide amide avec IBTFA a été précédemment décrite, peut être condensé avec un dérivé aminoacyle de formule P'-HN-CH(R4)-COOH, ce qui conduit à l'obtention d'un fragment de formule P-HN-CH (R2)-CO-NH-CH(R3)-NH=CO-CH(R4)-NH-P', qui après déprotection sélective du groupement P' peut être condensé avec un monoester C2-substitué de l'acide malonique de formule HOOC-CH(R5)-COOR, -R représentant un groupement protecteur tel que -$CH_3$, -$CH_2CH_3$, -$C(CH_3)_3$, -$CH_2$-CH=$CH_2$ ou -benzyl, ce qui conduit au fragment de formule

P-HN-CH(R2)-CO-NH-CH(R3)-NH-CO-CH(R4)-NH-CO-CH(R5)-COOR,

qui après déprotection sélective du groupement R, et condensation du fragment dont la fonction acide C-terminale est ainsi déprotégée avec un fragment de formule $H_2N$-CH(R6)-CO-...,
ce qui conduit au fragment de formule: P-HN-CH(R2)-CO-NH-CH(R3)-NH-CO-CH(R4)-NH-CO-CH(R5)-CO-NH-CH(R6)-CO-..., qui après déprotection sélective du groupe P peut être condensé avec le fragment ...-NH-CH(R1)-COOH dont l'extrémité N-terminale est protégée, ce qui conduit au dérivé de formule ...-NH-CH(R1)-CO-HN-CH(R2)-CO-NH-CH(R3)-NH-CO-CH(R4)-NH-CO-CH(R5)-CO-NH-CH(R6)-CO-....

Comme précédemment, l'obtention de peptides partiellement rétro-inverso par la méthode décrite ci-dessus, est conditionnée par le choix de la chiralité des résidus aminoacyles qui seront susceptibles d'être reliés à au moins un de leur voisins par une liaison de type -NH-CO- dans l'analogue obtenu.

L'invention a également pour objet les procédés d'obtention de peptides rétro-inverso, totalement rétro-inverso ou totalement rétro-partiellement inverso tels que décrits ci-dessus, et dont les extrémités N- et C- terminales sont modifiées pour mimer davantage les extrémités du peptide parent.

En effet, comme nous l'avons vu dans les exemples de synthèse précédents, partant du peptide parent représenté par la formule:

$H_2N$-CH(R1)CO-NH-CH(R2)-CO.....NH-CH(Rn-1)-CO-NH-CH(Rn)-COOH,

le rétropeptide obtenu après inversion de la totalité des liaisons -CO-NH- est représenté par la formule:

A-CH(R1)NH-CO-CH(R2)-NH.....-CO-CH(Rn-1)-NH-CO-CH(Rn)-B

dans laquelle A et B représentent respectivement HOOC- et $-NH_2$ ou tout autre groupe initialement situé en position N- ou C- terminale du peptide parent si l'analogue est construit sans modification de ses extrémités.

Il est cependant possible de modifier les groupes N- et C- terminaux du rétro analogue, de manière à mimer les extrémités N- et C- terminales du peptide parent

Il est notamment possible d'obtenir des rétro analogues de foxmule indiquée ci-dessus, dans laquelle A représente H-, $H_2N$-, P-HN-, RR'N-, $H_2NCO$-, RR'NCO-, RCO-, et B représente -H, -COOH, -COOR, $-CONH_2$,-CONRR', NHCOR, R, R' et P étant définis comme indiqué à propos de la formule (II) ci-dessus.

A titre d'illustration, l'obtention des rétro analogues pour lesquels A représente $H_2N$-, et B représente -COOH, peut être réalisée en incorporant un dérivé gem-diaminoalkyl ($H_2N$-CH(R1)-NH-) pour mimer l'extrémité N-terminale du peptide parent et en incorporant un dérivé C2-substitué de l'acide malonique (-CO-CH(Rn)-COOH) pour mimer l'extrémité C-terminale du peptide parent selon les méthodes indiquées ci-dessus.

L'obtention de rétro analogues dans lesquels A représente $H_2NCO$- et B représente -NHCOR, R répondant à la définition donnée ci-dessus, peut être réalisée par amidation du COOH de l'acide aminé N-protégé HOOC-CH(R1)-NH-P et par acylation de l'extrémité N-terminale; (à titre d'exemple, une acétylation est réalisée avec de l'anhydride acétique, en présence de DIEA [DIEA = diisopropyléthylamine], ce qui donne pour B: $-NHCOCH_3$).

En combinant les diverses techniques décrites ci-dessus d'introduction de groupes A et B, il est possible d'obtenir toutes les combinaisons possibles de groupes A et B aux extrémités des analogues de l'invention.

A ce titre, des rétro analogues particulièrement préférés dans le cadre de l'invention sont ceux pour lesquels A représente $H_2NCO$- et B représente COOH, obtenus en introduisant une terminaison carboxamide en C-terminale du rétro analogue, et en introduisant à l'extrémité N-terminale du rétro analogue, un dérivé C-2-substitué de l'acide malonique, selon les techniques décrites ci-dessus.

Il convient de préciser que les dérivés de l'acide malonique sont incorporés sous forme de racémique (noté -m(R,S)AAi-), et si l'on désire préserver un centre chiral au niveau de ce malonate dans le peptide, il est nécessaire en fin de synthèse de purifier le mélange des deux diastéréoisomères ainsi obtenus, notamment par chromatographie en phase liquide.

L'invention sera davantage illustrée à l'aide des exemples qui suivent d'obtention de peptides rétro-inverso tels que décrits ci-dessus, ainsi qu'à l'aide de la démonstration de leurs propriétés antigéniques et immunogéniques.

Légendes des figures:

Figure 1: elle concerne la représentation schématique du peptide naturel L-IRGERA et des peptides analogues.

Figure 2: elle concerne le test en ELISA de la réponse immune contre les quatre peptides analogues injectés en

tant que peptides couplés aux SUV dans des souris BALB/c. Les antisérums sont dilués 1 : 500 et testés avec les peptides homologues (A et B), ou avec H3 (protéine parente) (C,D). Le conjugué anti-souris IgG (H + L) révélant les anticorps de souris d'isotypes IgG1, IgG2a et IgG2b (symboles ouverts) et le conjugué anti-IgG3 souris (symboles fermés) sont tous deux dilués 1: 5000. Les résultats représentent les valeurs moyennes d'absorbance obtenues dans chaque groupe de deux souris immunisées avec le peptide IRGERA L (Δ, Δ), le peptide rétro-inverso (○, ●), le peptide-D (□, ■) et le peptide rétro (◈, ♦). Les flèches indiquent le schéma d'immunisation des souris.

Figure 3: elle concerne la résistance à la trypsine des quatre peptides analogues.La trypsine est immobilisés de façon covalente sur des sphères de nylon. Le mélange de digestion incubé pendant différents temps à 25°C est mis à incuber tout d'abord avec l'anticorps Mab 11x2 (4µg/ml) et est ensuite injecté avec le peptide-L immobilisé sur la matrice activée de dextrane du Biacore. Les résultats sont exprimés en fonction du pourcentage d'inhibition de la liaison Mab 11x2 au peptide-L immobilisé par les peptides compétiteurs soumis à la trypsine. La liaison MAb 11x2 au peptide-L en l'absence de peptide inhibiteur correspond à 800 RU. En présence d'un peptide non digéré, la liaison MAb 11x2 au peptide-L est de 220 RU, 240 RU, 260 RU et 250 RU respectivement pour le peptide-L, le peptide rétro-inverso, le peptide-D et le rétro-peptide.

Figure 4: elle concerne la synthèse de l'analogue rétro-inverso de l'épitope C-terminal de la protéine H3: IRGERA.
Séquence du peptide parent:

[H-Cys-Gly-Gly]-Ile-Arg-Gly-Glu-Arg-Ala-OH

Séquence du rétro-analogue:

HO-m(R,S)Ala-DArg-DGlu-Gly-DArg-DIle-[Gly-Gly-Cys-$NH_2$]

Dans cette figure:
a = MBHA résine; b = BOP, HOBT, DIEA/DMF; c = TFA; d = HF
abréviations: MBHA = 4-méthylbenahydyylamine, HOBT = Hydroxy-benzotriazol, TFA = acide trifluoroacétique, HF = Acide fluorhydrique, BOP = hexafluorophosphate de benzotriazolyl-oxy-tris(diméthylamino)phosphonium, DMF = diméthylformamide, DIEA = diisopropyléthylamine.

Figure 5: elle concerne la synthèse en solution d'un analogue rétro-inverso de l'épitope T (56-68) de la matrice de la grippe.
Séquence du peptide parent:

H-GLy-Ile-Leu-Gly-Phe-Val-Phe-Thr-Leu-OH

Séquence de l'analogue rétro-inverso:

HO-m(R,S)Leu-(D)Thr-(D)Phe-(D)Val-(D)Phe-Gly-(D)Leu-gIle-Gly-H

Dans cette figure:
a = BOP, HOBT, DIEA/DMF, b = IBTFA, CH3CN/H2O (1:1), c = TFA, d = NaOH (1N)/MeOH, g = gem-diaminoalkyl.

- Figure 6: réactivité en ELISA d'échantillons de sérum provenant de patients souffrant de LED et de SS avec le peptide parent et l'analogue RIb 277-291 de Ro52. Les sérums des patients sont dilués au 1:1000. Seule l'activité anticorps de sous-classe IgG est mesurée. Les valeurs de densité optique médianes sont indiquées. La ligne en pointillés représente la limite supérieure de population normale correspondant à la valeur de densité optique moyenne de 20 sérums normaux humains + 2 DS (déviation standard) (0.30 unités de DO).

- Figure 7: réactivité en ELISA de deux sérums de patients atteints de LED (A, B) avec le peptide parent L 277-291 de Ro52 (● - ●) et les deux diastéréoisomères RIa (□ - □) et RIb (o - o). Les sérums de patients sont dilués au 1:1000 et laissés à réagir à des concentrations variées de peptide.

- Figure 8: structure schématique du peptide L 304-324 de Ro60. Réaction en ELISA de sérums de 20 patients atteints de LED et de SS avec des analogues du peptide 304-324 de Ro60. Les sérums de patients sont dilués au 1:1000. Seule l'activité IgG est mesurée. Les valeurs de densité optique médianes sont indiquées. Les lignes en pointillés représentent la limite supérieure de la population normale correspondant à la valeur de la densité optique moyenne de 20 sérums normaux humains + 2 DS (déviation standard) (0.30 unités de DO).

- Figure 9: test immuno-enzymatique sur membrane ("dot immuno-essai") du peptide L 304-324 de Ro60 et des analogues peptidiques incubés avec du $^{65}$Zn. Un peptide témoin qui ne contient pas le motif liant du zinc (peptide 56-77 de U1-RNP polypeptide A, Barakat et al., Clin. Exp. Immunol., 86: p. 71-78, 1991) est utilisé comme témoin (bande 1). Des quantités croissantes des 5 peptides analogues et du peptide témoin sont déposées sur des feuilles de nitrocellulose (0.22 μm); les peptides sont incubés avec le $^{65}$Zn comme décrit précédemment (Mazen, A., Menissier-de Murcia, J., Molinete, M., Simonin, F., Gradwohl, G., Poirier, G., et de Murcia, G. (1989), Poly(ADP-ribose) polymerase: a novel finger protein. Nucl. Acids. Res., 17: 4689-4698; Muller, S., Briand, J.P., Barakat, S., Lagueux, J., Poirier, G.G., De Murcia, G., et Isenberg, D.A. (1994) Autoantibodies reacting with poly(ADP-ribose) and with a zinc-finger functional domain of poly(ADP-ribose) polymerase involved in the recognition of damaged DNA. Clin. Immunol. Immunopathol., 73: 187-196). Les feuilles de nitrocellulose sont autoradiographées pendant 5 heures (A) et 14 heures (B) à -70°C. Bandes 2, 2': peptide L: bandes 3,3': peptide RI; bandes 4,4': D-allo-Ile peptide RI; bande 5: peptide L bloqué; bandes 6,6': peptide RI bloqué. Les résultats proviennent de deux expériences indépendantes (A et B).

La figure 10 représente la liste des peptides synthétisés pour les tests d'immunomodulation thérapeutique.

La figure 11 représente de façon schématique le principe de la détection de l'assemblage HLA-peptide.

La figure 12 rassemble les résultats de fixation sur la molécule HLA-A2. Les peptides sont désignés en abscisses et les unités de fluorescence en ordonnées. Les barres pleines correspondent à des concentrations en peptide de 10 μg/ml et les barres hachurées à des concentrations en peptide de 1 μg/ml.

La figure 13 correspond à la mesure de la cytolyse (exprimée en %) (en ordonnées) en fonction du rapport en nombre de cellules effecteur/cible (en abscisses), dans le cadre du test décrit dans Martinon *et al*., Eur. J. Immunol. 1990, **20**, 2071-2176. La courbe avec des triangles correspond au peptide GG7, celle avec des carrés au peptide GG10, celle avec des ronds pleins au peptide M58-66 et celle avec des ronds vides au peptide GG0a.

**Exemple 1**

**Matériels et Méthodes**

Histone H3 et peptides

L'histone H3 d'érythrocyte de poulet est isolée et purifiée comme décrit précédemment (Van der Westhuyzen, D.R., & Von Holt, C. (1971), FEBS Lett., **14**, 333-337).

Trois analogues des peptides modèles de séquence IRGERA correspondant aux résidus COOH-terminal 130 à 135 de l'histone H3 sont préparés. La préparation et la purification des peptides L et D IRGERA ont été décrites précédemment (Benkirane et al., (1993), J. Biol. Chem., **268,** 26279-26285). Les deux nouveaux analogues, les peptides rétro-inverso et les peptides rétro sont synthétisés de la même façon que les L- et D-peptides, par la méthode en phase solide sur un synthétiseur de peptides multi-canaux (Neimark, J. & Briand, J.P., (1993), Peptide Res., **6**, 219-228). Les isomères rétro-inverso et rétro modifiés aux extrémités terminales sont assemblés en utilisant le groupe protecteur Boc sur une résine p-méthyl benzhydrylamine (Applied Biosystem, Roissy, France). L'assemblage de la chaîne peptidique protégée est effectué à l'échelle de 200 μmoles en utilisant le protocole de neutralisation *in situ* décrit précédemment (Neimark, J. & Briard, J.P., (1993), Peptide Res., **6**, 219-228). L'ester monobenzylique de l'acide (R-S)-2-méthyl malonique obtenu par alcoolyse de 2,2,5-trinnéthyl-1,3-dioxane-4,6-dione (Chorev et al., (1983), J. Med. Chem., **26**, 129-135) est incorporé dans la chaîne peptidique sous forme de racémique. Le couplage est contrôlé par le test à la ninhydrine. Après ce dernier couplage, le peptide-résine est lavé deux fois avec de l'éther et séché sous-vide dans un dessicateur. Les peptides sont clivés de la résine par traitement avec HF anhydre contenant 10% (v/v) d'anisol et 1% (v/v) de 1,2-éthanedithiol. Après élimination de HF sous-vide, les peptides sont extraits de la résine et lyophilisés. Les peptides bruts sont ensuite purifiés sur une colonne C18 en utilisant un appareil de pression moyenne (Kronwald Separation Technology, Sinsheim, FRG), par élution avec un gradient linéaire de 5 à 50% (v/v) d'acétonitrile, dans 0.06% d'acide trifluoroacétique aqueux. La pureté de chaque fraction est déterminée par passages analytiques sur une colonne de Novapak C18 de 5μm (3.9 x 150 mm), en utilisant un gradient linéaire de 7 à 32% (v/v) d'acétonitrile dans un tampon phosphate aqueux de triéthylammonium 0.1 M. Les passages sont effectués avec un appareil Waters (Waters Corporation, Milford, MA). Les fractions contenant le mélange pur de diastéréoisomères sont recueillies et

lyophilisées. Les spectres de masse sont obtenus sur un instrument à double concentration ZAB-2SE analytique VG et enregistrés sur un système de données VG 11-250 (VG Analytical, Manchester, UK) comme décrit (Briard et al., (1989), Peptide Res., **2,** 381-388). Les mesures de dichroisme circulaire sont effectuées comme décrites dans (Benkirane et al., (1993), J. Biol. Chem., **268,** 26279-26285).

Conjugaison support peptide

Afin de mettre les peptides sur une plaque pour effectuer un test ELISA-direct en phase solide, les analogues IRGERA sont d'abord conjugués à la BSA en utilisant du N-succinimidyl 3-[2-pyridyl dithio] propionate (SPDP) comme décrit précédemment (14). Pour l'immunisation des souris, les peptides sont couplés de façon covalente à des petits liposomes unilaméllaires (SUV) préformés contenant du 4-(p-maleimidophényl) butyrylphosphatidyl-éthanolamine (MBP-PE). Le monophosphoryl lipide A (MPLA) est incorporé dans les SUV comme adjuvant (Benkirane et al., (1993), J. Biol. Chem., **268**, 26279-26285; Friede et al., (1993), Molec. Immunol., **30**, 539-547).

Antisérums et anticorps monoclonaux

Les antisérums sont obtenus en immunisant des souris BALB/c avec des peptides associés à des liposomes comme décrit dans (Benkirane et al., (1993), J. Biol. Chem., **268**, 26279-26285). On injecte dans les souris par voie intrapéritonéale les différentes préparations de SUV contenant 1 µmol de lipide, 2 µg de MPLA, et 60 µg de peptide par injection par animal.
Les souris reçoivent trois injections à des intervalles de 3 semaines et elles sont saignées 5 jours après chaque injection, puis régulièrement pendant les 6 mois suivant la denaière injection. Le sérum témoin est prélevé de chacune des souris avant la première injection. Pour la préparation d'anticorps monoclonaux (Mab), on injecte les souris BALB/c 3 fois à des intervalles de 3 semaines avec des peptides L et D (100µg) liés de façon covalente aux SUV contenant du MPLA. Des injections de rappel sont faites aux jours 105, 106, 107 et 108, c'est-à-dire -4, -3, -2 et -1 avant la fusion. Les anticorps monoclonaux des peptides L et D sont préparés par des protocoles de fusion standard (16). Les descriptions détaillées de la génération et de la caractérisation des anticorps monoclonaux sont données ailleurs (N. Benkirane, G. Sommermeyer and S. Muller, Molecular Immunology, Vol. 24, N° 7, pp. 779-789, 1987).
En résumé, la production de ces trois anticorps monoclonaux a été réalisée de la manière suivante:

- Immunisation des souris BALB/c : voir ci-dessus,
- fusion proprement dite:
  - . peptide L: 2 x $10^7$ lymphocytes de la souris immunisée + 2 x $10^7$ cellules myélomateuses [PAI, ref. dans Muller et al., 1987]
  - . peptide D: 3.2 x $10^7$ lymphocytes + 3.5 x $10^7$ PAI 2 sous clonages successifs pour les deux fusions.
  - . Résumé du bilan de fusion:

| Peptide clones totaux (%) | Nbre de Nbre de clones positifs (%) | | 1e sous-clonage | 2e sous-clonage | Production en ascite |
|---|---|---|---|---|---|
| L(4) | 120/195 puits (62.5%) | 30/120 (25%) | 24 | 21 | 8 |
| D(11) | 155/192 puits (80.7%) | 30/155 (19.3%) | 16 | 14 | 7 |

6 clones ont été mis en culture à grande échelle pour obtenir plusieurs dizaines de mg d'Ac. 41.6 mg de l'Ac 11 x 2, 80 mg de l'Ac 11 x 7 et 62 mg de l'Ac 4 x 11 ont ainsi été produits et purifiés.
La réactivité de trois anticorps monoclonaux 4x11 (dérivé d'une souris immunisée contre le peptide L), 11x2 et 11x7 (dérivés d'une souris immunisée contre le peptide D), est décrite ci-après.
Par ailleurs, deux autres anticorps monoclonaux 4x8 et 4x10 ont été également obtenus et on a mesuré la constante d'affinité de ces anticorps vis à vis du peptide L- IRGERA et rétro-inverso IRGERA. Les résultats sont donnés ci-après (tableau 2).

ELISA

La procédure ELISA (test de liaison directe et de compétition) est comme décrite dans Benkirane et al, (1993) J. Biol. Chem. **268,** 26279-26285.

Analyse cinétique de la liaison des anticorps monoclonaux

Pour des expériences de liaison en temps réel, un système de biosenseur BIAcore (Pharmacia Biosensor, AB, Uppsala, Sweden) est utilisé. Les réactifs y compris les surfaces de réaction (dexiran) CM5, le tensioactif P20 et le kit de couplage contenant du N-hydroxysuccinimide (NHS), du N-éthyl-N'-(3-diméthylaminopropyl) carbodiimide (EDC), de la 2-(2-pyridinyldithio)éthane amine (PDEA) et de l'éthanolamine-HCl sont obtenus de Pharmacia Biosensor AB. Pour immobiliser les peptides sur la surface de réaction (dextran), le protocole standard décrit précédemment (17,18) est utilisé à l'exception du fait que la surface de dextran est d'abord activée par le mélange NHS-EDC, puis modifiée en injectant l'agent de couplage PDEA thiol (15 µl de 80 mM de PDEA dans du tampon borate 0,1M, pH 8.5) en laissant le groupe réactif thiol des peptides analogues se coupler à la matrice activée par une réaction d'échange thio-disulfure. Après le passage d'immobilisation du peptide, les groupes réactifs restant sur la surface de réaction sont désactivés par un flux de 4 min de cystéine 50 mM dans NaCl, 1 M, pH 4.3.

Les trois anticorps monoclonaux 4x11, 11x2 et 11x7 sont ensuite mis à interagir avec les surfaces de réaction, sur lesquelles les quatre analogues différents du peptide ont été immobilisés. Six à dix concentrations de chaque anticorps monoclonal allant de 50 à 800 nM dans HBS pH 7.4 (10 mM HEPES, 150 mM NaCl, 3,4 mM EDTA, 0.05% de tensioactif P20) sont utilisés dans chaque test. La préparation d'anticorps est injectée à une vitesse de flux constant de 3 µl par min pendant 7 min à 25°C et les points de report de calcul sont ensuite pris toutes les 10 secondes pendant 5 min en commençant 1.30 min après la fin de l'injection de l'anticorps monoclonal (Zeder et al., (1993), Molec. Recogn., **6**, 71-79).

Des essais de compétition sont effectués comme décrit précédemment (Zeder et al., (1993), Molec. Recogn., **6**, 71-79). Les anticorps monoclonaux 4x11, 11x2 et 11x7 (200 nM) sont incubés avec les peptides compétiteurs utilisés en excès molaire de 1.75 à 800 par rapport à l'anticorps.

Les constantes cinétiques des anticorps sont mesurées comme décrit (Zeder et al., (1993), Molec. Recogn., **6**, 71-79). La théorie de mesures cinétiques en utilisant le système Biosensor BIAcore a été décrite précédemment (Zeder et al., (1993), Molec. Recogn., **6,** 71-79; Altschuh et al. (1992), Biochemistry, **31,** 6298-6304).

Résistance à la trypsine

La résistance des quatre analogues de peptides à la trypsine est testée en utilisant l'enzyme protéolytique immobilisé de façon covalente sur des sphères de nylon de 3.2 mm de diamètre (Michalon et al., (1993), Eur. J. Biochem., **216,** 387-394). L'activité spécifique des sphères enzymatiques est équivalente à 18 nmol de p-toluènesulfonyl-L-arginine méthylester hydrolysées par minute par sphère de nylon. La digestion par la protéase est déclenchée en immergeant 15 sphères enzymatiques dans 1 ml d'une solution de peptide à 150 µg/ml maintenue à 25°C sous agitation constante. La digestion est effectuée dans HBS pH 7.4 pendant 5 à 240 min. La réaction est arrêtée en retirant les sphères enzymatiques. Le clivage des peptides est évalué par voie immunochimique. On laisse tout d'abord les mélanges de digestion incuber à 25°C avec le Mab 11x2 (200 nM) pendant 30 min puis on les expose au peptide L immobilisé sur la surface de réaction activée comme décrit précédemment. Dans cet essai, si le peptide en solution est résistant à la trypsine, il entre en compétition avec la liaison du MAb 11x2 au peptide L immobilisé sur la surface dextran activé comme décrit ci-dessus et le signal, exprimé en unités de résonance (RU) est faible. Inversement, si le peptide est dégradé par la trypsine, il n'y a pas de compétition et le signal est équivalent au témoin sans peptide de compétition.

**Résultats**

Peptides synthétiques

Quatre peptides sont utilisés dans cette étude (Figure 1). Le peptide parent IRGERA correspond à l'extrémité COOH terminale de l'histone H3, qui a été étudiée précédemment (Friede et al., (1993) Molec. Immunol., **30**, 539-547; Muller et al., (1982), EMBO J., **1,** 421-425; Briand et al., (1992), J. Immunol. Meth., **156,** 255-265 ). Une cystéine et deux résidus supplémentaires de glycine sont ajoutés à l'extrémité NH2 terminale pour permettre la conjugaison sélective des peptides aux liposomes ou à la BSA, et pour accroître l'accessibilité des peptides liés au support.

La Figure 1 représente la structure du peptide L et les trois analogues structuralement associés utilisés dans cette étude, c'est-à-dire l'énantiomère D (Benkirane et al., (1993), J. Biol. Chem. **268,** 26279-26285), les analogues

rétro-inverso et rétro modifiés aux extrémités. L'analogue rétro est obtenu en remplaçant les résidus normaux d'acides aminés L par les résidus correspondants D et en inversant la direction de la chaîne peptidique. Ceci entraîne le maintien de la topochimie des chaînes latérales, ce qui signifie que l'orientation spatiale originelle de toutes les chaînes latérales se trouve maintenue (Goodman, M. et Chorev, M. (1979), Acc. Chem. Res., **12,** 1-7). Dans le cas de l'analogue rétro, la chaîne peptidique est inversée mais la chiralité des acides aminés dans la séquence est retenue ce qui entraîne une non complémentarité de la chimie des chaînes latérales entre cet analogue et le peptide parent. Ce rétroanalogue est ainsi topochimiquement associé à l'énantiomère D. Cependant, dans de tels peptides linéaires, les deux paires de peptides topochimiquement associés ne possèdent pas les mêmes groupements aux extrémités et leurs charges ne sont pas complémentaires. Pour résoudre ce problème, un résidu *gem*-diaminoalkyl peut être introduit du côté amino-terminal et un acide malonique-2-substitué à l'extrémité carboxy terminale (Goodmann, M. et Chorev, M. (1979), Acc. Chem. Res., **12,** 1-7). Cependant, les *gem*-diaminoalkyls monoacylés sont hydrolysés et on devrait s'attendre à ce que la demie vie des peptides incorporant de tels résidus soit de 10 à 50 heures à 25°C (Loudon et al. (1981), J. Am. Chem. Soc., **103,** 4508-4515). Par conséquent, et parce que la cystéine NH2-terminale ne fait pas partie de l'épitope, on a choisi d'utiliser une extrémité carboxamide. L'acide (R,S)-2-méthylmalonique est incorporé dans les peptides sous forme de racémique générant ainsi une paire de diastéréoisomères. Les deux diastéréoisomères des deux analogues rétro et rétro-inverso sont identifiés par HPLC analytique 6.35 et 6.64 min pour le rétro-inverso et 6.50 et 6.78 min pour le rétro, mais la séparation n'est pas suffisante pour permettre aux diastéréoisomères d'être purifiés. Les mélanges des diastéréoisomères sont considérés comme étant purs sur la base de l'analyse HPLC 87% pour le rétro-inverso et 86% pour le rétro et la spectroscopie de masse $(M+1)^+$: 946.4.

L'ellipsité négative trouvée à 198 nm dans le spectre CD du L-peptide et des rétropeptides indique une forme non ordonnée. Comme mentionné précédemment pour le peptide-L parent et son énantiomère D (Benkirane et al., (1993), J. Biol. Chem., **268**, 26279-26285), les spectres CD des analogues rétro et rétro-inverso sont des images par rapport à un miroir.

<u>Anticorps polyclonaux vis à vis des analogues d'IRGERA</u>

Des groupes de deux souris BALB/c sont injectés avec les quatre analogues d'IRGERA conjugués aux liposomes. La réaction des anticorps vis à vis des quatre peptides avec les analogues peptidiques et avec H3 est mesurée dans un test d'ELISA direct. Dans ce test, H3 et les quatre analogues peptidiques conjugués à BSA au moyen du SPDP sont utilisés pour recouvrir les plaques. Comme représenté sur les Figures 2A et 2B, une forte réponse anticorps vis à vis des quatre peptides est obtenue dans les souris immunisées. Dans le cas du peptide L, les anticorps appartiennent aux sous-classes IgG1, 2a et 2b. La réponse anticorps IgG3 apparaît légèrement plus tard (saignée 4) que la réponse anticorps IgG1, 2a et 2b (saignée 2). Dans le cas des peptides rétro-inverso, D et rétro, la réponse anticorps IgG3 est prédominante. La plupart des sous-classes d'anticorps réagissent de façon croisée avec l'histone H3 parente (Figures 2C et 2D) à l'exception des anticorps IgG1, 2a et 2b dirigés contre le peptide D et le peptide rétro (Figure 2D), qui montrent très peu de réaction avec H3. Il faut noter que bien que la réponse d'anticorps IgG3 vis à vis du peptide rétro-inverso et du peptide rétro soit particulièrement forte, la durée de la réponse anticorps est similaire à celle induite contre les peptides L et D.

Les anticorps d'isotypes IgG1, 2a et 2b obtenus dirigés contre le peptide L et le peptide rétro-inverso reconnaissent à la fois le peptide L et le peptide rétro-inverso ainsi que l'histone H3 mais pas le peptide D et le peptide rétro. Inversement, les anticorps d'isotypes IgG1, 2a et 2b obtenus contre le peptide D et le peptide rétro reconnaissent à la fois le peptide D et le peptide rétro, mais ni le peptide L ni le peptide rétro-inverso. Les anticorps d'isotype IgG3 induits contre les quatre peptides réagissent de façon croisée également bien avec les quatre analogues de peptides et avec H3. L'immunoréactivité des conjugués de peptide est confirmée dans un test de liaison par compétition avec les peptides libres en solution (Tableau 1).

De façon générale, les résultats obtenus avec les deux types de tests (conjugués BSA-peptides adsorbés ou peptides libres) indiquent que:

**1)** les anticorps d'isotype IgG peuvent être obtenus facilement contre le peptide rétro et le peptide rétro-inverso;
**2)** s'agissant des immunorétroïdes et du peptide D, une forte réponse IgG3 est produite;
**3)** le peptide rétro-inverso IRGERA, mime bien le peptide L naturel mais ni le peptide D ni le peptide rétro, tandis que le peptide rétro mime bien le peptide D mais ni le peptide L ni le peptide rétro-inverso.

<u>Anticorps monoclonaux vis à vis des analogues L- et D-IRGERA</u>

Plusieurs expériences de fusion sont effectuées avec des cellules de rate de souris BALB/c immunisées avec les différents analogues d'IRGERA décrits précédemment (9). De l'ensemble des anticorps monoclonaux qui sont obtenus, on a choisi trois anticorps sur la base de leur réactivité en ELISA avec les quatre analogues de peptides. Le

Mab 4x11 (IgG1) est généré à partir de splénocytes d'une souris immunisée avec le peptide L; il réagit en ELISA avec le peptide L et le peptide rétro-inverso mais pas avec le peptide D et seulement faiblement avec le peptide rétro. Les anticorps monoclonaux 11x2 et 11x7 (tous les deux IgG3) sont générés à partir des splénocytes d'une souris immunisée avec le peptide D; ils réagissent en ELISA avec les quatre analogues IRGERA, ainsi qu'avec l'histone H3 parente.

La liaison de ces anticorps aux quatre analogues de peptides est mesurée dans un BIAcore en utilisant des peptides liés de façon covalente à la matrice de dextrane à travers leur groupe SH libre. Les constantes cinétiques et les constantes d'affinité à l'équilibre des trois anticorps monoclonaux pour les quatre analogues de peptides sont rassemblées dans le tableau 2. Les constantes d'affinité à l'équilibre (Ka) de MAbs 4x11, 11x2 et 11x7 vis à vis des peptides homologues sont $3x10^6$ $M^{-1}$, $1.3 \times 10^{10}$ $M^{-1}$ et $1.2x10^7$ $M^{-1}$, respectivement (Tableau 2). Il faut noter que les deux anticorps monoclonaux 4x11 et 11x7 montrent une valeur de Ka qui est au moins 50 fois plus faible pour le peptide homologue que pour un peptide hétérologue, à savoir le peptide rétro-inverso dans le cas de l'anticorps monoclonal 4x11 et le peptide réiro dans le cas de l'anticorps monoclonal 11x7 (Tableau 2). L'anticorps monoclonal 11x2 réagit avec les quatre peptides. Cependant, en comparaison avec l'affinité de l'anticorps vis à vis du peptide homologue, les valeurs de Ka sont 30 fois plus faible pour le peptide rétro hétérologue et $10^3$-$10^4$ plus faible pour le peptide L et le peptide rétro-inverso (Tableau 2).

Afin de confirmer la différence dans l'immunoréactivité des différents analogues peptidiques, des essais d'inhibition avec les trois anticorps monoclonaux sont effectués dans le système BIAcore. On laisse réagir les trois anticorps monoclonaux préincubés avec des concentrations variées des quatre analogues peptides avec les peptides homologues immobilisés sur la surface du SENSOR. Comme indiqué dans le Tableau 3, la liaison de l'anticorps monoclonal 11x2 aux peptides L et D est inhibée par les quatre analogues peptidiques (50% d'inhibition de la liaison de l'anticorps observée avec 0.5 à 25 excès molaires des différents peptides vis à vis des anticorps). La liaison de l'anticorps monoclonal 4x11 au peptide L est inhibée seulement par les peptide libres L et rétro-inverso, tandis que la liaison de l'anticorps 11x7 au peptide D est inhibée seulement par le peptide D et le peptide rétro libres. Ces résultats sont ainsi en complet accord avec ceux obtenus dans les tests ELISA et le test direct en BIAcore décrit ci-dessus. Sur le plan quantitatif cependant, lorsque la réactivité des anticorps avec les quatre peptides analogues est comparée en ELISA et dans le système BIAcore, dans quelques cas on trouve une faible corrélation (Tableau 4). Par exemple, des valeurs similaires de densité optique (DO) sont obtenues en ELISA lorsque l'anticorps monoclonal 11x2 est mis à réagir avec les peptides L et D, tandis que les valeurs de Ka mesurées dans le système biosensor présentent une différence de 4 logs.

Anticorps polyclonaux anti H3 de lapin vis à vis des analogues D, RI IRGERA et du peptide parent.

Le tableau 5 montre que les anticorps anti-protéine parente (histone H3) sont capables de reconnaître de façon identique en ELISA le peptide IRGERA parent et son analogue, le peptide RI. Ce résultat ouvre toutes les possibilités d'utilisation des immunorétroïdes en diagnostic dans tous les cas où l'on veut détecter des anticorps contre une protéine exogène (par exemple en virologie, microbiologie) ou endogènes (par exemple autoimmunité, maladies neurogénératives).

Nous avons vu précédemment que des anticorps monoclonaux anti-peptide parent peuvent reconnaître le peptide RI avec une affinité beaucoup plus élevée que celle mesurée dans le cadre de la reconnaissance du peptide parent par lesdits anticorps monoclonaux (voir tableau 2, anticorps monoclonal Mab 4x11), ce qui peut permettre d'améliorer dans certains cas le test de diagnostic, par exemple en utilisant des anticorps, notamment monoclonaux, anti-RI pour "piquer" la protéine recherchée dans un mélange biologique.

Résistance des analogues de peptide à la trypsine

Un des avantages potentiels de l'utilisation des analogues pepüdiques contenant des résidus d'amino acides D ou des liaisons peptidiques inversées, réside dans leur plus forte résistance à la protéase, ce qui pourrait accroître leur immunogénicité en comparaison des peptides naturels. En utilisant la rubredoxine radioactive Dintzis et al. (Dintzis et al., (1993), Proteins, **16**, 306-308) ont montré par exemple que la forme D de la protéine était retenue au moins 4 fois plus longtemps dans l'organisme que la forme L. Wade et al. (Wade et al., (1990), Proc. Natl. Acad. Sci., USA, **87,** 4761-4765) ont montré que les énantiomères D de plusieurs antibiotiques peptidiques sont résistants à la dégradation enzymatique. On a testé ici la résistance des analogues de peptides à la trypsine dont la spécificité est fondée sur les chaînes latérales d'arginine et de lysine positivement chargées. On a utilisé l'enzyme immobilisé de façon covalente sur des sphères de nylon et mesuré la capacité résiduelle de peptides protéolysés à entrer en compétition avec le peptide L pour la liaison de l'anticorps monoclonal 11x2 dans le BIAcore. L'anticorps monoclonal 11x2 est utilisé dans cet essai parce qu'il reconnaît les quatre analogues en solution (Tableau 3). L'avantage de l'utilisation de l'enzyme immobilisé sur des sphères de nylon réside dans sa plus grande stabilité, l'absence d'adjonction d'un agent bloquant l'action de la protéase pour terminer la réaction et une maîtrise expérimentale totale de l'expérience (Michalon

et al., (1993), Eur. J. Biochem., **216,** 387-394). Comme représenté sur la Figure 3, le peptide L est rapidement digéré dans ces conditions. La moitié de son activité antigénique est perdue après 7 min et aucune activité ne reste après 10 min. Le peptide rétro est légèrement moins sensible (demie vie: 9 juin, complète perte d'activité immunologique 40 min), tandis que les peptide D et rétro-inverso sont beaucoup plus résistants à la trypsine (demie vie: 73 et 67 min respectivement, complète perte d'activité non encore observée après 240 min).

TABLEAU I

| Reconnaissance en test de compétition d'ELISA du peptide IRGERA et analogues d'IRGERA par les anticorps de souris induits contre les peptides homologues et analogues | | | | |
|---|---|---|---|---|
| Antigènes utilisés comme inhibiteurs | Excès molaire du peptide inhibiteur requis pour inhiber 50% de la liaison entre les anticorps anti-peptide et les antigènes homologues | | | |
| | anti-L | anti-RI | anti-D | anti-R |
| Réponse IgG1, 2a, 2b | | | | |
| peptide L | 10 | 80 | -* | - |
| peptide RI | 10 | 10 | - | - |
| peptide D | - | - | 10 | 20 |
| peptide R | - | - | 5 | 10 |
| H3 | 5 | 18 | - | - |
| Réponse IgG3 | | | | |
| peptide L | 10 | 50 | 5 | 70 |
| peptide RI | 50 | 10 | 10 | 70 |
| peptide D | 10 | 70 | 5 | 30 |
| peptide R | 5 | 70 | 50 | 10 |
| H3 | 5 | 60 | 5 | 90 |

Des plaques de microtitration sont recouvertes avec 2 µM de peptide conjugué à la BSA à l'aide de SPDP (rapport molaire du peptide au porteur 1:10), et incubées avec des antisérums de souris dirigés contre le peptide homologue (dilution de sérum 1: 500) et préincubées avec les différents peptides et avec H3 utilisé en tant qu'inhibiteur. Un peptide témoin correspondant à la séquence 149-158 de la protéine du virus de la mosaïque du tabac est utilisé en tant que référence interne et n'a aucun effet sur la liaison avec les anticorps.

Les conjugués anti IgG de souris couplés à la peroxydase sont tous deux dilués 1 : 5000.

Les excès molaires du peptide inhibiteur sont exprimés en fonction du peptide déposé dans les puits des plaques de microtitration et sont calculés comme décrit (Benkirane et al., (1993), J. Biol. Chem., **268**, 26279-26285).

***** -, réactivité croisée non détectable (jusqu'à 125 µg/ml de peptide compétiteur)

L = peptide L parent, RI = peptide rétro-inverso, D = peptide D, R = peptide rétro.

TABLEAU 2

| Constantes cinétiques et constantes d'affinité à l'équilibre des Mab 4x11, 11x2 et 11x7 pour les quatre analogues IRGERA, et 4x8 et 4x10, pour L-IRGERA et RI-IRGERA. | | | | |
|---|---|---|---|---|
| MAbs | peptide utilisé comme antigène | ka (x$10^3$) ($M^{-1}s^{-1}$) | kd(x$10^{-5}$) ($s^{-1}$) | Ka(x$10^6$) ($M^{-1}$) |
| 4x11 (IgG1) (anti-peptide L) | L | 3 ± 0.2 100 ± 0.3 | | 3 |
| | RI | 18 ± 0.5 | 8± 0.2 | 225 |
| | D | nb* | - | - |
| | R | nb | - | - |

* nb, aucune liaison

TABLEAU 2 (suite)

| Constantes cinétiques et constantes d'affinité à l'équilibre des Mab 4x11, 11x2 et 11x7 pour les quatre analogues IRGERA, et 4x8 et 4x10, pour L-IRGERA et RI-IRGERA. | | | | |
|---|---|---|---|---|
| MAbs | peptide utilisé comme antigène | ka (x10$^3$) (M$^{-1}$s$^{-1}$) | kd(x10$^{-5}$) (s$^{-1}$) | Ka(x10$^6$) (M$^{-1}$) |
| 11 x2 (IgG3)L (anti-peptide D) | L | 2 ± 0.3 100 ± 0.2 | | 2 |
| | RI | 13 ± 0.6 | 160± 0.3 | 8 |
| | D | 130 ± 0.3 | 1 ± 0.4 | 13 000 |
| | R | 5 ± 0.5 | 1 ± 0.3 | 500 |
| 11x7 (IgG3) (anti-peptide D) | L | nb | - | - |
| | RI | nb | - | - |
| | D | 3 ± 0.4 | 25 ± 0.5 | 12 |
| | R | 6 ± 0.6 | 1 ± 0.4 | 600 |
| 4x8 (IgG1) (anti-peptide L) | L | 13 ± 0.2 | 15 ± 0.2 | 80 |
| | RI | 419 ± 0.4 | 75 ± 0.5 | 558 |
| 4x10 (IgG1) (anti-peptide L) | L | 987 ± 0.7 | 22 ± 0.2 | 4486 |
| | RI | 910 ± 0.5 | 25 ± 0.4 | 3640 |

Les constantes du taux d'association (ka) et de dissociation (kd) sont les valeurs moyennes obtenues dans deux à quatre expériences indépendantes.

L = peptide L parent; RI = peptide rétro-inverso; D = peptide D; R = peptide rétro.

TABLEAU 3

| Reconnaissance en essais de compétition dans le système BIAcore du peptide IRGERA et des analogues d' IRGERA par les anticorps MAb 4x11, 11x2 et 11x7 | | | | |
|---|---|---|---|---|
| Peptides utilisés comme inhibiteurs | Excès molaires du peptide inhibiteur requis pour inhiber 50% de la liaison entre les MAb et | | | |
| | peptide-L | | peptide-D | |
| | MAb4x11 | MAb11x2 | MAb11x2 | MAb11x7 |
| L | 1 | 1 | 10 | -* |
| RI | 1 | 3 | 15 | - |
| D | - | 1 | 10 | 10 |
| R | - | 0.5 | 25 | 10 |

L = peptide L parent, RI = peptide rétro-inverso, D = peptide D, R = peptide rétro. les excès molaires de l'inhibiteur sont exprimés par rapport à l'anticorps (200 nM MAb).

*-, réactivité croisée non détectable (jusqu'à 250 µg/ml de peptide compétiteur).

TABLEAU 4

| Réactivité en ELISA des MAb 4x11, 11x2 et 11x7 avec les quatre analogues IRGERA. Constantes d'affinité à l'équilibre des trois MAb pour les quatre analogues. | | | | | |
|---|---|---|---|---|---|
| MAb | Tests | Réactivité avec les peptides | | | |
| | | L | RI | D | R |
| 4x11 | ELISA (DO) | 1.29 | 1.12 | 0.19 | 0.40 |
| | BIAcore (Ka $M^{-1}$) | $3x10^6$ | $2x10^8$ | nb* | nb |
| 11x2 | ELISA (DO) | 2.75 | 1.78 | 2.92 | 1.92 |
| | BIAcore(Ka $M^{-1}$) | $2x10^6$ | $8x10^6$ | $1x10^{10}$ | $5x10^8$ |
| 11x7 | ELISA (DO) | 0.47 | 0.89 | 1.45 | 0.78 |
| | BIAcore (Ka $M^{-1}$) | nb | nb | $1x10^7$ | $6x10^8$ |

* nb, aucune liaison.

Pour l'ELISA, les plaques de microtitration sont recouvertes avec 2 µM du peptide conjugué à la BSA à l'aide de SPDP et incubées avec les MAb (4µg/ml). Les conjugués anti-IgG de souris - peroxydase sont dilués 1: 5000.

L = peptide L parent, RI = peptide rétro-inverso, D = peptide D, R = peptide rétro.

TABLEAU 5

| Réactivité d'anticorps anti-histone H3 ( protéine parente) induits chez le lapin avec les peptides IRGERA L, D et RI | | | | | |
|---|---|---|---|---|---|
| Antigènes (peptides testés) | Dilution du sérum anti-H3 | Antisérums anti-H3 | | | Sérum normal de lapin |
| | | lapin 1 | lapin 2 | lapin3 | |
| PEPTIDE L | 1 : 1000 | 2.71[(1)] | 2.29 | 1.98 | 0.06 |
| | 1 : 2000 | 1.61 | 1.45 | 1.10 | 0.03 |
| | 1 : 4000 | 0.92 | 0.88 | 0.57 | 0.02 |
| PEPTIDE RI | 1 : 1000 | 2.79 | 2.39 | 1.95 | 0.08 |
| | 1 : 2000 | - | 1.38 | 1.15 | 0.15 |
| | 1 : 4000 | 0.92 | 0.91 | 0.71 | 0.15 |
| PEPTIDE D | 1 : 1000 | 0.04 | 0.02 | 0.02 | 0.03 |
| | 1 : 2000 | 0.03 | 0.02 | 0.01 | 0.05 |
| | 1 : 4000 | 0.18 | 0.01 | 0.01 | 0.04 |

(1) Valeurs de la densité optique à 450 nm.

**Exemple 2**

**Peptides rétro-inverso et diagnostic.**

Pendant la dernière décade, les immuno-essais en phase solide, tels que le test ELISA et les tests radioimmunologiques en phase solide sont devenus de plus en plus courants, et ces tests sont maintenant largement utilisés pour déterminer l'activité antigénique de peptides synthétiques à la fois à des fins de diagnostic et expérimentales. En particulier, un certain nombre d'immuno-essais basés sur l'utilisation de peptides synthétiques pour la détection et la quantification d'auto-anticorps présents dans les sérums de patients atteints de maladies auto-immunes ont été développés (Elkon, K.B., (1992) Use of synthetic peptides for the detection and quantification of autoantibodies. Molec. Biol. Rep., 16: 207-212; Muller, S. (1994), Use of synthetic peptides for the analysis of B-cell epitopes in autoantigens, In: M. Zouali (Ed.) Autoimmunity: Experimental Aspects. Springer-Verlag, Berlin, Heidelberg, p. 76-90). Il est important

de souligner que pendant les tests, les peptides utilisés, soit libres en solution, soit adsorbés directement sur des plaques de microtitration, peuvent être altérés par les protéases qui sont présentes dans les sérums de patients. L'une des caractéristiques de la réaction inflammatoire aiguë est en effet la libération d'enzymes protéolytiques variées, conduisant à une lésion des tissus et la libération ultérieure d'autres protéases (Kaplan, A.P. et Silverberg, M. (1988) Mediators of inflammation: an overview. Meth. Enzymol., 163: 3-23). Dans les maladies auto-immunes telles que la polyarthrite rhumatoïde ou le lupus érythémateux disséminé (LED), la dégradation du tissu inflammatoire peut aussi être causée par des complexes immuns circulants (Levinson, S.S. (1994), Humoral mechanisms in autoimmune disease. J. Clin. Immunoassay, 17: 72-84). La dégradation protéolytique des peptides peut être contournée par leur conjugaison à une protéine porteuse. Cependant, cette étape peut représenter un inconvénient technique pour certains laboratoires, ou, selon la séquence du peptide en question, une stratégie de couplage peut être difficile à adopter lorsque les résidus qui ne font pas partie de l'épitope ne sont pas disponibles. Comme alternative, l'invention vise selon l'un de ses aspects à convertir des peptides antigéniques en analogues peptidiques mimant les peptides parents ("peptidomimétiques") qui soient résistants à la dégradation protéolytique tout en conservant une haute activité antigénique.

**2. Matériels et méthodes.**

**2.1** Peptides.

Tous les analogues des peptides sont synthétisés par la méthode en phase solide sur un multisynthétiseur de peptides (Neimark, J. et Briand, J.P. (1993) Development of a fully automated multichannel peptide synthesizer with integrated TFA cleavable capability. Peptide Res. 6: 219-228), en utilisant un mode complètement automatique pour les peptides L et un mode semi-automatique pour les peptides rétro-inverso. Les dérivés d'acides aminés L et D protégés proviennent de Neosystem (Strasbourg). La représentation schématique des analogues peptidiques utilisés dans cet exemple est représentée sur le Tableau 6.

**2.1.1** Peptide 130-135 de l'histone H3.

La synthèse des peptides L et rétro-inverso a été décrite précédemment (Guichard, G., Benkirane, N., Zeder-Lutz, G., Van Regenumortel, M. H. V., Briand, J. P. et Muller, S. (1994) Antigenic mimicry of natural L-peptides with retro-inverso-peptidomimetics. Proc. Natl. Acad. Sci., USA, 91: 9765-9769).

**2.1.2** Peptide 277-291 de la protéine 52 kD de SSA/Ro (Ro52).

Les peptides L et rétro-inverso sont assemblés par chimie Boc sur une résine Boc Leu Pam (phénylacétamidométhyl) et sur une résine p-méthyl benzhydrylamine, respectivement. L'assemblage de la chaîne peptidique protégée est effectué à une échelle de 100 µmol en utilisant la neutralisation *in situ* décrite précédemment (Neimark, J. et Briand, J.P. (1993) Development of a fully automated multichannel peptide synthesizer with integrated TFA cleavable capability. Peptide Res. 6: 219-228). Pour les peptides rétro-inverso, on mime l'extrémité C-terminale du peptide parent en utilisant un dérivé malonate. L'ester monobenzylique de l'acide (R,S)-2-isobutyl malonique obtenu par alcoolyse de 2,2-diméthyl-5,-isobutyl-1,3-dioxane-4-6 dione (Chorev, M., Rubini, E., Gilon, C., Wormser, U. et Selinger, Z. (1983) Synthesis of partially modified retro-inverso substance P analogues and their biological activity. J. Med. Chem., 26: 129-135) est incorporé dans la chaîne peptidique sous forme de racémate générant ainsi une paire de diastéréoisomères. Les peptides sont clivés de la résine par traitement avec l'acide fluorhydrique anhydre (HF), contenant 10% (v/v) d'anisol et 1% (v/v) de 1,2 éthanedithiol.

Après élimination de l'HF sous vide, les peptides sont extraits de la résine et lyophilisés. Les peptides bruts sont ensuite purifiés sur une colonne Aquapore C18 ODS (100 x 10 mm) en utilisant un appareil HLPC préparatif (Perkin Elmer, Saint-Quentin en Yvelines, France). En ce qui concerne l'analogue rétro-inverso, les deux diastéréoisomères ont pu être séparés et purifiés par HPLC. Ils sont identifiés selon leur temps de rétention. L'isomère rétro-inverso le plus rapidement élué est désigné par "peptide RIa" et le peptide le moins rapidement élué par "peptide RIb" (Tableau 6). Le taux d'isomérisation des diastéréoisomères séparés a été suivi à 4°C et à 37°C. Tous les deux s'isomérisent lorsqu'ils sont incubés pendant 12 heures à 37°C (conditions normalement utilisées pour sensibiliser les plaques ELISA), les composants RIa et RIb conduisent à un mélange à l'équilibre de diastéréoisomères d'environ 50: 50 (RIa/RIb) et de 70:30 (RIb/RIa) respectivement. Au contraire, à 4°C les deux isomères apparaissent très stables, étant donné qu'on observe aucun changement dans les profils HPLC respectifs. Le revêtement des plaques de plastique avec les analogues peptidiques Ro52 277-291 a ainsi été effectué à 4°C tout au long de cette étude.

**2.1.3** Peptide 304-324 de la protéine 60kD de SSA/Ro.

Deux séries de peptides ont été synthétisées. Dans la première série, les extrémités N- et C-terminales du peptide parent et les extrémités inversées du peptide rétro-inverso ne sont pas protégées. Ils sont assemblés en chimie Fmoc sur une résine d'alcool p-alkoxybenzylique. Dans l'analogue rétro-inverso D-allo-Ile, D-allo-Ile est introduit à la place de D-Ile. Dans la seconde série d'analogues, les extrémités terminales N et C du peptide parent et des peptides rétro-inverso sont acétylées et amidées, respectivement. Ces peptides bloqués sont assemblés en chimie Fmoc sur une résine Fmoc -2,4-diméthoxy-4'-(carboxyméthyloxy)-benzhydrylamine.

L'assemblage des chaînes peptidiques protégées est effectué à une échelle de 25 μmol selon une méthodologie Fmoc classique. Les peptides de la résine sont clivés avec le réactif K (King, D., Fields, C., et Fields, G. (1990) A cleavage method which minimizes side reactions following Fmoc solid-phase peptide synthesis. Int. J. Pept. Protein Res., 36: 255-266) pendant deux heures et chaque peptide est récupéré dans un tube rempli d'éther de tert-butyl-méthyl à 4°C. Après centrifugation, les culots sont lavés deux fois avec de l'éther froid. Après la dernière centrifugation, chaque peptide est dissout dans une solution aqueuse pour être lyophilisé. Les peptides bruts sont finalement purifiés comme décrit ci-dessus.

**2.1.4** Peptide 28-45 de l'histone H3.

Quatre analogues peptidiques ont été synthétisés, à savoir le peptide parent L, le peptide L dans lequel le groupe COOH terminal libre est remplacé par un groupe carboxamide $-CONH_2$ et le peptide L et rétro-inverso avec les extrémités N- et C-terminales bloquées (Tableau 6). Les quatre peptides sont préparés en chimie Fmoc comme décrit ci-dessus.

Tous les peptides utilisés dans cette étude ont été contrôlés par HPLC et analysés par spectrométrie de masse (FAB) MS.

**2.2** Liaison du zinc aux analogues peptidiques du peptide 304-324 de Ro60.

La capacité du peptide parent Ro60 304-324 et des analogues rétro-inverso de lier des ions zinc a été testée en utilisant du $^{65}Zn$. Les peptides ont été immobilisés sur de la nitrocellulose comme décrit précédemment (Mazen, A., Menissier-de Murcia, J., Molinete, M., Simonin, F., Gradwohl, G., Poirier, G., et de Murcia, G. (1989), Poly(ADP-ribose)polymerase: a novel finger protein. Nucl. Acids. Res., 17: 4689-4698; Muller, S., Briand, J.P., Barakat, S., Lagueux, J., Poirier, G., De Murcia, G., et Isenberg, D. A. (1994) Autoantibodies reacting with poly(ADP-ribose) and with a zinc-finger functional domain of poly(ADP-ribose) polymerase involved in the recognition of damaged DNA. Clin. Immunol. Immunopathol., 73: 187-196).

**2.3** Couplage du peptide 130-135 de H3.

Le peptide 130-135 de H3 et son analogue rétro-inverso ont été conjugués à la sérum albumine bovine (BSA) au moyen de N-succinimidyl 3-[2-pyridyl dithio] propionate (SPDP). Le rendement de couplage a été mesuré par spectrophotométrie en étudiant le relargage de la 2-thiopyridone à partir de BSA modifiée par le SPDP lors de l'interaction avec les peptides contenant une cystéine (Muller, S. (1988) Peptide-carrier conjugation. Dans: R. H. Burdon et P. H. van Kippenberg (Eds.), Synthetic Polypeptides as Antigens. Elsevier, Amsterdam, p. 95-130).

**2.4** Sérums auto-immuns humains et de souris.

Des sérums de souris femelles F1 lupiques (NZB/W) ont été obtenus de S. Batsford (Freibourg, Allemagne). Les sérums humains proviennent de patients souffrant de lupus érythémateux disséminé (LED) et de syndrome de Sjögren (SS). Ils ont été obtenus de D.A. Isenberg (Londres), O. Meyer (Paris) et G. Fournié (Toulouse). Les sérums de volontaires sains ont été obtenus de J.L. Pasquali (Strasbourg). La plupart de ces sérums ont été utilisés dans des études antérieures (Ricchiuti, V., Briand, J. P., Meyer, O., Isenberg, D. A., Pruijn, G. et Muller, S. (1994) Epitope mapping with synthetic peptides of 52kD SSA/Ro protein reveals heterogeneous antibody profiles in human autoimmune sera. Clin. Exp. Immunol., 95: 397-407; Ricchiuti, V. et Muller, S. (1994) Use of peptides for the mapping of B-cell epitopes recognized by anti-Ro (SS-A) antibodies. Dans: D. A. Isenberg et A. C. Horsfall (Eds.), Autoimmune Diseases: focus on Sjögren's syndrome. Bios Scientific Publishers, Oxford, p. 101-106).

**2.5** Test ELISA.

Les sérums de souris lupiques ont été testés par ELISA comme décrit précédemment (Benkirane, N., Friede,

M., Guichard, G., Briand, J. P., Van Regenmortel, M. H. V. et Muller, S. (1993) Antigenicity and immunogenecity of modified synthetic peptides containing D-amino acid residues. J. Biol. Chem., 268: 26279-26285). Seuls les anticorps d'isotype IgG ont été testés. Le test des anticorps d'isotype IgG réagissant avec les peptides de Ro52 et Ro60 a été réalisé comme décrit par Barakat et al. (Barakat, S., Meyer, O., Torterotot, F., Youinou, P., Briand, J. P., Kahn, M. F. et Muller, S. (1992) IgG antibodies from patients with primary Sjögren's syndrome and systemic lupus erythematosus recognize different epitopes in 60-kD SSA/Ro protein. Clin. Exp. Immunol., 89: 38-45) et Ricchiuti et al. (Ricchiuti, V., Briand, J.P., Meyer, O., Isenberg, D. A., Pruijn, G. et Muller, S. (1994) Epitope mapping with synthetic peptides of 52kD SSA/Ro protein reveals heterogeneous antibody profiles in human autoimmune sera. Clin. Exp. Immunol., 95: 397-407) excepté dans le cas du peptide Ro52 277-291, où la sensibilisation des plaques ELISA a été effectué à 4°C au lieu de 37°C (voir les raisons ci-dessus). La valeur seuil de positivité de chaque test incluant celle des tests basés sur l'utilisation des analogues peptidiques rétro-inverso a été déterminée en utilisant 20 sérums d'individus sains. L'activité antigénique des peptides est également mesurée par ELISA, en utilisant un test d'inhibition en phase liquide. La procédure est comme celle décrite précédemment (Ricchiuti, V., Briand, J.P., Meyer, O., Isenberg, D. A., Pruijn, G. et Muller, S. (1994) Epitope mapping with synthetic peptides of 52kD SSA/Ro protein reveals heterogeneous antibody profiles in human autoimmune sera. Clin. Exp. Immunol., 95: 397-407).

**3.** Résultats.

**3.1** Peptides synthétiques et analogues rétro-inverso.

S'agissant de la séquence peptidique native, la modification rétro-inverso des polypeptides linéaires implique l'assemblage synthétique dans un ordre inverse des acides aminés avec la stéréochimie du carbone en $\alpha$ opposée à celle des acides aminés L correspondants. Le résultat est que les positions des groupes amino et carboxyl dans chacune des liaisons peptidiques sont changées tandis que la topologie des chaînes latérales est similaire ou identique à celle du peptide naturel. L'inversion des groupes terminaux crée un problème majeur dans la complémentarité de structure et de charge. Cependant, un ensemble d'approches a été envisagé pour traiter ce problème (Goodman, M. et Chorev, M. (1979) On the concept of linear modified retro-peptide structures. Acc. Chem. Res., 12: 1-7). Par exemple, les analogues rétro-inverso du peptide 130-135 de H3 et 277-291 de Ro52 sont synthétisés comme des isomères rétro-inverso modifiés à leurs extrémités terminales. On a introduit un résidu d'acide malonique substitué en C-2 pour mimer de façon proche le carbone C-terminal des peptides parents et du groupe carboxamide ($-NH_2-CO-$) à la place du groupe amino terminal libre. Les deux diastéréoisomères du peptide rétro-inverso 277-291 de Ro52 (analogues RIa et RIb, Tableau 6) ont pu être séparés par HPLC.
Une alternative pour limiter le problème de l'extrémité terminale consiste à préparer des peptides bloqués et des analogues rétro-inverso bloqués. Cette solution a été adoptée pour les peptides 304-324 de Ro60 et 28-45 de H3. L'utilisation de cette stratégie implique évidemment comme préalable que les peptides ayant leurs extrémités terminales bloquées soient encore reconnus par des anticorps.
Un autre problème dans l'approche des peptides rétro-inverso est que les centres asymétriques secondaires des chaînes latérales de thréonine et d'isoleucine doivent retenir leur chiralité correcte. Ceci a été étudié avec le peptide 304-324 de Ro60 qui a été synthétisé dans sa forme rétro-inverso, soit avec des résidus D-Ile, soit avec des résidus D-allo-Ile (Tableau 6).
La pureté des 13 peptides analogues utilisés dans cette étude est supérieure à 80% comme montré en HPLC analytique. L'analyse MS-(FAB) donne les résultats attendus pour tous les composés.

**3.2** Reconnaissance de l'analogue rétro-inverso de l'hexapeptide C-terminal de H3 par des sérums de souris lupiques.

L'hexapeptide C-terminal de l'histone H3 correspond à un épitope majeur de la protéine (Muller, S., Himmelspach, K. et Van Regenmortel, M. H. V. (1982) Immunochemical localization of the C-terminal hexapeptide of histone H3 at the surface of chromatin subunits. EMBO J., 1: 421-425). Ce segment a été utilisé comme peptide modèle dans les études antérieures de peptides modifiés, en particulier pour montrer que les analogues rétro-inverso peuvent être beaucoup plus résistants aux enzymes protéolytiques que les peptides L (Guichard, G., Benkirane, N., Zeder-Lutz, G., Van Regenmortel, M. H. V., Briand, J. P. et Muller, S. (1994) Antigenic mimicry of natural L-peptides with retro-inverso-peptidomimetics. Proc. Natl. Acad. Sci., USA, 91: 9765-9769). Tout d'abord, on a criblé une série de sérums de souris femelles F1 (NZB/W) avec le peptide parent 130-135 de H3, et choisi un certain nombre de sérums positifs. Ces sérums ont ensuite été testés en ELISA pour leur capacité à réagir avec le peptide 130-135 rétro-inverso. Comme montré sur le Tableau 7, les sérums de souris lupiques réagissent aussi bien avec le peptide 130-135 rétro-inverso qu'avec le peptide 130-135 parent.

**3.3.** Reconnaissance de l'analogue rétro-inverso de Ro52 277-191 par les sérums de patients auto-immuns.

Trente sérums de patients atteints de LED et de SS ont tout d'abord été testés pour leur capacité à réagir avec le peptide parent 277-291 de Ro52 qui a été précédemment identifié comme contenant un épitope reconnu par les anticorps de sous-classe IgG de patients auto-immuns (Ricchiuti, V., Briand, J.P., Meyer, O., Isenberg, D. A., Pruijn, G. et Muller, S. (1994) Epitope mapping with synthetic peptides of 52kD SSA/Ro protein reveals heterogeneous antibody profiles in human autoimmune sera. Clin. Exp. Immunol., 95: 397-407). En utilisant une valeur seuil de positivité correspondant à 0.30 unités DO (Ricchiuti, V., Briand, J.P., Meyer, O., Isenberg, D. A., Pruijn, G. et Muller, S. (1994) Epitope mapping with synthetic peptides of 52kD SSA/Ro protein reveals heterogeneous antibody profiles in human autoimmune sera. Clin. Exp. Immunol., 95: 397-407), on a trouvé que parmi ces 30 sérums, 5 contenaient des anticorps de sous-classe IgG réagissant avec le peptide L (Figure 6; plaques de microtitration sensibilisées avec les peptides à 4°C au lieu de 37°C). Les 30 sérums (positifs ou négatifs vis à vis du peptide L) ont été testés en parallèle avec les analogues RIa et RIb. On a montré que tous les sérums positifs avec le peptide L étaient aussi positifs avec les peptides rétro-inverso. Les valeurs de densité optique étaient significativement plus élevées avec l'analogue RIb (Figure 7). De façon très intéressante, parmi les 25 sérums négatifs avec le peptide L, 10 sérums ont réagi avec le peptide RIb (Figure 6). La densité optique moyenne correspondant à la moyenne arithmétique de toutes les valeurs de densité optique, y compris les valeurs sous la ligne seuil de positivité est de 0.21 (déviation standard 0.25) et 0.51 (déviation standard 0.62) avec les peptides L et RIb, respectivement.

Des tests de compétition ont été effectués avec les sérums de 11 patients montrant en test ELISA direct des valeurs de densité optique supérieure ou égale à 0.40 avec le peptide RIb 277-291. Lorsque le peptide L 277-291 est utilisé comme inhibiteur et le peptide RIb comme antigène pour sensibiliser les plaques de microtitration, on a trouvé que le peptide L possédait une activité inhibitrice dans tous les cas. Selon les sérums testés, on a trouvé jusqu'à 75,8% d'inhibition. Le peptide homologue testé en parallèle dans les mêmes conditions a inhibé jusqu'à 79,2% la liaison des anticorps au peptide RIb.

On a ainsi démontré que non seulement le peptide 277-291 RIb mimait le peptide L, mais aussi qu'il était généralement mieux reconnu que le peptide parent par les anticorps de patients. Ceci a permis de détecter la présence d'anticorps anti-peptide 277-291 dans 50% des sérums de patients testés, tandis que seulement 17% des sérums avaient réagi avec le peptide L (Figure 6).

**3.4** Reconnaissance de l'analogue peptidique rétro-inverso du peptide Ro60 304-324 par les sérums de patients auto-immuns.

La région 304-324 de Ro60 a été identifiée précédemment comme contenant un épitope majeur de la protéine (Ricchiuti, V. et Muller, S. (1994) Use of peptides for the mapping of B-cell epitopes recognized by anti-Ro (SS-A) antibodies. Dans: D. A. Isenberg et A. C. Horsfall (Eds.), Autoimmune Diseases: focus on Sjögren's syndrome. Bios Scientific Publishers, Oxford, p. 101-106). Vingt sérums de patients souffrant de LED et de SS ont tout d'abord été testés avec les analogues L et rétro-inverso. Six sérums (30%) ont réagi avec le peptide L (densité optique moyenne 0.21, (déviation standard 0.26) tandis que 15 sérums (75%) réagissent avec le peptide rétro-inverso (densité optique moyenne 0.81, déviation standard 0.54). Les sérums de patients ont réagi légèrement mieux avec l'analogue rétro-inverso D-allo-Ile comme représenté sur la figure 8: 16 sérums (80%) ont réagi avec ce dernier analogue (densité optique moyenne 0.81, déviation standard 0.50).

On a ensuite testé si les analogues peptidiques Ro60 304-324 ayant l'extrémité N-terminale acétylée et C-terminale carboxamidée étaient reconnus par les auto-anticorps. Si l'on compare avec la réactivité obtenue avec le peptide naturel L, on trouve que le peptide L bloqué est beaucoup mieux reconnu par les anticorps de patients auto-immuns (Figure 8). Quatorze sérums (70%) ont réagi avec le peptide L bloqué (densité optique moyenne 0.86, déviation standard 0.87). La densité optique moyenne et le nombre de sérums positifs sont encore augmentés en utilisant l'analogue rétro-inverso ayant les extrémités terminales bloquées (15/20 sérums positifs soit 75%; densité optique moyenne 1.15, déviation standard 0.97). Selon les sérums, la réaction positive avec l'analogue rétro-inverso bloqué est encore détectable à la dilution 1:4000 du sérum. La liaison des anticorps à l'analogue bloqué rétro-inverso peut être inhibée à la fois par l'analogue rétro-inverso bloqué homologue et par le peptide L bloqué hétérologue.

Il a été montré très récemment que le peptide 304-324 de la protéine Ro60 qui contient un motif en doigt de zinc lie de façon efficace le zinc radioactif (Muller, S., Briand, J.P., Barakat, S., Lagueux, J., Poirier, G., De Murcia, G., et Isenberg, D. A. (1994) Autoantibodies reacting with poly(ADP-ribose) and with a zinc-finger functional domain of poly(ADP-ribose) polymerase involved in the recognition of damaged DNA. Clin. Immunol. Immunopathol., 73: 187-196). On a donc vérifié que les peptides analogues, et plus particulièrement les peptides rétro-inverso étaient capables de lier le $^{65}Zn$. Comme représenté sur la figure 9, le peptide L bloqué ainsi que les trois analogues rétro-inverso lient effectivement le $^{65}Zn$. Ce résultat implique que les analogues rétro-inverso, comme le peptide L naturel, peuvent aisément former une structure en doigt stabilisée par un atome de zinc central coordonné de manière tétrahédrique, en utilisant les deux résidus cystéine et les deux résidus histidine de la séquence comme ligands (résidus 305, 309, 320 et 323; Figure 8).

**3.5** Reconnaissance de l'analogue rétro-inverso du peptide 28-45 de H3.

Le peptide 28-45 de H3 et son analogue rétro-inverso ont été testés avec des sérums auto-immuns. Dans des études précédentes, le peptide 30-45 de H3 n'avait pas été trouvé comme possédant une activité antigénique significative (Muller, S., et Van Regenmortel, M. H. V. (1993), Histones. Dans: M. H. V. Van Regenmortel (Ed.), Structure of Antigens. CRC Press, Boca Raton, p. 149-178). On a testé en ELISA 69 sérums de patients atteints de différentes maladies rhumatismales systémiques y compris 22 sérums de patients lupiques. Parmi les 69 sérums, 12 sérums (17.4%) sont positifs avec le peptide L naturel 28-45 (8 sur 22 sérums LED), 20 sérums (29%) sont positifs avec le peptide L bloqué 28-45 (12 sur 22 sérums LED), et 21 sérums (30.4%) réagissent avec l'analogue rétro-inverso bloqué (12 sérums sur 22 sérums LED).

**4.** Discussion.

Dans le cadre de cette invention, on montre la possibilité d'utiliser des peptides rétro-inverso à la place de peptides L linéaires naturels pour détecter les anticorps dans le sérum de patients auto-immuns. Dans tous les cas examinés dans ce travail (régions antigéniques 28-45 et 130-135 de H3, 277-291 de Ro52 et 304-324 de Ro60), on a trouvé que les peptides rétro-inverso possèdent une activité antigénique au moins égale ou supérieure à celle du peptide énantiomère L naturel. Ceci a permis de détecter davantage de sérums positifs réagissant avec chaque peptide, et en général d'augmenter de façon significative les valeurs de densité optique des sérums individuels vis à vis des sondes peptidiques sans changer la valeur du seuil de positivité déterminé avec des sérums normaux. Il faut noter que les anticorps réagissant avec les peptides rétro-inverso ne constituent pas un sous-ensemble d'anticorps particuliers présents dans les sérums de patients, étant donné que la liaison des anticorps de patients à l'analogue rétro-inverso est aussi bien inhibée par les peptides L que par les peptides rétro-inverso. Ce nouveau développement des peptides rétro-inverso dans le diagnostic est donc très prometteur.

Comme il est largement développé ci-dessus, le succès de cette approche repose sur un parfait contrôle des peptides sondes. Ainsi, il y a lieu de prêter une attention particulière à l'inversion des charges des extrémités qui peuvent altérer la réactivité antigénique des peptides rétro-inverso. Par exemple, beaucoup d'énantiomères de peptides biologiquement actifs décrits dans la littérature pour leur utilisation en pharmacologie ne sont pas du tout actifs. Un certain nombre d'approches ont été envisagées pour contourner ce problème (Goodman, M., et Chorev, M. (1979) On the concept of linear modified retro-peptide structures. Acc. Chem. res. 12: 1-7); (Chorev, M., et Goodman, M. (1993) A dozen years of retro-inverso peptidomimetics. Ace. Chem. Res., 26: 266-273). Par exemple, un résidu *gem*-diaminoalkyl peut être introduit à l'extrémité N-terminale du peptide rétro-inverso et l'acide malonique substitué en 2 peut être introduit à l'extrémité carboxyterminale. Cependant, les monoacyl *gem*-diaminoalkyl sont hydrolysables, et il faut s'attendre à ce que la demi-vie des peptides incluant de tels résidus soit de 10 à 50 heures à 25°C (Loudon, G. M., Merrick, R. A. et Jacob, J. N. (1981) Mechanism of hydrolysis of *N*-(1-aminoalkyl) amides. J. Am. Chem. Soc., 103: 4508-4515). En conséquence, dans le cas des peptides rétro-invsrso 130-135 de H3 et 277-291 de Ro52, on a choisi une terminaison carboxamide à la place d'un groupe amino libre des peptides parents. L'acide malonique substitué en C-2 est incorporé dans ces peptides sous forme de racémate pour mimer l'extrémité C-terminale des peptides L. Il faut relever que dans le cas de l'analogue rétro-inverso de Ro60 304-324, on a trouvé que l'analogue rétro-inverso avec les extrémités terminales inverses non bloquées était mieux reconnu que le peptide L (Figure 8) suggérant que pour ce peptide particulier, l'inversion des groupes terminaux n'affecte pas son antigénicité.

En comparaison avec les peptides parents ayant les extrémités N- et C-terminales libres, on a trouvé que les peptides bloqués 304-324 de Ro60 et 28-45 de H3 sont mieux reconnus par les anticorps de sérums auto-immuns. Ceci peut être corrélé au fait que les anticorps sont vraisemblablement induits contre les protéines complexées avec d'autres protéines ou avec des acides nucléiques (ADN ou ARN). Lorsque des séquences internes dans la structure primaire de la protéine sont spécifiquement reconnues par des auto-anticorps, il se peut que les peptides comportant des groupes amino et carboxyl bloqués miment mieux la région interne de la protéine (Gras-Masse, H. S., Jolivet, M. E., Audibert, F. M., Beachey, E. H., Chedid, L. A. et Tartar, A. L. (1986) Influence of $COHN_2$ or COOH as C-terminus groups on the antigenic characters of immunogenic peptides. Molec. Immunol., 23: 1391-1395). En coréllaire de ce qui est dit plus haut et concernant la présence des extrémités terminales inverses dans les analogues rétro-inverso bloqués, on a trouvé à nouveau que l'inversion des extrémités terminales n'a pas d'effet sur l'antigénicité. Il est conseillé cependant de contrôler ce résultat pour tout nouveau peptide testé.

La présence des résidus thréonine et isoleucine qui contiennent deux centres chiraux peut également présenter un problème pour respecter la chiralité correcte du composé rétro-inverso. Le peptide Ro60 304-324 contenant deux résidus isoleucine en position 319 et 324, deux analogues rétro-inverso ont été synthétisés soit avec des D-Ile,

soit avec des D-allo-Ile, et on a trouvé que l'analogue rétro-inverso D-allo-Ile était un peu mieux reconnu par les anticorps des patients que l'analogue rétro-inverso contenant les résidus D-Ile. Il faut noter cependant que les deux peptides 277-291 de Ro52 et 28-45 de H3 contiennent 2 résidus thréonine, et apparemment, ceci ne semble pas affecter leur activité antigénique.

Enfin, dans le cas de peptides contenant des résidus proline, l'analogue rétro-inverso peut présenter à ce niveau des contraintes conformationnelles locales (Goodman, M., et Chorev, M. (1979) On the concept of linear modified retro-peptide structures. Acc. Chem. Res. 12: 1-7); (Chorev, M., et Goodman, M. (1993) A dozen years of retro-inverso peptidomimetics. Acc. Chem. Res., 26: 266-273), qui peuvent influencer la liaison du peptide rétro-inverso à l'anticorps. Etant donné que le peptide rétro-inverso de Ro60 304-324 qui contient un résidu proline en position 321 est très bien reconnu par les auto-anticorps et peut efficacement lier le zinc, cela suggère que ce résidu proline 321 présent dans le motif en doigt de zinc (Figure 8) sert seulement à maintenir les 2 résidus histidine à une distance appropriée et qu'il ne compromet ni la liaison des ions métalliques, ni l'interaction avec des anticorps.

**Tableau 6**

**Séquence primaire des quatre domaines antigéniques étudiés au moyen des peptides L naturels et des analogues rétro-inverso (1).**

**Peptide C-terminal 130-135 de l'histone H3 (6 résidus) (2)**

. Peptide L

H-Cys → Gly → Gly → Ile → Arg → Gly → Glu → Arg → Ala-OH

. Peptide RI

$H_2$N-D-Cys ← Gly ← Gly ← D-Ile ← D-Arg ← Gly ← D-Glu ← D-Arg ← (*R,S*)-*m*Ala-OH

**Domaine interne 277-291 de la protéine 52kD de SSA/Ro (Ro52) (15 résidus)**

. Peptide L

H → Gly → Leu → Lys → Lys → Nle → Leu → Arg → Thr → Cys → Ala → Val → His → Ile → Thr → Leu → OH

. Peptides RIa et RIb

$H_2$N-Gly ← D-Leu ← D-Lys ← D-Lys ← D-Nle ← D-Leu ← D-Arg ← D-Thr ← D-Cys ← D-Ala ← D-Val ← D-His ← D-Ile ← D-Thr ← (*R,S*)-*m*Leu-OH

**Domaine interne 304-324 de la protéine 60kD de SSA/Ro (21 résidus)**

. Peptide L

H-Val → Cys → Glu → Lys → Leu → Cys → Asn → Glu → Lys → Leu → Leu → Lys → Lys → Ala → Arg → Ile → His → Pro → Phe → His → Ile-OH

. Peptide RI

HO-D-Val ← D-Cys ← D-Glu ← D-Lys ← D-Leu ← D-Cys ← D-Asn ← D-Glu ← D-Lys ← D-Leu ← D-Leu ← D-Lys ← D-Lys ← D-Ala ← D-Arg ← D-Ile ← D-His ← D-Pro ← D-Phe ← D-His ← D-Ile-H

. Peptide RI D-allo-Ile

HO-D-Val ← D-Cys ← D-Glu ← D-Lys- ← D-Leu ← D-Cys ← D-Asn ← D-Glu ← D-Lys ← D-Leu ← D-Leu ← D-Lys ← D-Lys ← D-Ala ← D-Arg← D-a-Ile ← D-His ← D-Pro ← D-Phe ← D-His ← D-a-Ile-H

. Peptide L bloqué

$CH_3CO$-Val → Cys → Glu → Lys → Leu → Cys → Asn → Glu → Lys → Leu → Leu → Lys → Lys → Ala → Arg → Ile → His → Pro → Phe → His → Ile-$NH_2$

. Peptide RI bloqué

$H_2N$-D-Val ← D-Cys ← D-Glu ← D-Lys ← D-Leu ← D-Cys ← D-Asn ← D-Glu ← D-Lys ← D-Leu ← D-Leu ← D-Lys ← D-Lys ← D-Ala ← D-Arg ← D-Ile ← D-His ← D-Pro ← D-Phe ← D-His ← D-Ile-$COCH_3$

**Domaine interne 28-45 de l'histone H3 (18 résidus)**

. Peptide L

H-Ser → Ala → Pro → Ala → Thr → Gly → Gly → Val → Lys → Lys → Pro → His → Arg → Tyr → Arg → Pro → Gly → Thr-OH

. Peptide L carboxamidé

H-Ser → Ala → Pro → Ala → Thr → Gly → Gly → Val → Lys → Lys → Pro → His → Arg → Tyr → Arg → Pro → Gly → Thr-$NH_2$

. Peptide L bloqué

$CH_3CO$-Ser → Ala → Pro → Ala → Thr → Gly → Gly → Val → Lys → Lys → Pro → His → Arg → Tyr → Arg → Pro → Gly → Thr-$NH_2$

. Peptide RI bloqué

$H_2N$-D-Ser ← D-Ala ← D-Pro ← D-Ala ← D-Thr ← Gly ← Gly ← D-Val ← D-Lys ← D-Lys ← D-Pro ← D-His ← D-Arg ← D-Tyr ← D-Arg ← D-Pro ← Gly ← D-Thr-$COCH_3$

**(1)** Les flèches indiquent le sens de la liaison CO-NH dans le squelette peptidique.

**(2)** Une séquence CGG est ajoutée pendant la synthèse afin de permettre au peptide d'être conjugué à la protéine porteuse par son groupe thiol au moyen de SPDP.

**Tableau 7**

**Réactivité en ELISA de sérums de souris lupiques avec le peptide naturel C-terminal 130-135 de l'histone H3 et avec le peptide rétro-inverso.**

| Souris (NZB/W) F1 | Peptide L | Peptide rétro-inverso | Puits sans antigène |
|---|---|---|---|
| 11/3 | > 3 | > 3 | 0.07 |
| 33/1 | 1.92 | 2.12 | 0.15 |
| 33/2 | 2.22 | 2.70 | 0.14 |
| 33/5 | 0.39 | 0.41 | 0.08 |
| 66/2 | 0.97 | 0.63 | 0.13 |
| 22/5 | 0.12 | 0.10 | 0.11 |

Des plaques de microtitration sont recouvertes directement avec des conjugués peptide-BSA (2 µM exprimé en peptide). Les sérums de souris sont dilués au 1:500 et laissés à incuber dans les puits pendant 1 heure comme décrit dans Benkirane, N., Friede, M., Guichard, G., Briand, J. P., Van Regenmortel, M. H. V. et Muller, S. (1993) Antigenicity and immunogenecity of modified synthetic peptides containing D-amino acid residues. J. Biol. Chem., 268: 26279-26285). L'IgG de chèvre anti-souris conjuguée à la péroxydase de raifort utilisée pour révéler la réaction, est dilué au 1:5000. Les résultats sont exprimés en valeurs de densité optique mesurées à 450 nm.

**Exemple 3**

**Peptides rétro-inverso et vaccin.**

A titre d'illustration dans le domaine de la vaccination, l'invention a plus particulièrement pour objet des peptides totalement rétro-partiellement inverso correspondant au déterminant antigénique majeur situé sur la protéine $VP_1$ du virus de la fièvre aphteuse (foot-and-mouth disease virus, FMDV, en anglais).

La fièvre aphteuse demeure l'une des principales maladies affectant les animaux tels que bovins, ovins et caprins, et peut donner lieu à des pertes économiques considérables. Bien que cette maladie entraîne rarement la mort de l'animal, les pertes de production restent très élevées et les séquelles nombreuses obligent à l'abattage des animaux.

Le FMDV appartient à la famille des picornaviridae qui rassemble les virus les plus simples que l'on connaisse. Seule une approche chimique a permis de comprendre la structure exacte du FMDV: l'enveloppe est constituée de l'association de 60 copies de chacune des 4 protéines appelées $VP_1$, $VP_2$, $VP_3$ et $VP_4$. On sait aujourd'hui que c'est la protéine $VP_1$ qui déclenche la synthèse d'anticorps (Ac) neutralisants.

Les vaccins existent classiquement sous deux formes, atténués ou inactivés, mais leur préparation et leur manipulation présentent de nombreux inconvénients. Depuis quelques temps, une nouvelle génération de vaccins plus sûrs et plus stables que les produits traditionnels est en train de voir le jour. Ainsi, dans le cas du FMDV, plusieurs groupes de chercheurs ont étudié la possibilité de faire produire grâce aux techniques du génie génétique des quantités importantes de la protéine $VP_1$. Cependant, les doses nécessaires pour induire une protection du bétail sont, comme pour la protéine $VP_1$ naturelle isolée des particules virales, encore trop élevées.

Parallèlement, une autre approche envisagée a constitué à imiter par synthèse chimique le fragment 141-160 de la protéine $VP_1$. Ce fragment, en effet, correspond à une région particulière de la protéine $VP_1$, sur lequel le ou les anticorp(s) neutralisant(s) se fixe(nt) spécifiquement. D'autre part, ce même peptide couplé à une protéine porteuse induit chez le cobaye une réponse immune telle que l'animal immunisé est protégé contre la fièvre aphteuse (ces animaux constituent un très bon modèle biologique pour l'étude de la maladie). Il a été constaté qu'une seule injection de peptide conjugué était suffisante pour protéger des animaux infectés.

Des recherches fondamentales sont cependant encore nécessaires pour donner à ces peptides de synthèse toute leur efficacité en tant que vaccins. Il s'avère en effet souvent difficile, voire impossible d'obtenir des titres d'Ac anti-peptide neutralisants suffisants. Ceci pourrait être lié aux problèmes liés à la stabilisation d'une conformation "optimale" d'une séquence linéaire ainsi qu'à la dégradation rapide des peptides injectés chez l'animal. Dans le cadre de l'invention, a donc été entreprise l'étude des propriétés antigéniques et immunogéniques d'analogues rétro-inverso (RI) issus de la boucle immunodominante de trois variants de sérotype A12 du FMDV. Les séquences de ces peptides et des analogues RI correspondants sont représentés sur le tableau 8 (remarque: la séquence parente du peptide étudié couvre la région 141-159; un résidu cystéine est rajouté en position N-terminale à des fins de couplage).

## TABLEAU 8

**Séquences de peptides synthétiques (région 141-159) issus de la boucle immunodominante de trois variants du sérotype A12 du virus de la fièvre aphteuse (FMDV).**

| | |
|---|---|
| peptide FP (souche USA) | C-G$^{141}$-S-G-V-R-G-D-**F**-G-S-L-A-**P**-R-V-A-R-Q-L$^{159}$ |
| Peptide FL | C-G$^{141}$-S-G-V-R-G-D-**F**-G-S-L-A-**L**-R-V-A-R-Q-L$^{159}$ |
| Peptide SL (souche A) | C-G$^{141}$-S-G-V-R-G-D-**S**-G-S-L-A-**L**-R-V-A-R-Q-L$^{159}$ |

**Séquences des analogues rétro-inverso correspondants**

HO-m(R ou S)Leu-q-r-a-v-r-(*)-a-l-s-G-(**)-d-G-r-v-G-s-G-c-$NH_2$

| | | | | |
|---|---|---|---|---|
| (**) | : | f | f | s |
| (*): | p | l | l | |

L'étude se divise en 3 parties:

1) Etude des propriétés antigéniques des analogues.

On dispose de sérums de cobayes immunisés contre le virus ("anti-virion"), la protéine $VP_1$ ("anti-protéine $VP_1$"), contre le peptide 141-159 (variant USA; "anti-peptide FP") ainsi que du sérum provenant de cobayes infectés par le virus ("convalescent"). Du sérum normal (lot négatif) de cobaye sert de témoin. Les résultats sont représentés dans le tableau 9. Les deux diastéréomères RIa et RIb ont été séparés, purifiés par HPLC et testés séparément en ELISA. L'isomère RI le plus rapidement élué en HPLC est appelé RIa, le 2e pic élué (isomère le moins rapidement élué) renferme l'isomère baptisé RIb. Seuls les résultats avec le système FP sont montrés.

On notera que les analogues RI sont aussi bien et souvent bien mieux reconnus que le peptide FP-L parent. Les résultats sont analogues dans le cas des peptides FL et SL.

Des études d'inhibition ont été réalisées en BIAcore. Dans le tableau 10, on montre les quantités d'analogues qu'il faut utiliser pour inhiber 50% de la liaison des divers anticorps au peptide parent FP-L immobilisé sur la matrice de dextrane (par la cystéine). Dans cette série d'expériences, on a étudié l'effet de la position en N-t ou C-t de la cystéine et l'effet de bloquer l'extrémité C-t ou les 2 extrémités Net C-t.

On notera que les analogues RI bloqués ou non sont tout aussi bons compétiteurs que le peptide FP-L parent.

## TABLEAU 9

**Réactivité en ELISA avec le peptide parent et les analogues rétro-inverso d'anti-sérums de cobayes anti-peptide 141-159 (FMDV, sérotype A12, variant USA), anti-protéine $VP_1$ et anti-virion, et de sérums de cobayes convalescents.**

| Sérums | Dilution sérum | Peptides (0,2 µM) | | | Sans Ag |
|---|---|---|---|---|---|
| | | FP-L | FP-RIa | FP-RIb | |
| normal | 1/5 000 | 0,06 | 0,04 | 0,11 | 0,02 |
| anti-peptide FP | 1/20 000 | 1,24 | 1,22 | 2,41 | 0,09 |
| anti-protéine $VP_1$ | 1/20 000 | 1,09 | 1,57 | 1,22 | 0,01 |
| anti-virion | 1/20 000 | 0,86 | 1,23 | 2,47 | 0,01 |
| convalescent | 1/20 000 | 1,23 | 2,31 | 1,91 | 0,01 |

PBS-T-gélatine 3%
↓
1H, 37°

peptide ← sérum ← conjugué anti IgG cobaye ← substrat: ← lecture 450 nm

non conjugué 0.2 µM
revêtement à 4°C pendant 1 nuit

cobaye différentes dilutions dilué dans PBS-T-gélatine 1% 1h, 37°C

couplé à la peroxydase 30 min, 37°C

$TMB/H_2O_2$ 15 min, 37°C
TMB = tétraméthylbenzidine

## TABLEAU 10

**Reconnaissance en test de compétition BIAcore du peptide FP et des analogues par les anticorps polyclonaux**

| Antisérum de cobaye | Quantité de peptides inhibiteurs (en µg) requise pour inhiber 50% de la liaison des anticorps au peptide (c) **FP-L** | | | | | | |
|---|---|---|---|---|---|---|---|
| | (c) FP-L | (c) FP-RIa | (c) FP-RIb | FP (c)-L | FP* (c)-L | *FP* (c)-L | *FP* (c)-RI |
| Anti-peptide FP | 25 | - | 25 | 25 | 25 | 20 | 20 |
| Anti-protéine $VP_1$ | 40 | 30 | 130 | 55 | 40 | 150 | 30 |
| Convalescent | 25 | 25 | 25 | 25 | 20 | 20 | 20 |
| Anti-virion | 30 | 25 | 120 | 50 | 40 | 140 | 25 |

Les antisérums de cobaye ont été dilués à 1:10

*: terminaux bloqués (Acétyl N-t; carboxamide C-t)

Principe du test

antisérum + peptides compétiteurs
↓ (différentes concentrations)
FP-L

2) Etude des propriétés immunogéniques des analogues.

On a injecté le peptide parent (-L) et les analogues RIa et RIb dans des lapins et mesuré la réponse en BLISA à l'égard des peptides homologues.

Les résultats sont montrés avec le peptide FL sur le tableau 11. On notera que le peptide FL-RIb a induit chez le lapin "Cannes" des titres d'anticorps 8 fois plus élevés. De plus, alors que l'animal n'est plus réimmunisé, le titre chute lentement chez le lapin "Cannes" alors qu'il chute très rapidement dans le lapin anti-peptide FL-L ("Antibes") pour être nul à jour 143 (au même moment, le titre en anticorps chez le lapin "Cannes" est encore de 6000).

**TABLEAU 11**

**Test en ELISA des sérums de lapins immunisés contre le peptide FL parent et les analogues RI du peptide FL.**

| | | Lapin Antibes | Lapin Romarin | Lapin Cannes |
|---|---|---|---|---|
| Saignées (2) | | Anti-FL-L/FL-L | Anti-FL-RIa/FL-RIa | Anti FL-RIb/FL-RIb |
| 1 | jours 23 | 0 (3) | 250 | 4000 |
| 2 | 37 | 4000 | 6000 | 6000 |
| 3 | 52 | 8000 | 16000 | 100000 |
| 4 | 64 | 24000 | 24000 | 200000 |
| 5 | 78 | 550 | 4000 | 64000 |
| 6 | 113 | 275 | 500 | 48000 |
| 7 | 143 | 0 | 0 | 6000 |
| 8 | 173 | 0 | 0 | 1000 |
| 9 | 203 | 0 | 0 | 0 |

(1) Les lapins ont été immunisés aux jours 0, 14, 30, 43 et 58 avec les analogues peptidiques (60 µg/injection) couplés de façon covalente à des petits liposomes unilamellaires contenant du monophosphoryl lipide A comme adjuvant (injections sous cutanée).

(2) Jours après la 1ère injection.

(3) Les valeurs sont exprimées en tant que titres (dilution la plus élevée de sérum à laquelle la valeur d'absorbance en ELISA est de 1.0). Les saignées sont testées en ELISA en utilisant 2 µM des conjugués peptide-BSA pour sensibiliser les plaques.

Principe du test:

Peptides couplés ← sérum anti-peptide ← anti-lapin ← $TMB/H_2O_2$

| Peptides couplés | sérum anti-peptide | anti-lapin | $TMB/H_2O_2$ |
|---|---|---|---|
| à la BSA par la cystéine (au moyen de SPDP) 2 µM | conc. variables | IgG peroxydase 1: 40 000 | |
| une nuit à 37°C | 1h, 37°C | 30 min, 37°C | 15 min, 37°C |

SPDP = N-succinimidyl-3(2-pyridyldithio)propionate

Sur le même principe que celui présenté sur le tableau 10, on a testé la reconnaissance des différents analogues par les anticorps de lapin anti-peptide en utilisant les analogues en solution dans le BIAcore (tableau 12, test de compétition).

## TABLEAU 12

**Reconnaissance en test <u>de compétition BIAcore</u> du peptide FL et analogues par les anticorps polyclonaux.**

| Antisérums de lapin | Quantité de peptides inhibiteurs (en µg) requises pour inhiber 50% de la liaison des anticorps au peptide (c) **FL-L** | | | | | |
|---|---|---|---|---|---|---|
| | (c) FL-L | (c) FL-RIa | (c) FL-RIb | (c) **FP-L** | (c) FP-RIa | (c) FP-RIb |
| Antibes [anti-(c) FL-L] | 80 | 80 | 60 | 85 | 70 | 60 |
| Cannes [anti-(c) FL-RIb] | 25 | 60 | 50 | 30 | 60 | 50 |

Les antisérums de lapins ont été dilués à 1:2.

On note que les Ac anti-FL reconnaissent aussi bien les peptides de la série FL et de la série FP et qu'il y a réaction croisée entre peptides-L, RIa et RIb des deux séries FL et FP.

**3)** Etude des propriétés immunogéniques des analogues: réponse neutralisante.

Les peptides FP-L et RIb ont été injectés dans des cobayes et on a mesuré *in vitro* la réponse neutralisante sur le virus. Les préparations utilisées correspondent aux peptides analogues liés à des liposomes de type petits liposomes unilamellaires préparés selon le procédé décrit par Benkirane et al. (J. Biol. Chem., 268: 26279-26285, 1993). Le test de neutralisation a été effectué selon Francis et Black (1983) J. Hyg. Camb., 91: 329-334. Les résultats sont reportés sur le tableau 13.

TABLEAU 13

| **Résultats de neutralisation *in vitro* de la 1e et 2e saignée (3 animaux par peptide)** | | |
|---|---|---|
| | 1ère saignée | 2ème saignée |
| | | |
| FP-L | nul | 1.0 |
| | 1.0 | 2.0 |
| | nul | - |
| | | |
| FP-RI | 0.5 | 1.0 |
| | 0.5 | 1.5 |
| | 0.5 | 2.5 |

1ère injection: j 0
1ère saignée: j 20
2éme injection: j 43
2ème saignée: j 74

Les résultats sont exprimés en $\log_{10}$ et correspondent à la différence entre le titre du virus incubé avec du sérum normal et celui du virus incubé avec du sérum de cobaye immunisé (dilution des sérums 1/20).

Les tests ont été réalisés dans le laboratoire du professeur F. Brown (Département américain d'agriculture, Centre des maladies animales de Plum Island, Greenport, NY 11944, USA).

On note que l'efficacité du peptide FP-L est similaire à celle du même peptide précédemment décrite (Rowlands et al., Nature, 306: 694-697, 1983) et que ces résultats sont tout à fait reproduits avec le peptide FP-RI.

**Exemple 4**

**Immunomodulation thérapeutique.**

Récemment, le phénomène d'antagonisme TCR (récepteur des cellules T) par des peptides analogues d'épitopes T a été montré et l'utilisation d'antagonistes spécifiques du TCR a été décrite (De Magistris, M. T., Alexander, J., Coggeshall, M., Altmon, A., Gaeta, F. C. A., Grrey, H. M. et Sette, A. (1992) Cell 68: 625). Ces peptides sont capables d'inhiber la prolifération des cellules T induites par un antigène. De tels peptides antagonistes sont obtenus par substitution d'un acide aminé parmi les résidus du peptide antigène en contact avec la cellule T, ou bien par incorporation de résidus en contact avec le TCR dans une séquence de poly-alanines.

La présente invention tient son originalité dans le fait qu'elle a précisément pour objet l'utilisation d'immunorétroïdes correspondant à des peptides agonistes ou antagonistes des TCR pour l'obtention de "médicaments" destinés au traitement viral (effet agoniste sur les cellules T suppressives) ou au traitement de maladies autoimmunes (effet antagoniste sur les cellules T auxilliaires ou "helper" en anglais par exemple).

On a pris pour exemple les immunorétroïdes partiellement rétro-inverso d'un épitope T cytotoxique (épitope 56-68) de la matrice du virus de la grippe reconnu par la molécule HLA-A2 (molécule de classe I). Dans ce travail, on a remplacé systématiquement une à une les liaisons amide du peptide parent par des liaisons rétro-inverso. Les pseudopeptides ayant servi à cette étude ont été synthétisés par la méthodologie en solution comme décrit ci-dessus. La liste de ces peptides est présentée figure 10. La terminologie a, b a été utilisée lorsque les deux isomères optiques ont pu être séparés par HPLC. Sur la figure 10, le contenu de la parenthèse précédant M58-66 indique la position de la liaison rétro-inverso. A titre d'exemple pour le peptide GG5, [gLeu$^{60}$-mGly$^{61}$] M58-66 signifie que la liaison rétro-inverso est entre l'acide aminé 60 et l'acide aminé 61. Il faut noter que lorsque la liaison rétro-inverso porte sur Gly-Ile, Ile a été remplacé par Leu (cf. peptides GG0a et GG0b) et lorsque la liaison rétro-inverso porte sur Phe-Thr, Thr a été remplacé par Ala (cf. peptide GG11), pour des facilités de synthèse étant donné que cette mutation n'affecte pas la liaison à la molécule HLA.

On a dans un premier temps étudié l'interaction de chaque immunorétroïde avec la molécule présentatrice de peptide HLA-A2, puis chaque peptide capable de se lier a été testé pour ses capacités à inhiber ou activer la réponse T cytotoxique.

. Le premier test (test de liaison des peptides à la molécule HLA-A2) est réalisé comme suit:

**1. Source de HLA-A2**

<u>**1.1** A partir de la lignée cellulaire T2.</u>

T2 est un variant de la lignée T1 produite par la fusion d'une cellule d'un lymphome T (CEM) et d'une lignée cellulaire lymphoblastoïde B (721.174). Par immunofluorescence, on ne détecte quasiment pas de molécules HLA de classe I à la surface des T2. Ceci est dû à une mutation entraînant l'absence de transporteurs de peptides dans le réticulum endoplasmique. Comme il n'y a pas de peptides pour stabiliser l'association chaîne lourde et β2microglobuline (β2m) à part peut-être quelques peptides endogènes signaux, on considère ces cellules comme une excellente source de molécules HLA classe I "vides", prêtes à accueillir des peptides exogènes dans leur site accepteur.

En ajoutant du peptide exogène, les molécules HLA sont stabilisées.

**1.2 A partir de molécules HLA purifiées.**

<u>**1.2.1** Purification des molécules HLA classe I.</u>

Les molécules HLA ont été purifiées sur une colonne d'affinité à partir de cellules lymphoblastoïdes B immortalisées par le virus d'Epstein-Barr (EBV). Pour cela, les cellules ont été lysées dans un tampon Tris-HCl 50 mM, NaCl 150 mM, EDTA (acide éthylènediaminetétraacétique) 5 mM, Nonidet P40 (NP40) 0.5% contenant des inhibiteurs de protéases. Les lysats ont été ensuite passés sur une série de colonnes sur lesquelles sont fixés des anticorps anti-HLA. On a récupéré par élution en pH basique les molécules du CMH fixées sur ces colonnes d'affinité.

<u>**1.2.2** Dénaturation des molécules HLA classe I.</u>

Les molécules HLA classe I panifiées doivent être "vidées" de leurs peptides endogènes avant de pouvoir être testées pour l'assemblage avec des peptides exogènes. Pour cela, on réalise une dénaturation avec NaOH 12.5 mM, Urée 1.5 mM, sérum albumine bovine (BSA) très pure (Sigma) 1% pendant 1h à 4°C. Les chaînes lourdes sont récupérées après passage des molécules HLA dénaturées sur colonne G25/PD10 Sephadex (Pharmacia). Les peptides qui pourraient reformer un tri-complexe avec la chaîne lourde et la β2m sont éliminés par rétention dans 1a colonne. Les chaînes légères β2m (12 kd) devraient sortir avec les chaînes lourdes de la colonne (exclusion < 10 kd), mais certaines étant potentiellement retenues, on ajoute secondairement de la β2m exogène (2 μg/ml, SIGMA).

**2. Anticorps.**

Les anticorps monoclonaux suivants ont été employés pour piéger les chaînes lourdes des molécules HLA au cours des étapes de chromatographie d'affinité et lors du point 3. ci-dessous:

- W6/32 et B9.12.1 qui sont dirigés contre tous les types HLA mais qui reconnaissent des épitopes distincts,
- B1.23.2 pour les HLA-B,
- BB7.2, spécifique du HLA-A2,
- A11.1, spécifique des HLA-A3, A11 et A24,
- M28 qui reconnaît la β2m.

**3. Détection de l'assemblage HLA-peptide.**

- Avec les cellules déficientes en transporteurs de peptides: 800 000 cellules ont été lysées pendant 1h à 37°C dans du tampon Tris-HCl 50 mM, NaCl 150 mM, EDTA 5 mM, Nonidet P40 0.5% avec des inhibiteurs de protéases en présence ou non de peptide exogène à la concentration voulue. Après centrifugation, les surnageants ont été récupérés et complétés à 200 µl avec du tampon phosphate salin (PBS) tween 0,05%, BSA 1%, PMSF (fluorure de phénylméthylsulfonyle) 1 mM, inhibiteur de trypsine (TI) 10 mg/ml.
- Avec le HLA purifié et dénaturé: des fractions aliquotes de 1 µg de HLA ont été incubées en présence ou non de peptide exogène à la concentration voulue pendant 24 ou 48h à 4°C. Les produits de renaturation ont été amenés à un volume de 200 µl avec du PBS tween 0,05%, BSA 1%, PMSF 1 mM, TI 10 mg/ml.

Dans tous les cas, les échantillons ont été déposés pendant une nuit à 4°C en duplicate de 100 µl dans les puits de microplaques sensibilisées avec des anticorps monoclonaux spécifiques de chaînes lourdes des HLA étudiés. Ces anticorps ont été au préalable immobilisés à 10 µg/ml dans du PBS pendant 2h à 37°C ou la nuit à 4°C puis une saturation a été faite avec du PBS contenant de la BSA 1% pendant 1h à 22°C.

Comme la fixation de la β2m à la chaîne lourde d'un HLA ne se fait que si un peptide est présent pour stabiliser le complexe, on révèle uniquement les HLA ayant lié un peptide grâce à l'anticorps M28. Cet anticorps anti-β2m est couplé à la phosphatase alcaline. La présence de l'enzyme, et donc de l'anticorps, a été révélée par son action sur le substrat 4-méthyl umbelliferyl P (MuP, Sigma) qui est transformé en méthyl umbelliférone. Ce dernier émet un signal fluorescent lors d'une excitation à 405/492 nm. La lecture a été faite avec l'appareil Fluoscan (Millipore).

La figure 11 résume le principe de la détection.

Les résultats sont montrés sur la figure 12: on voit que le peptide rétro-inverso GG0a est au moins aussi actif que le peptide parent M58-66 (les peptides Leu 59 et Ala 65 qui renferment des changements Ile → Leu en position 59 et Thre → Ala en position 65 sont également très actifs). Les analogues RI GG0b, GG7, GG10 et GG11 sont un peu moins actifs mais se lient significativement à la molécule HLA-A2. Les autres analogues RI (GG1, GG5, GG8 et GG12) ne se lient pas à la molécule HLA-A2.

. Pour être efficace en immunomodulation, il ne suffit pas qu'un peptide se lie à la molécule HLA, il doit encore être reconnu par le TCR. Le deuxième test est donc un test biologique dont le principe est le suivant:

<table>
<tr><th>EFFECTEUR:</th><th>CIBLE:</th></tr>
<tr><td>lymphocytes T d'un individu typé HLA A2 et ayant eu la grippe récemment (cellules stimulées plusieurs fois par le peptide 58-66 en présence de cellules B et T irradiées)</td><td>lymphocytes T du même donneur traités par le virus d'Epstein-Barr + peptides<br>1 µg/ml<br>GG0a<br>GG10<br>GG7</td></tr>
<tr><td colspan="2">mélange de l'effecteur et de la cible<br>↓<br>Mesure de la cytolyse</td></tr>
</table>

Les résultats sont montrés sur la figure 13: le peptide GG0a est au moins (sinon plus) efficace que le peptide M58-66 parent pour inhiber la cytolyse. Le peptide GG10 est moins efficace. Le peptide GG7 n'induit pas de cytolyse. Ce dernier peptide est donc tout aussi intéressant que GG0a. On en déduit que GG0a semble être un très bon agoniste de la cytolyse, et que GG7 pourrait être un antagoniste de la cytolyse.

## Revendications

**1.** Utilisation

* de composés du type peptidique,
dont l'une au moins des liaisons -CO-NH-, et avantageusement toutes les liaisons - CO-NH-, de la chaîne peptidique du peptide parent correspondant, (ne comportant pas de liaison -NH-CO- dans sa chaîne peptidique), est (sont) remplacée(s) par une (des) liaison(s) -NH-CO-,
la chiralité de chaque résidu aminoacyle, qu'il soit impliqué ou non dans une ou plusieurs liaisons -NH-CO- susmentionnées, étant inversée par rapport aux résidus aminoacyles correspondants constituant ledit peptide parent,
ces composés du type peptidique étant encore désignés peptides "rétro-inverso",
lesdits peptides rétro-inverso dérivant des peptides parents qui répondent à la formule (I) suivante :

$$X\text{-}AA1\text{-}(AA2\text{-----}AAn\text{-}1)_i\text{-}AAn\text{-}Y \qquad (I)$$

dans laquelle :

- AA1 représente un résidu aminoacyle pouvant être désaminé et dont la fonction amine en α, si elle existe, peut être protégée par un groupement X, X représentant P-, R- ou RCO-
- i = 0 ou 1,
- n représente 2 lorsque i = 0, et n représente un nombre entier allant de 3 à 1000, et de préférence de 5 à 100, lorsque i = 1, étant entendu que lorsque n = 3, le peptide rétro-inverso correspondant répond à la formule AA1-AA2-AA3,
- AAn représente un résidu aminoacyle pouvant être décarboxylé dont la fonction acide en α, si elle existe, est protégée éventuellement par un groupement Y, Y étant de type ester : -OR ou amide $-NH_2$, ou -NRR', les groupements R, R' pouvant représenter des atomes d'hydrogène, des radicaux alkyle de 1 à 25 atomes de carbone, des radicaux contenant un groupe allyle et ayant de 3 à 25 atomes de carbone ou des radicaux contenant un groupe aryle et ayant de 6 à 25 atomes de carbone, et notamment $-CH_3$ (méthyl), $-CH_2CH_3$ (éthyl), $-CH(CH_3)_2$ (isopropyl), $-C(CH_3)_3$ (tertio-butyl), $-\Phi$ (phényl), $-CH_2\Phi$ (benzyl),$-CH_2\text{-}CH_2\Phi$ (2-phényléthyl), $-CH_2CHCH_2$ (allyl), méthyl-fluorényl, $-CH_2CONH_2$ (glycolamide), $-CH_2CON\Phi_2$ (benzhydrylglycolamide), cette liste n'étant pas limitative,
le groupement P étant de type uréthane (Boc (tertbutyloxycarbonyl), Fmoc (fluorénylméthyloxycarbonyl), Z (benzyloxycarbonyl), $CH_2CHCH_2OCO$-(allyloxycarbonyl) ou autre),
et dans ces peptides parents de formule (I), l'une au moins des liaisons entre deux résidus aminoacyles de la formule I étant une liaison -NH-CO-, et le cas échéant, l'un au moins des résidus aminoacyles AA1 à AAn étant de chiratité opposée à celle du részdu aminoacyle correspondant dans le peptide parent,
et notamment les peptides rétro-inverso répondant à la formule (II) suivante :

$$A\text{-}CH(R_i)\text{-}NH\text{-}[CO\text{-}CH(R_k)\text{-}NH]_{j\text{-}i}\text{-}CO\text{-}CH(R_{j+1})\text{-}B \qquad (II)$$

dans laquelle :

- $R_i$, $R_k$, $R_{j+1}$ représentent les chaînes latérales des résidus impliqués dans une ou plusieurs liaisons -NH-CO,
- le nombre total de résidus de la séquence est fixé à n où n est un nombre entier supérieur à 1, et de préférence de 3 à 1000, de préférence de 3 à 100,
- i, j, k, sont des paramètres entiers définis de la façon suivante :
  $1 \le i \le j \le n$,
  k = 0 si i = j et k prend les valeurs i+1 à j,

quatre cas de figure pouvant se présenter concernant la nature des blocs A et B:

<u>1/ i = 1 et j+1 = n :</u>

A = H-, $H_2N$-, P-HN-, RR'N-, $H_2NCO$-, RR'NCO-, RCO-
B = H-, -COOH, -COOR, $-CONH_2$, -CONRR', -NHCOR,

<u>2/ i = 1 et j+1 ≠n :</u>

A = H-, $H_2N$-, P-HN-, RR'N-, $H_2NCO$-, RR'NCO-, RCO-
B = -CO-NH-CH($R_{j+2}$)-CO-...-NH-CH($R_n$)-CO-Y
avec Y = -OH, -OR, -$NH_2$, -NRR',

3/ $i \neq 1$ et $j+1 = n$ :

A = X-HN-CH($R_1$)-CO-...-NH-CH($R_{i-1}$)CO-NH-
avec X = H-, P-, R-, RCO-
B = H-, -COOH, -COOR, -$CONH_2$, -CONRR', -NHCOR,

4/ $i \neq 1$ et $j+1 \neq n$ :

A = X-HN-CH($R_1$)-CO-...-NH-CH($R_{i-1}$)CO-NH-
avec X = H-, P-, R-, RCO-
B = -CO-NH-CH($R_{j+2}$)-CO-...-NH-CH($R_n$)-CO-Y
avec Y = -OH, -OR, -$NH_2$, -NRR',

les groupements R, R' pouvant représenter des atomes d'hydrogène, des radicaux alkyle de 1 à 25 atomes de carbone, des radicaux contenant un groupe allyle et ayant de 3 à 25 atomes de carbone ou des radicaux contenant un groupe aryle et ayant 6 à 25 atomes de carbone, et notamment -$CH_3$ (méthyl), -$CH_2CH_3$ (éthyl),-$CH(CH_3)_2$ (isopropyl), -$C(CH_3)_3$ (tertio-butyl), -$\Phi$ (phényl), -$CH_2\Phi$ (benzyl), -$CH_2$-$CH_2\Phi$ (2-phényl-éthyl), -$CH_2CHCH_2$ (allyl), méthyl-fluorényl, -$CH_2CONH_2$ (glycolamide), -$CH_2CON\Phi_2$ (benzhydrylglycolamide), cette liste n'étant pas limitative,
le groupement P étant de type uréthane (Boc (tertbutyloxycarbonyl), Fmoc (fluorénylméthyloxycarbonyl), Z (benzyloxycarbonyl), $CH_2CHCH_2OCO$-(allyloxycarbonyl) ou autre),
la chiralité de chaque résidu, qu'il soit impliqué ou non dans une ou plusieurs liaisons -NH-CO-, étant inversée par rapport aux résidus aminoacyles correspondants constituant le peptide parent,
ou

* d'anticorps dirigés contre les susdits peptides rétro-inverso (anticorps anti-rétro-inverso),
  lesdits peptides rétro-inverso étant susceptibles de former un complexe avec les susdits anticorps anti-rétro-inverso ainsi qu'avec les anticorps dirigés contre les peptides ou protéines parents (désignés anticorps anti-parents), pour la préparation :

  - ♦ d'un médicament destiné à la prévention ou au traitement de pathologies d'origine virale ou bactérienne, ou de pathologies autoimmunes, ou de maladies neurodégénératives,
  - ♦ d'un médicament destiné à la prévention ou au traitement de pathologies impliquant les molécules du complexe majeur d'histocompatibilité et/ou les récepteurs des cellules T,

  ou pour la mise en oeuvre d'une méthode de diagnostic *in vitro* des pathologies susmentionnées.

**2.** Utilisation selon la revendication 1, dans laquelle les pathologies sont choisies parmi :

- ♦ les pathologies impliquant des molécules du complexe majeur d'histocompatibilité et/ou les récepteurs des cellules T,
- ♦ les maladies auto-immunes, et notamment la thyroïdite de Hashimoto, la maladie de Basedow, la maladie d'Addison, l'insuffisance hypophysaire, la gastrite de Biermer, certaines stérilités, le diabète juvénile de type 1, le syndrome de Goodpasture, la myasthénie, le rhumatisme articulaire aigu, le pemphigus, la pemphigoïde bulleuse, la dermatite herpétiforme, le vitiligo, le pelade, le psoriasis, l'ophtalmie sympathique, l'uvéite, le syndrome de Guillain-Baré, la sclérose en plaques, l'anémie hémolytique, le purpura thrombopénique idiopathique, la leucopénie idiopathique, la cirrhose biliaire primitive, l'hépatite chronique active, la rectocolite hémorragique, l'iléite de Crohn, le syndrome de Gougerot-Sjögren, la polyarthrite rhumatoïde, la dermatopolymyosite, la sclérodermie, la connectivite mixte, le lupus érythémateux discoïde, le lupus érythémateux disséminé,
- ♦ les maladies neurodégénératives,
- ♦ les maladies d'origine virale, notamment :

  - le SIDA provoqué par le virus de l'immuno-déficience humaine HIV-1 et HIV-2,

- la paraplégie associée à HTVL-1, ou la leucémie à cellules T de l'adulte, provoquée par le virus de la leucémie humaine à cellules T (virus HTLV),
- les infections provoquées par le virus respiratoire synticial,
- les infections provoquées par le virus coxsakie, par exemple les méningites aiguës lymphocytaires,
- les infections provoquées par le virus d'Epstein-Barr, par exemple la mononucléose infectieuse,
- les infections provoquées par le cytomégalovirus, par exemple la maladie des inclusions cytomégaliques,
- l'herpès provoqué par le virus de l'herpès humain,
- l'herpès provoqué par le virus 6 de l'herpès simplex,
- les infections provoquées par le parvovirus B19 humain, par exemple les gastro-entérites infectieuses,
- l'hépatite B provoquée par le virus de l'hépatite B,
- l'hépatite C provoquée par le virus de l'hépatite C,
- la grippe provoquée par le virus influenza,
- la rubéole provoquée par le virus de la rubéole,
- les infections provoquées par le virus de la Dengue, par exemple les arboviroses,
- les rhumes, rhinites, coryza provoqués par les rhinovirus,
- la fièvre aphteuse provoquée par le virus de la fièvre aphteuse.

3. Utilisation selon la revendication 1, **caractérisée en ce que** les peptides rétro-inverso sont des peptides totalement rétro-inverso, ou partiellement rétro-inverso ayant au moins deux liaisons "rétro-inverso" consécutives, et notamment deux liaisons rétro-inverso consécutives.

4. Anticorps anti-rétro-inverso polyclonaux ou monoclonaux tels qu'obtenus par immunisation d'un animal avec au moins un peptide rétro-inverso selon la revendication 1, lesdits anticorps étant susceptibles de former un complexe avec au moins l'un des susdits peptides rétro-inverso, et avec les peptides ou protéines parents correspondant à ces derniers, et **caractérisés en ce qu'**ils reconnaissent le peptide parent ou la protéine parente avec une affinité au moins égale à celle présentée par les anticorps anti-peptide parent ou anti-protéine parente vis à vis du peptide parent ou de la protéine parente.

5. Complexe antigène-anticorps **caractérisé en ce qu'**il est constitué par l'un des complexes suivants :

- protéine parente - anticorps dirigé contre un peptide rétro-inverso correspondant à un peptide parent issu de la protéine parente
- peptide parent - anticorps dirigé contre un peptide rétro-inverso correspondant au peptide parent
- peptide rétro-inverso correspondant à un peptide parent - anticorps dirigé contre le peptide parent ou la protéine parente
- peptide rétro-inverso correspondant à un peptide parent - anticorps dirigé contre un peptide rétro-inverso correspondant au peptide parent

le peptide rétro-inverso étant tel que défini dans la revendication 1 et l'anticorps dirigé contre le peptide rétro-inverso étant défini tel que dans la revendication 4,

lesdits complexes étant tels qu'ils présentent une stabilité au moins égale à celle de ces complexes, dans lesquels lorsque intervient un peptide rétro-inverso, il est remplacé par un peptide parent ou une protéine parente, et lorsque intervient un anticorps dirigé contre un peptide rétro-inverso, il est remplacé par un anticorps antipeptide parent ou antiprotéine parente.

6. Complexe entre un peptide rétro-inverso tel que défini dans la revendication 1 ou la revendication 3, et un élément du complexe d'histocompatibilité majeur (encore désigné complexe MHC-peptide rétro-inverso), et éventuellement un récepteur de cellules T (encore désigné complexe MHC-peptide rétro-inversa-récepteur T).

7. Complexe entre un peptide rétro-inverso tel que défini dans la revendication 1 ou la revendication 3, et un récepteur de cellules T.

8. Méthode de diagnostic *in vitro* telle que définie dans la revendication 1 de pathologies associées à la présence dans l'organisme d'un patient, d'une protéine exogène ou endogène, susceptible d'être directement ou indirectement impliquée dans le processus d'apparition et/ou de développement de ces pathologies, **caractérisée en ce qu'**elle comprend :

- la mise en contact d'un échantillon biologique provenant d'un patient susceptible d'être porteur d'anticorps

dirigé contre ladite protéine, avec un peptide rétro-inverso selon la revendication 1, le peptide rétro-inverso correspondant à tout ou partie de ladite protéine endogène ou exogène, ou correspondant à un peptide susceptible d'être reconnu par des anticorps reconnaissant eux-mêmes la protéine exogène ou endogène, ou dans des conditions permettant la réaction entre les anticorps dirigés contre la protéine et susceptibles d'être présents dans l'échantillon biologique, et le susdit peptide rétro-inverso ;

- la détention *in vitro* du complexe antigène - anticorps selon la revendication 5, susceptible d'être formé à l'étape précédente ou
- la détection *in vitro* d'anticorps circulants chez le patient par un test de compétition en utilisant un anticorps anti-rétro-inverso.

**9.** Méthode de diagnostic *in vitro* telle que définie dans la revendication 1 de pathologies associées à la présence dans l'organisme d'un patient d'une protéine exogène ou endogène, susceptible d'être directement ou indirectement impliquée dans le processus d'apparition et/ou de développement de ces pathologies, ladite méthode étant **caractérisée en ce qu'**elle comprend :

- la mise en contact d'un échantillon biologique provenant d'un patient susceptible d'être porteur de ladite protéine, avec l'un au moins des anticorps selon la revendication 4, les anticorps étant avantageusement dirigés contre un peptide rétro-inverso consistant en un peptide rétro-inverso correspondant à tout ou partie de ladite protéine endogène ou exogène, ou
  dans des conditions permettant la réaction entre la protéine susceptible d'être présente dans l'échantillon biologique, et les susdits anticorps dirigés contre le susdit peptide rétro-inverso ;
- la détection *in vitro* du complexe antigène - anticorps selon la revendication 5, susceptible d'être formé à l'étape précédente, ou
- la détection d'antigènes circulants chez le patient dans des tests de compétition en utilisant un peptide rétro-inverso selon la revendication 1.

**10.** Méthode de diagnostic *in vitro* selon la revendication 8, dans laquelle les pathologies sont choisies selon la revendication 2.

**11.** Méthode de diagnostic *in vitro* selon la revendication 9, dans laquelle les pathologies sont choisies selon la revendication 2.

**12.** Nécessaire ou kit pour la mise en oeuvre de méthodes de diagnostic *in vitro* selon l'une des revendications 8 à 11, comprenant :

- un peptide rétro-inverso selon la revendication 1 correspondant à tout ou partie de ladite protéine endogène ou exogène, ou correspondant à un peptide susceptible d'être reconnu par des anticorps reconnaissant eux-mêmes la protéine exogène ou endogène, ou bien des anticorps selon la revendication 4, dirigés contre ce peptide rétro-inverso ;
- des réactifs pour rendre un milieu apte à la formation d'une réaction immunologique ;
- des réactifs permettant de détecter le complexe antigène - anticorps selon la revendication 5, qui a été produit à l'issue de la réaction immunologique, lesdits réactifs contenant éventuellement un marqueur ou étant susceptibles d'être reconnus à leur tour par un réactif marqué, plus particulièrement dans le cas où le peptide rétro-inverso ou les anticorps anti-rétro-inverso susmentionnés ne sont pas marqués.

**13.** Utilisation d'au moins un peptide rétro-inverso selon la revendication 1, pour la préparation d'un médicament destiné à la prévention ou au traitement de pathologies associées à la présence dans l'organisme d'un individu, d'une protéine exogène ou endogène susceptible d'être directement ou indirectement impliquée dans le processus d'apparition et/ou de développement de ces pathologies.

**14.** Utilisation d'au moins un peptide rétro-inverso selon 1a revendication 1, pour la préparation d'un vaccin dans le cadre de la prévention de pathologies associées à la présence dans l'organisme d'un individu, d'une protéine exogène ou endogène susceptible d'être reconnue par les anticorps dirigés contre les peptides rétro-inverso.

**15.** Composition pharmaceutique **caractérisée en ce qu'**un peptide rétro-inverso selon la revendication 1, est associé à une molécule porteuse protéique ou non pouvant induire *in vivo* la production d'anticorps neutralisant la protéine exogène ou endogène responsable de la pathologie, ou induire *in vivo* une réponse immune cellulaire cytotoxique.

**16.** Composition pharmaceutique, **caractérisée en ce qu'**elle comprend des anticorps selon la revendication 4, en association avec un véhicule physiologiquement acceptable.

## Claims

**1.** Use

* of compounds of the peptide type, in which at least one of the -CO-NH- bonds, and advantageously all the -CO-NH- bonds, of the peptide chain of the corresponding parent peptide (containing no -NH-CO- bond in its peptide chain) is (are) replaced by (a) -NH-CO- bond(s), the chirality of each aminoacyl residue, whether involved or not in one or more of the abovementioned -NH-CO- bonds, being reversed with respect to the corresponding aminoacyl residues which constitute said parent peptide,
  these compounds of the peptide type also being called "retro-inverso" peptides,
  said retro-inverso peptides deriving from parent peptides which have the following formula (I):

$$X\text{-}AA1\text{-}(AA2\text{----}AAn\text{-}1)_i\text{-}AAn\text{-}Y \quad (I)$$

in which:

- AA1 represents an aminoacyl residue, which may be deaminated and in which the amine function in the $\alpha$-position, if this exists, can be protected by a group X, X representing P-, R- or RCO-,
- i = 0 or 1,
- n represents 2 if i = 0, and n represents an integer from 3 to 1,000, and preferably from 5 to 100, if i = 1, it being understood that if n = 3, the corresponding retro-inverso peptide has the formula AA1-AA2-AA3,
- AAn represents an aminoacyl residue which may be decarboxylated and in which the acid function in the $\alpha$-position, if this exists, is optionally protected by a group Y, Y being of the ester -OR or amide $-NH_2$ or -NRR' type,
  it being possible for the groups R and R' to represent hydrogen atoms, alkyl radicals having 1 to 25 carbon atoms, radicals containing an allyl group and having 3 to 25 carbon atoms or radicals containing an aryl group and having 6 to 25 carbon atoms, and in particular $-CH_3$ (methyl), $-CH_2CH_3$ (ethyl), $-CH(CH_3)_2$ (isopropyl), $-C(CH_3)_3$ (tert-butyl), $-\Phi$ (phenyl), $-CH_2\Phi$ (benzyl), $-CH_2\text{-}CH_2\Phi$ (2-phenyl-ethyl), $-CH_2CHCH_2$ (allyl), methyl-fluorenyl, $-CH_2CONH_2$ (glycolamide), or $-CH_2CON\Phi_2$ (benzhydrylglycolamide), this list being not limiting,
  the group P being of the urethane type (Boc (tert-butyloxycarbonyl), Fmoc (fluorenylmethyloxycarbonyl), Z (benzyloxycarbonyl), $CH_2CHCH_2OCO$-(allyloxycarbonyl) or other),
  and in these parent peptides of the formula (I), at least one of the bonds between two aminoacyl residues of the formula I being a -NH-CO- bond, and, where appropriate, at least one of the aminoacyl residues AA1 to AAn being of opposite chirality to that of the corresponding aminoacyl residue in the parent peptide,
  and particularly the retro-inverso peptides corresponding to the following formula (I)):

$$A\text{-}CH(R_i)\text{-}NH\text{-}[CO\text{-}CH(R_k)\text{-}NH]_{j\text{-}i}\text{-}CO\text{-}CH(R_{j+1})\text{-}B \quad (II)$$

  in which:

  - $R_i$, $R_k$ and $R_{j+1}$ represent side chains of the residues involved in one or more -NH-CO- bonds,
  - the total number of residues of the sequence is fixed to n, where n is an integer greater than 1, and preferably from 3 to 1,000, preferably 3 to 100,
  - i, j, k are whole parameters defined in the following manner:
    $1 \leq i \leq j \leq n$,
    k = 0 if i = j and k assumes the values i + 1 to j,

  where four cases can be given regarding the nature of blocks A and B:

1/ i = 1 and j + 1 = n:

A = H-,$H_2N$-, P-HN-, RR'N-, $H_2NCO$-, RR'NCO-, RCO-
B = H-, -COOH, -COOR, -$CONH_2$, -CONRR', -NHCOR,

2/ i = 1 and j + 1 ≠ n:

A = H-, $H_2N$-, P-HN-, RR'N-, $H_2NCO$-, RR'NCO-, RCO-
B = -CO-NH-CH($R_{j+2}$)-CO-...-NH-CH($R_n$)-CO-Y
where Y = -OH, -OR, -$NH_2$, -NRR',

3/ i ≠ 1 and j + 1 = n:

A = X-HN-CH($R_1$)-CO-...-NH-CH($R_{i-1}$)CO-NH-
where X = H-, P-, R-, RCO-
B = H-, -COOH, -COOR, -$CONH_2$, -CONRR', -NHCOR,

4/ i ≠ 1 and j + 1 ≠ n:

A = X-HN-CH($R_1$)-CO-...-NH-CH($R_{i-1}$)CO-NH-
where X = H-, P-, R-, RCO-
B = -CO-NH-CH($R_{j+2}$)-CO-...-NH-CH($R_n$)-CO-Y
where Y = -OH, -OR, -$NH_2$, -NRR',

it being possible for the groups R and R' to represent hydrogen atoms, alkyl radicals having 1 to 25 carbon atoms, radicals containing an allyl group and having 3 to 25 carbon atoms or radicals containing an aryl group and having 6 to 25 carbon atoms, and in particular -$CH_3$ (methyl), -$CH_2CH_3$ (ethyl), -$CH(CH_3)_2$ (isopropyl), -$C(CH_3)_3$ (tert-butyl), -Φ (phenyl), -$CH_2$Φ (benzyl), -$CH_2$-$CH_2$Φ (2-phenyl-ethyl), -$CH_2CHCH_2$ (allyl), methyl-fluorenyl, -$CH_2CONH_2$ (glycolamide), -$CH_2CON\Phi_2$ (benzhydyylglycolamide), this list being not limiting,
the group P being of the urethane type (Boc (tert-butyloxycarbonyl), Fmoc (fluorenylmethyloxycarbonyl), Z (benzyloxycarbonyl), $CH_2CHCH_2OCO$-(allyloxycarbonyl) or other),
the chirality of reach residue, whether involved or not in one or more -NH-CO-bonds, being reversed with respect to the corresponding aminoacyl residues which constitute the parent peptide,
or

* of antibodies directed against said retro-inverso peptides (anti-retro-inverso antibodies),
  said retro-inverso peptides being capable of forming a complex with said anti-retro-inverso antibodies as well as with antibodies directed against the parent peptides or proteins (called anti-parent antibodies), for the preparation:

  ♦ of a medicament intended for prevention or treatment of diseases of a viral or bacterial origin, or autoimmune diseases, or neurodegenerative diseases,
  ♦ of a medicament intended for prevention or treatment of diseases involving molecules of major histocompatibility complex and/or T cell receptors,

  or for implementation of a method of *in vitro* diagnosis of the abovementioned diseases.

**2.** Use according to claim 1, in which the diseases are chosen among;

♦ diseases involving molecules of major histocompatibility complex and/or T cell receptors,
♦ autoimmune diseases, and particularly Hashimoto's thyroiditis, Basedow's disease, Addison's disease, hypophysial insufficiency, Biermer's gastritis, certain sterilities, type I juvenile diabetes, Goodpasture's syndrome, myasthenia, acute articular rheumatism, pemphigus, bullous pemphigoid, dermatitis herpetiformis, vitiligo, alopecia, psoriasis, sympathetic ophthalmia, uveitis, Guillain-Baré syndrome, plaque sclerosis, haemolytic anaemia, idiopathic thrombopenic purpura, idiopathic leukopenia, primary biliary cirrhosis, active chronic hepatitis, haemorrhagic rectocolitis, Crohn's ileitis, Gougerot-Sjögren syndrome, rheumatoid polyarthritis, dermatopolymyositis, scleroderma, mixed connectivitis, discoid lupus erythematosus, disseminated lupus erythematosus,

- ♦ neurodegenerative diseases,
- ♦ diseases of a viral origin, particularly:

  - AIDS caused by the human immunodeficiency virus HIV1 and HIV2,
  - paraplegia associated with HTVL-1, or adult T cell leukaemia caused by the human T cell leukaemia virus (HTLV virus),
  - infections caused by the respiratory syncytial virus,
  - infections caused by the Coxsackie virus, for example acute lymphocytic meningitis,
  - infections caused by the Epstein-Barr virus, for example infectious mononucleosis,
  - infections caused by the cytomegalovirus, for example cytomegalic inclusion disease,
  - herpes caused by the human herpes virus,
  - herpes caused by the herpes simplex 6 virus,
  - infections caused by the human Parvovirus B19, for example infectious gastroenteritis,
  - hepatitis B caused by the hepatitis B virus,
  - hepatitis C caused by the hepatitis C virus,
  - influenza caused by the influenza virus,
  - rubeola caused by the rubeola virus,
  - infections caused by the Dengue virus, for example arboviroses,
  - colds, rhinitis or coryza caused by rhinoviruses,
  - aphthous fever caused by the aphthous fever virus.

**3.** Use according to claim 1, **characterized in that** the retro-inverso peptides are totally retro-inverso or partly retro-inverso peptides, having at least two consecutive "retro-inverso" bonds, and in particular two consecutive retro-inverso bonds.

**4.** Anti-retro-inverso polyclonal or monoclonal antibodies such as obtained by immunization of an animal with at least one retro-inverso peptide according to claim 1, said antibodies being capable of forming a complex with at least one of the above-mentioned retro-inverso peptides, and with the parent peptides or proteins corresponding to the latter, and **characterized in that** they recognize the parent peptide or the parent protein with an affinity at least equal to that shown by the anti-parent peptide or anti-parent protein antibodies with respect to the parent peptide or the parent protein.

**5.** Antigen-antibody complex, **characterized in that** it is constituted by one of the following complexes:

- parent protein - antibody directed against a retro-inverso peptide corresponding to a parent peptide derived from the parent protein
- parent peptide - antibody directed against a retro-inverso peptide corresponding to the parent peptide
- retro-inverso peptide corresponding to a parent peptide - antibody directed against the parent peptide or the parent protein
- retro-inverso peptide corresponding to a parent peptide - antibody directed against a retro-inverso peptide corresponding to the parent peptide

the retro-inverso peptides being such as defined in claim 1, and the antibody directed against the retro-inverso peptide being such as defined in claim 4,

said complexes being such as they have a stability which is at least equal to that of these complexes in which, if a retro-inverso peptide occurs, it is replaced by a parent peptide or a parent protein, and if an antibody directed against a retro-inverso peptide occurs, it is replaced by an anti-parent peptide or anti-parent protein antibody.

**6.** Complex between a retro-inverso peptide as defined in claim 1 or claim 3, and an element of the major histocompatibility complex (also called MHC-retro-inverso peptide complex), and optionally a T cell receptor (also called MHC-retro-inverso peptide-T receptor complex).

**7.** Complex between a retro-inverso peptide as defined in claim 1 or claim 3, and a T cell receptor.

**8.** Method for *in vitro* diagnosis, such as defined in claim 1, of diseases associated with the presence in the organism of a patient of an exogenous or endogenous protein capable of being involved directly or indirectly in the process of the appearance and/or development of these diseases, **characterized in that** it comprises:

- bringing a biological sample originating from a patient who may carry antibodies directed against said protein into contact with a retro-inverso peptide according to claim 1, the retro-inverso peptide corresponding to all or part of said endogenous or exogenous protein, or corresponding to a peptide which is capable of being recognized by antibodies which themselves recognize the exogenous or endogenous protein,
  under conditions which allow reaction between the antibodies directed against the protein, which antibodies may be present in the biological sample, and said retro-inverso peptide;
- *in vitro* detection of the antigen-antibody complex according to claim 5, which may be formed in the preceding stage, or
- *in vitro* detection of the antibody in the patient's circulation by a competition test using an anti-retro-inverso antibody.

**9.** Method for *in vitro* diagnosis, such as defined in claim 1, of diseases associated with the presence in the organism of a patient of an exogenous or endogenous protein capable of being involved directly or indirectly in the process of the appearance and/or development of these diseases, said method being **characterized in that** it comprises:

- bringing a biological sample originating from a patient who may carry said protein into contact with at least one of the antibodies according to claim 4, the antibodies being advantageously directed against a retro-inverso peptide consisting of a retro-inverso peptide corresponding to all or part of said endogenous or exogenous protein,
  under conditions which allow reaction between the protein which may be present in the biological sample and said antibodies directed against said retro-inverso peptide;
- *in vitro* detection of the antigen-antibody complex according to claim 5, which may be formed in the preceding stage, or
- detection of antigens in the patient's circulation in competition tests using a retro-inverso peptide according to claim 1

**10.** Method for *in vitro* diagnosis according to claim 8, in which the diseases are chosen according to claim 2.

**11.** Method for *in vitro* diagnosis according to claim 9, in which the diseases are chosen according to claim 2.

**12.** Kit for implementation of the methods for *in vitro* diagnosis according to one of claims 8 to 11, comprising:

- a retro-inverso peptide according to claim 1, corresponding to all or part of said endogenous or exogenous protein, or corresponding to a peptide which is capable of being recognized by antibodies which themselves recognize the exogenous or endogenous protein, or antibodies, according to claim 4, directed against this retro-inverso peptide;
- reagents for making a medium capable of the formation of an immunological reaction;
- reagents which allow detection of the antigen-antibody complex according to claim 5, which has been produced as a result of the immunological reaction, said reagents optionally comprising a marker or being capable of being recognized in their turn by a marked reagent, more particularly in the case where the above-mentioned retro-inverso peptide or anti-retro-inverso antibodies are not marked.

**13.** Use of at least one retro-inverso peptide according to claim 1, for the preparation of a medicament intended for prevention or treatment of diseases associated with the presence in the organism of an individual, of an exogenous or endogenous protein capable of being involved directly or indirectly in the process of the appearance and/or development of these diseases.

**14.** Use of at least one retro-inverso peptide according to claim 1, for the preparation of a vaccine in the frame of the prevention of diseases associated with the presence in the organism of an individual, of an exogenous or endogenous protein which is capable of being recognized by antibodies directed against the retro-inverso peptides.

**15.** Pharmaceutical composition, **characterized in that** a retro-inverso peptide according to claim 1 is combined with a protein or non-protein carrier molecule which can induce *in vivo* production of antibodies which neutralize the exogenous or endogenous protein responsible for the disease, or induce *in vivo* a cytotoxic cell immune response.

**16.** Pharmaceutical composition, **characterized in that** it comprises antibodies according to claim 4, in combination with a physiologically acceptable vehicle.

## Patentansprüche

**1.** Verwendung

* von Verbindungen vom Peptidtyp,
von denen wenigstens eine der -CO-NH--Bindungen, und vorteilhafterweise alle -CO-NH--Bindungen, der Peptidkette des entsprechenden Ausgangspeptids, (welches keine -NH-CO- -Bindung in seiner Peptidkette enthält), durch eine (mehrere) -NH-CO--Bindung(en) ersetzt wird (werden), wobei die Chiralität jedes Aminoacylrestes, welcher entweder in eine oder mehrere der vorher erwähnten - NH-CO--Bindungen eingeschlossen ist oder nicht, im Vergleich zu den entsprechenden Aminoacylgruppen, die das Ausgangspeptid bilden, umgekehrt ist, wobei diese Verbindungen vom Peptidtyp auch als "retro-inverse" Peptide bezeichnet werden, wobei diese retro-inversen Peptide von Ausgangspeptiden abgeleitet werden, welche der folgenden Formel (I) entsprechen:

$$X\text{-}AA1\text{-}(AA2\text{-----}AAn\text{-}1)_i\text{-}AAn\text{-}Y \qquad (I)$$

in der:

- AA1 eine Aminoacylgruppe darstellt, welche desaminiert werden kann und deren Aminfunktionalität am $\alpha$-Atom, falls sie vorhanden ist, durch eine Gruppe X geschützt werden kann, wobei X P-, R- oder RCO- darstellt,
- i=0 oder 1 ist,
- falls i=0 ist, n 2 darstellt, und falls i=1 ist, n eine ganze Zahl zwischen 3 und 1000, bevorzugt zwischen 5 und 100, darstellt, wenn n=3 ist, entspricht das entsprechende retro-inverse Peptid der Formel AA1-AA2-AA3,
- AAn eine Aminoacylgruppe darstellt, die decarboxyliert werden kann und deren Säurefunktionalität am $\alpha$-Atom, falls sie vorhanden ist, eventuell durch eine Gruppierung Y geschützt wird, wobei Y vom Estertyp ist: -OR oder Amid, -$NH_2$ oder NRR', wobei
  die Gruppen R, R' Wasserstoffatome, Alkylradikale mit 1 bis 25 Kohlenstoffatomen, Radikale mit einer Allylgruppe mit 3 bis 25 Kohlenstoffatomen oder Radikale mit einer Arylgruppe mit 6 bis 25 Kohlenstoffatomen sein können, und insbesondere -$CH_3$ (Methyl), -$CH_2CH_3$ (Ethyl), -$CH(CH_3)_2$ (Isopropyl), - $C(CH_3)_3$ (tertiäres Butyl), -$\Phi$ (Phenyl), -$CH_2\Phi$ (Benzyl), -$CH_2$-$CH_2\Phi$ (2-Phenylethyl), -$CH_2CHCH_2$ (Allyl), Methylfluorenyl, -$CH_2CONH_2$ (Glykolamid), - $CH_2CON\Phi_2$ (Benzylhydroglykolamid), wobei diese Liste nicht begrenzend ist, wobei
  die Gruppe P vom Urethantyp ist (Boc (tertiäres Butyloxycarbonyl), Fmoc (Fluorenylmethyloxycarbonyl), Z (Benzyloxycarbonyl), $CH_2CHCH_2OCO$- (Allyloxycarbonyl) oder andere), und in diesen Ausgangspeptiden der Formel (I) wenigstens eine der Bindungen zwischen zwei Aminoacylresten der Formel (I) eine -NH-CO--Bindung ist, und gegebenenfalls wenigstens einer der Aminoacylreste AA1 bis AAn eine zu dem entsprechenden Aminoacylrest im Ausgangspeptid entgegengesetzte Chiralität hat, und insbesondere die retro-inversen Peptide der folgenden Formel (II) entsprechen:

$$A\text{-}CH(R_i)\text{-}NH\text{-}\ [CO\text{-}CH(R_k)\text{-}NH]_{j\text{-}i}\text{-}CO\text{-}CH(R_{j+1})\text{-}B \qquad (II)$$

  in der:

  - $R_i$, $R_k$, $R_{j+1}$ die Seitenketten der an einer oder mehreren -NH-CO--Bindungen beteiligten Reste darstellen,
  - die Gesamtanzahl der Reste der Sequenz auf n festgesetzt ist, wobei n eine ganze Zahl größer als 1 und bevorzugt 3 bis 1000 und bevorzugt 3 bis 100 ist,
  - i, j, k die folgendermaßen definierten Parameter sind:
    $1 \leq i \leq j \leq n$,

  wobei k = 0 ist, falls i =j ist und k die Werte i+1 bei j annimmt, wobei sie
  vier Möglichkeiten der Konfiguration bezüglich der Art der Blöcke A und B aufweisen können:

1/ i = 1 und j+1 = n:

A = H-, $H_2N$-, P-HN-, RR'N-, $H_2NCO$-, RR'NCO-, RCO-
B = H-, -COOH, -COOR, -$CONH_2$, -CONRR', -NHCOR,

2/ i = 1 und j+1 ≠ n:

A = H-, $H_2N$-, P-HN-, RR'N-, $H_2NCO$-, RR'NCO-, RCO-
B = -CO-NH-CH($R_{j+2}$)-CO-...-NH-CH($R_n$)-CO-Y
mit Y = -OH, -OR, $NH_2$, -NRR',

3/ i ≠ 1 und j+1 = n:

A = X-HN-CH($R_1$)-CO-...-NH-CH($R_{i-1}$)CO-NH-
mit X = H-, P-, R-, RCO-
B = H-, -COOH, -COOR, -$CONH_2$, -CONRR', -NHCOR,

4/ i ≠ 1 und j+1 ≠ n:

A = X-HN-CH($R_1$)-CO-...-NH-CH($R_{i-1}$)CO-NH-
mit X = H-, P-, R-, RCO-
B = -CO-NH-CH($R_{j+2}$)-CO-...-NH-CH($R_n$)-CO-Y
mit Y = -OH, -OR, $NH_2$, -NRR', wobei

die Gruppen R, R' Wasserstoffatome, Alkylradikale mit 1 bis 25 Kohlenstoffatomen, Radikale mit einer Allylgruppe mit 3 bis 25 Kohlenstoffatomen oder Radikale mit einer Arylgruppe mit 6 bis 25 Kohlenstoffatomen sein können, und insbesondere -$CH_3$ (Methyl), -$CH_2CH_3$ (Ethyl), -$CH(CH_3)_2$ (Isopropyl),-$C(CH_3)_3$ (tertiäres Butyl), -Φ (Phenyl), -$CH_2$Φ (Benzyl), -$CH_2$-$CH_2$Φ (2-Phenylethyl), -$CH_2CHCH_2$ (Allyl), Methylfluorenyl, -$CH_2CONH_2$ (Glykolamid),-$CH_2CON$$Φ_2$ (Benzylhydroglykolamid), wobei diese Liste nicht begrenzend ist, wobei
die Gruppe P vom Urethantyp ist (Boc (tertiäres Butyloxycarbonyl), Fmoc (Fluorenylmethyloxycarbonyl), Z (Benzyloxycarbonyl), $CH_2CHCH_2OCO$- (Allyloxycarbonyl) oder andere), wobei die Chiralität jedes Aminoacylrestes, welcher entweder in eine oder mehrere -NH-CO--Bindungen eingeschlossen ist oder nicht, im Vergleich zu den entsprechenden Aminoacylgruppen, die das Ausgangspeptid bildenden, umgekehrt ist, oder

* von gegen die oben erwähnten retro-inversen Peptide gerichteten Antikörpern (anti-retro-inverse Antikörper), wobei die retro-inversen Peptide mit den oben erwähnten anti-retro-inversen Antikörpern sowie mit den gegen die Ausgangspeptide oder -proteine gerichteten Antikörpern (bezeichnet als anti-Ausgangs-Antikörper) einen Komplex bilden können

für die Herstellung:

- ♦ eines Medikaments, für die Vorbeugung oder Behandlung von Erkrankungen viralen oder bakteriellen Ursprungs oder von Autoimmunkrankheiten oder neurodegenerativen Erkrankungen,
- ♦ eines Medikaments, für die Vorbeugung oder Behandlung von Erkrankungen mit Beteiligung der Moleküle des Haupthistokompatibilitätskomplexes und/oder der T-Zell-Rezeptoren,

oder für die *in vitro*-Anwendung eines diagnostischen Verfahrens für die vorher erwähnten Krankheiten.

**2.** Verwendung nach Anspruch 1, wobei die Krankheiten ausgewählt sind aus:

- ♦ den Krankheiten mit Beteiligung von Molekülen des Haupthistokompatibilitätskomplexes und/oder der T-Zell-Rezeptoren,
- ♦ den Autoimmunerkrankungen, insbesondere die Hashimoto-Thyroiditis, die Basedow-Krankheit, die Addison-Krankheit, die Hypophyseninsuffizienz, die Biermer-Gastritis, bestimmte Sterilitäten, juveniler Diabetes vom Typ 1, das Goodpasture-Syndrom, die Myasthenie, der akute Gelenkrheumatismus, der Pemphigus, das bullöse Pemphigoid, die Dermatitis herpetiformis, die Vitiligo, die Pelade, die Psoriasis, die Ophthalmia sympathica, die Uveitis, das Guillain-Barré-Syndrom, Multiple Sklerose, die hämolytische Anämie, die idiopathische

thrombozytopenische Purpura, die idiopathische Leukopenie, die primäre biliäre Zirrhose, die aktive chronische Hepatitis, die hämorrhagische Rektocolitis, die Crohn-Krankheit, das Gougerot-Sjögren-Syndrom, die rheumatoide Polyarthritis, der Dermatomyositis-Polymyositis-Komplex, die Sklerodermie, das Sharp-Syndrom (connectivite mixte), der diskoide Lupus erythematodes, der systemische Lupus erythematodes,
- ♦ den neurodegenerativen Erkrankungen
- ♦ den Erkrankungen mit viralem Ursprung, insbesondere:
    - AIDS, ausgelöst durch das Virus der humanen Immunschwäche HIV-1 und HIV-2,
    - die mit HTVL-1 verbundene Paraplegie, oder die adulte T-Zell-Leukämie, ausgelöst durch das Virus der humanen T-Zell-Lymphome (Virus: HTLV),
    - die durch das respiratorische Synzytial-Virus ausgelösten Infektionen,
    - die durch das Coxsackie-Virus ausgelösten Infektionen, zum Beispiel die akute lymphozytäre Meningitis,
    - die durch das Epstein-Barr-Virus ausgelösten Infektionen, zum Beispiel die infektiöse Mononukleose,
    - die durch das Zytomegalie-Virus ausgelösten Infektionen, zum Beispiel die Einschlusskörperchen-Krankheit,
    - der Herpes, ausgelöst durch das humane Herpes-Virus,
    - der Herpes, ausgelöst durch das humane Herpes simplex-Virus 6,
    - die durch das humane Parvovirus B19 ausgelösten Infektionen, zum Beispiel die gastro-enteralen Infektionen,
    - die durch das Hepatitis B-Virus ausgelöste Hepatitis B,
    - die durch das Hepatitis C-Virus ausgelöste Hepatitis C,
    - die durch das Influenza-Virus ausgelöste Grippe,
    - die durch das Rötelvirus ausgelösten Röteln,
    - die durch das Dengue-Virus ausgelösten Infektionen, zum Beispiel die Arbovirosen,
    - durch die Rhinoviren ausgelöste Erkältung, Rhinitis und Schnupfen,
    - die durch das Maul- und Klauenseuche-Virus ausgelöste Maul- und Klauenseuche.

**3.** Verwendung nach Anspruch 1, **dadurch gekennzeichnet, dass** die retro-inversen Peptide vollständig retro-inverse Peptide oder teilweise retro-invers mit wenigstens zwei aufeinanderfolgenden "retro-inversen" Bindungen sind, und insbesondere zwei aufeinanderfolgende "retro-inverse" Bindungen haben.

**4.** Polyklonale oder monoklonale anti-retro-inverse Antikörper, wie sie durch die Immunisierung eines Tieres mit wenigstens einem retro-inversen Peptid nach Anspruch 1 erhalten werden, wobei die Antikörper einen Komplex mit wenigstens einem der oben genannten retro-inversen Peptide und mit den Ausgangspeptiden oder -proteinen, die dem letzteren entsprechen, bilden können und **dadurch gekennzeichnet sind, dass** sie das Ausgangspeptid oder das Ausgangsprotein mit einer Affinität erkennen, welche wenigstens gleich jener ist, welche im Vergleich die Antikörper gegen das Ausgangspeptid oder das Ausgangsprotein zu dem Ausgangspeptid oder dem Ausgangsprotein aufweisen.

**5.** Antigen-Antikörper-Komplex, **dadurch gekennzeichnet, dass** er aus einem der folgenden Komplexe besteht:

- Ausgangsprotein Antikörper, gerichtet gegen ein retro-inverses Peptid, das einem vom Ausgangsprotein abgeleiteten Ausgangspeptid entspricht
- Ausgangspeptid Antikörper, gerichtet gegen ein retro-inverses Peptid, das einem Ausgangspeptid entspricht
- retro-inverses Peptid, das einem Ausgangspeptid entspricht Antikörper, gerichtet gegen das Ausgangspeptid oder das Ausgangsprotein
- retro-inverses Peptid, das einem Ausgangspeptid entspricht Antikörper, gerichtet gegen ein retro-inverses Peptid, das einem Ausgangspeptid entspricht

wobei das retro-inverse Peptid wie im Anspruch 1 definiert ist und der gegen das retro-inverse Peptid gerichtete Antikörper wie im Anspruch 4 definiert ist,

die Komplexe eine Stabilität aufweisen, die wenigstens gleich jener der Komplexe ist an denen, wenn ein retro-inverses Peptid beteiligt ist, es durch ein Ausgangspeptid oder ein Ausgangsprotein ersetzt wird, und wenn ein gegen ein retro-inverses Peptid gerichteter Antikörper beteiligt ist, er durch einen Antikörper gegen das Ausgangspeptid oder gegen das Ausgangsprotein ersetzt wird.

**6.** Komplex zwischen einem retro-inversen Peptid, wie in Anspruch 1 oder Anspruch 3 definiert, und einem Bestandteil des Haupthistokompatibilitätskomplexes (auch als MHC-retro-inverses Peptid-Komplex bezeichnet) und eventuell

einem T-Zell-Rezeptor (auch als MHC-retro-inverses Peptid-T-Rezeptor-Komplex bezeichnet).

**7.** Komplex zwischen einem retro-inversen Peptid, wie in Anspruch 1 oder Anspruch 3 definiert, und einem T-Zell-Rezeptor.

**8.** *In vitro*-Diagnostikverfahren, wie in Anspruch 1 definiert, für Krankheiten, die mit der Anwesenheit eines exogenen oder endogenen Proteins im Organismus eines Patienten zusammenhängen, welches direkt oder indirekt am Prozess des Auftretens und/oder der Entwicklung der Krankheiten beteiligt sein kann, **dadurch gekennzeichnet**, das es umfasst:

- das in Kontakt bringen einer biologischen Probe eines Patienten, welcher Träger von gegen das Protein gerichteter Antikörper sein kann, mit einem retro-inversen Peptid nach Anspruch 1, wobei das retro-inverse Peptid dem endogenen oder exogenen Protein vollständig oder teilweise entspricht, oder einem Peptid entspricht, welches durch Antikörper erkannt werden kann, die selbst das exogene oder endogene Protein erkennen, oder unter Bedingungen, welche die Reaktion zwischen den gegen das Protein gerichteten Antikörpern, welche in der biologischen Proben enthalten sein können, und dem oben erwähnten retro-inversen Peptid ermöglichen;
- den *in vitro*-Nachweis des Antigen-Antikörper-Komplexes nach Anspruch 5, welcher im vorhergehenden Schritt gebildet werden kann, oder
- den *in vitro*-Nachweis von im Patienten zirkulierenden Antikörper durch Kompetitionstests unter Verwendung eines anti-retro-inversen Antikörpers.

**9.** *In vitro*-Diagnostikverfahren, wie in Anspruch 1 definiert, für Krankheiten, die mit der Anwesenheit eines exogenen oder endogenen Proteins im Organismus eines Patienten zusammenhängen, welches direkt oder indirekt am Prozess des Auftretens und/oder der Entwicklung dieser Krankheiten beteiligt sein kann, **dadurch gekennzeichnet**, das es umfasst:

- das in Kontakt bringen einer biologischen Probe eines Patienten, welcher Träger des Proteins sein kann, mit wenigstens einem der Antikörper nach Anspruch 4, wobei der Antikörper vorteilhafterweise gegen ein retro-inverses Peptid gerichtet sind, welches aus einem retro-inversen Peptid besteht, das dem endogenen oder exogenen Protein vollständig oder teilweise entspricht, oder unter Bedingungen, welche die Reaktion zwischen dem Protein, welches in der biologischen Probe enthalten sein kann, und den vorher erwähnten, gegen das retro-inverse Peptid gerichteten Antikörpern ermöglichen;
- den *in vitro*-Nachweis des Antigen-Antikörper-Komplexes nach Anspruch 5, welcher im vorhergehenden Schritt gebildet werden kann, oder
- den Nachweis von im Patienten zirkulierenden Antigenen durch Kompetitionstests unter Verwendung eines retro-inversen Peptids nach Anspruch 1.

**10.** *In vitro*-Diagnostikverfahren nach Anspruch 8, wobei die Krankheiten nach Anspruch 2 ausgewählt sind.

**11.** *In vitro*-Diagnostikverfahren nach Anspruch 9, wobei die Krankheiten nach Anspruch 2 ausgewählt sind.

**12.** Kit zur Durchführung der diagnostischen *in vitro*-Verfahren nach einem der Ansprüche 8 bis 11 mit:

- einem retro-inversen Peptid nach Anspruch 1, welches vollständig oder teilweise dem endogenen oder exogenen Protein entspricht, oder einem Peptid entspricht, welches durch Antikörper erkannt werden kann, die wiederum das exogene oder endogene Protein erkennen, oder auch gegen das retro-inverse Peptid gerichtete Antikörper nach Anspruch 4;
- Mittel zum Anpassen einer Umgebung für die Durchführung einer immunologischen Reaktion;
- Mittel für den Nachweis des Antigen-Antikörper-Komplexes nach Anspruch 5, welcher aufgrund der immunologischen Reaktion gebildet wurde, wobei diese Mittel eventuell eine Markierung enthalten oder nacheinander durch ein markiertes Mittel erkannt werden können, insbesondere in dem Fall, dass das retro-inverse Peptid oder die oben erwähnten anti-retro-inversen Antikörper nicht markiert sind.

**13.** Verwendung wenigstens eines retro-inversen Peptids nach Anspruch 1, für die Herstellung eines Medikaments, welches für die Vorbeugung oder Behandlung von Krankheiten, die mit der Anwesenheit eines exogenen oder endogenen Protein im Organismus einer Person zusammenhängen, welches direkt oder indirekt am Prozess des Auftretens und/oder der Entwicklung dieser Krankheit beteiligt sein kann.

**14.** Verwendung wenigstens eines retro-inversen Peptids nach Anspruch 1, für die Herstellung einer Vakzine im Rahmen der Vorbeugung von Krankheiten, die mit der Anwesenheit eines exogenen oder endogenen Proteins im Organismus einer Person zusammenhängen, welches durch die gegen die retro-inversen Peptide gerichteten Antikörper erkannt werden kann.

**15.** Pharmazeutische Zusammensetzung, **dadurch gekennzeichnet, dass** ein retro-inverses Peptid nach Anspruch 1 mit einem Protein-tragendem Molekül zusammenhängt oder nicht, wobei es *in vivo* die Produktion von Antikörpern induzieren kann, welche das für die Krankheit verantwortliche exogene oder endogene Protein neutralisieren, oder *in vivo* eine zelluläre zytotoxische Immunantwort induzieren kann.

**16.** Pharmazeutische Zusammensetzung, **dadurch gekennzeichnet, dass** sie Antikörper nach Anspruch 4 in Verbindung mit einem physiologisch akzeptablen Träger umfasst.

PEPTIDE L

A

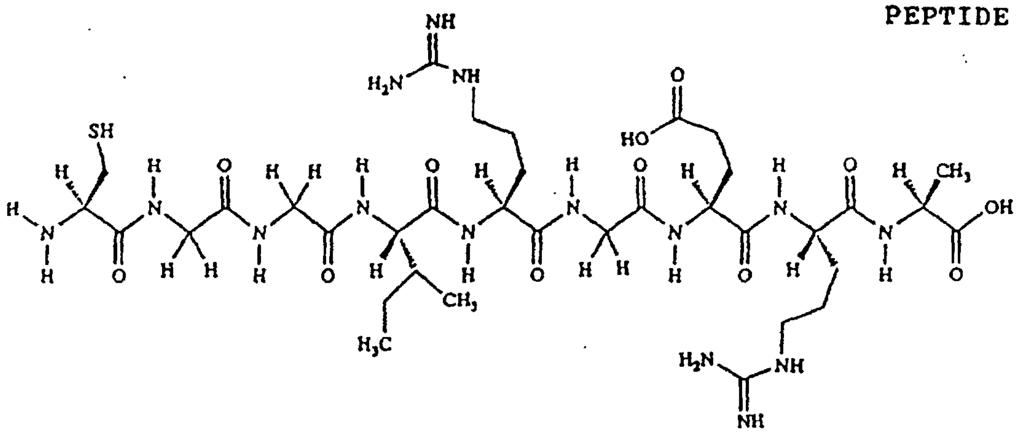

B

PEPTIDE
RETRO-INVERSO

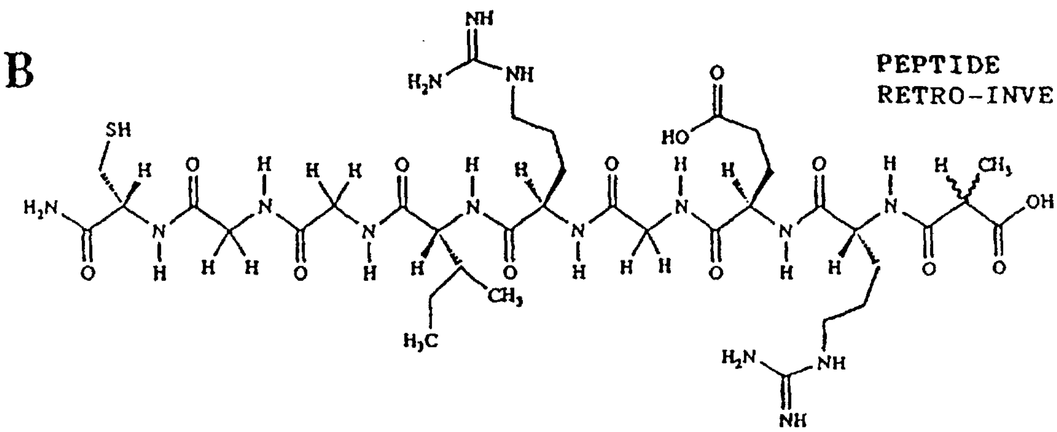

Figure 1

C

PEPTIDE D

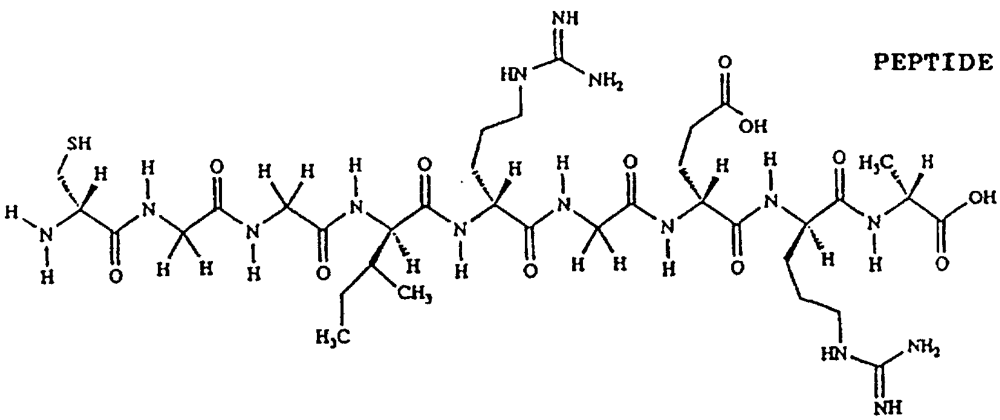

D

PEPTIDE RETRO

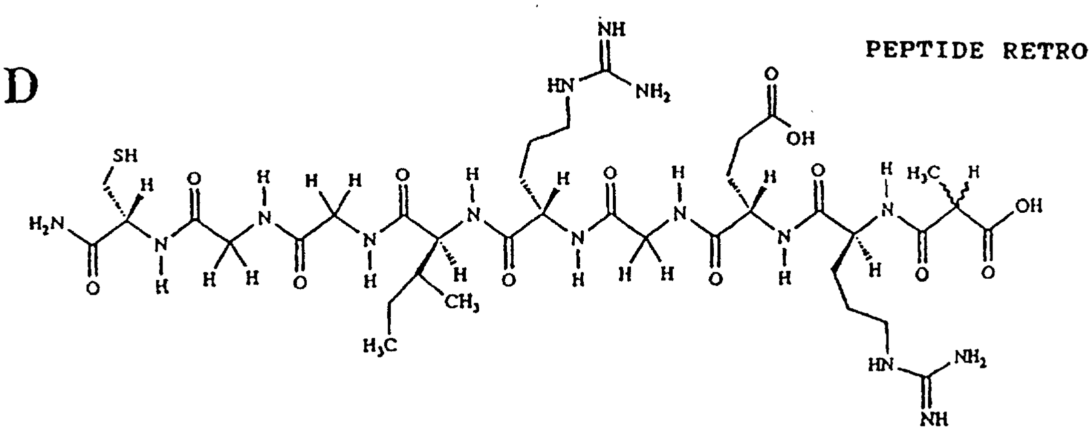

Figure 1 suite

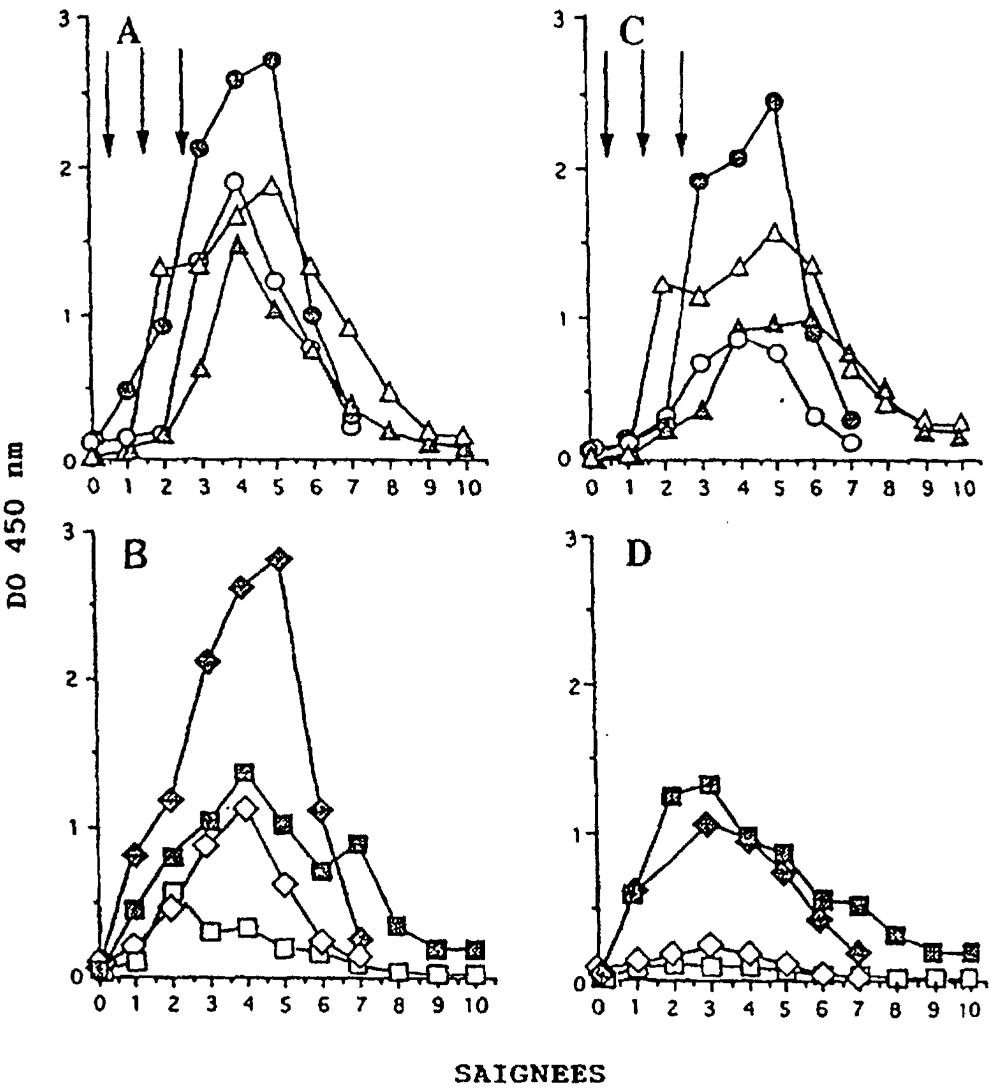


Figure 2

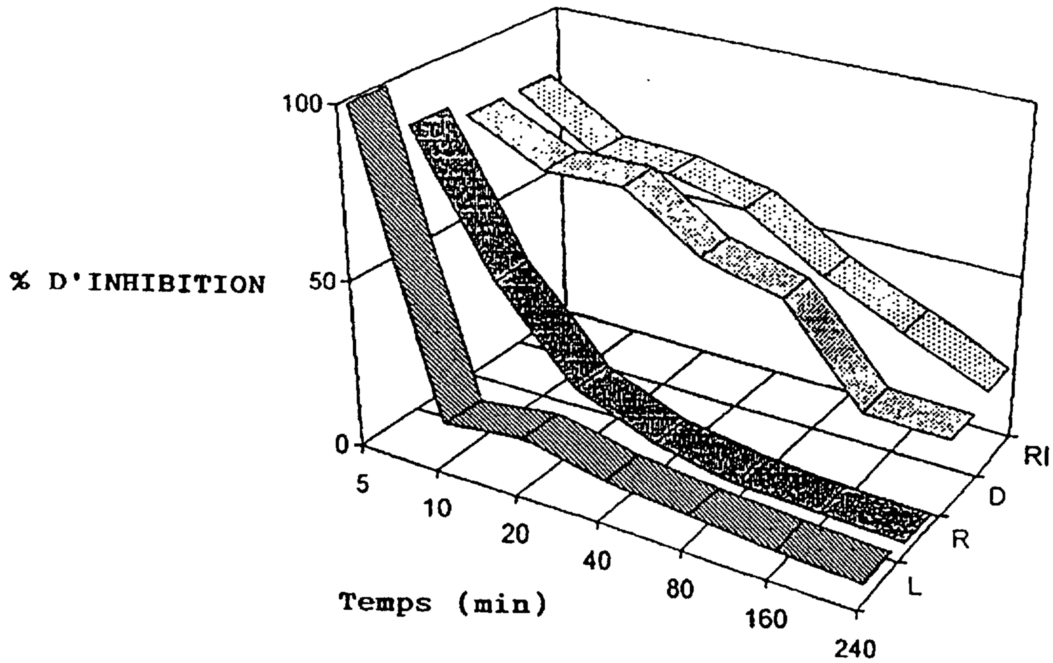


Figure 3

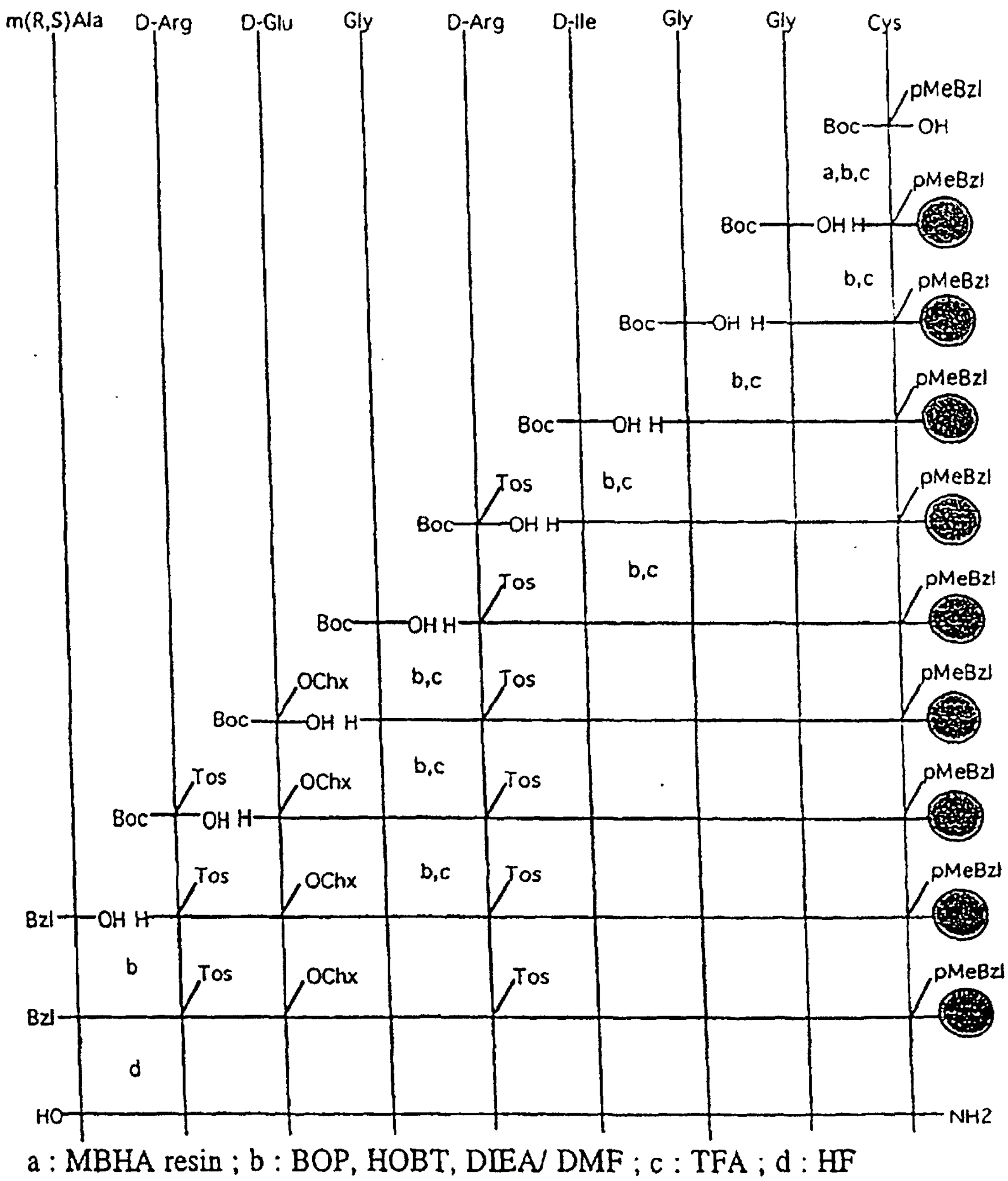


a : MBHA resin ; b : BOP, HOBT, DIEA/ DMF ; c : TFA ; d : HF

**Figure 4**

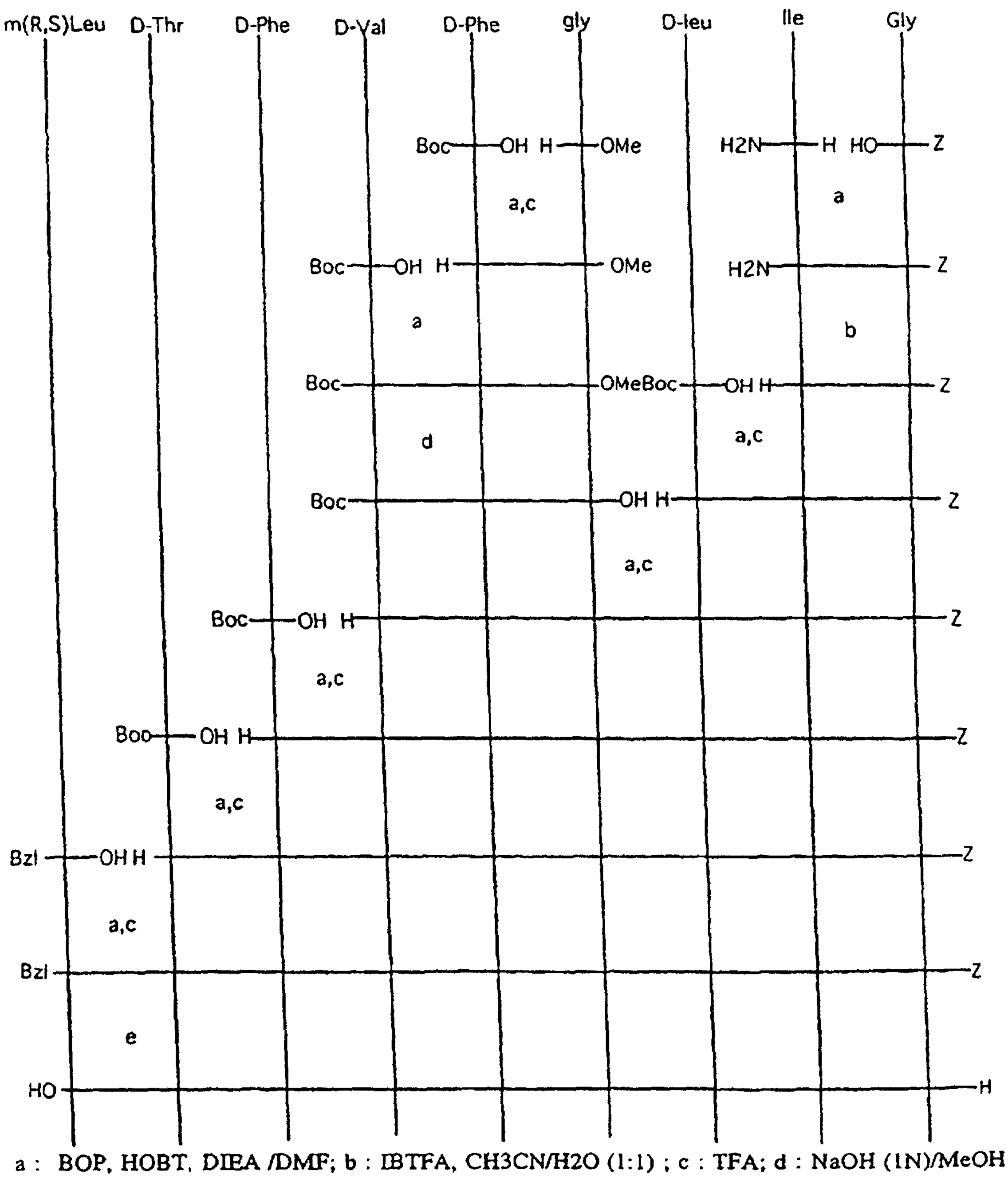


a : BOP, HOBT, DIEA /DMF; b : IBTFA, CH3CN/H2O (1:1) ; c : TFA; d : NaOH (1N)/MeOH ; e : H2, Pd/C.

**Figure 5**

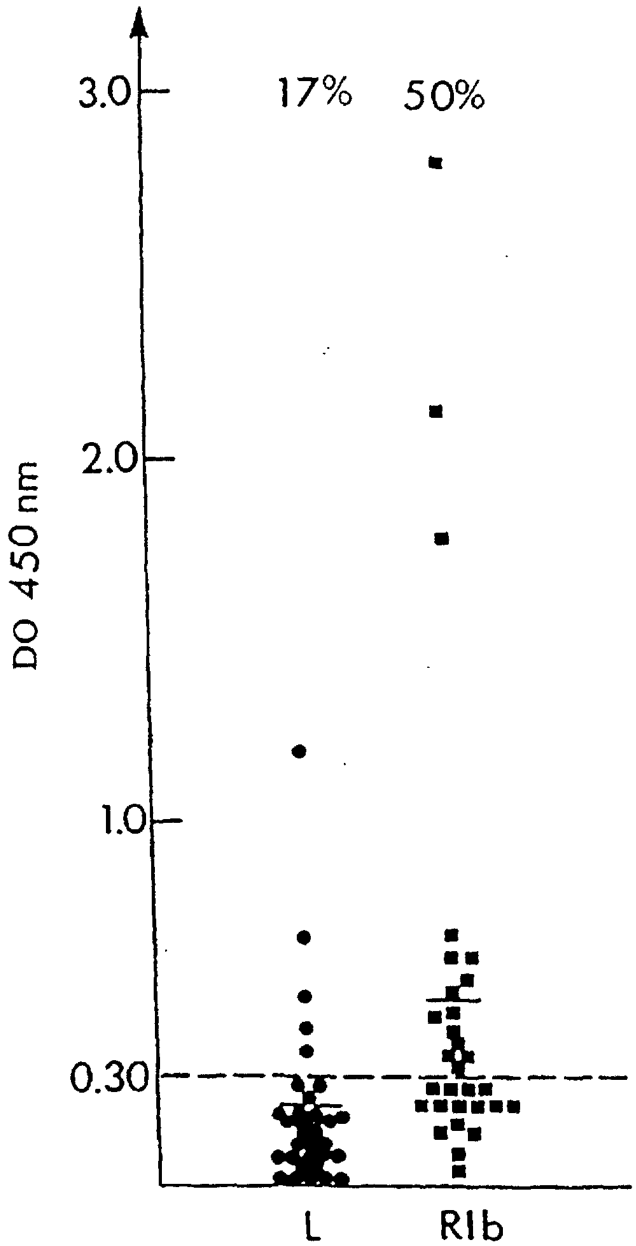


Analogues 277-291 de Ro52

**Figure 6**

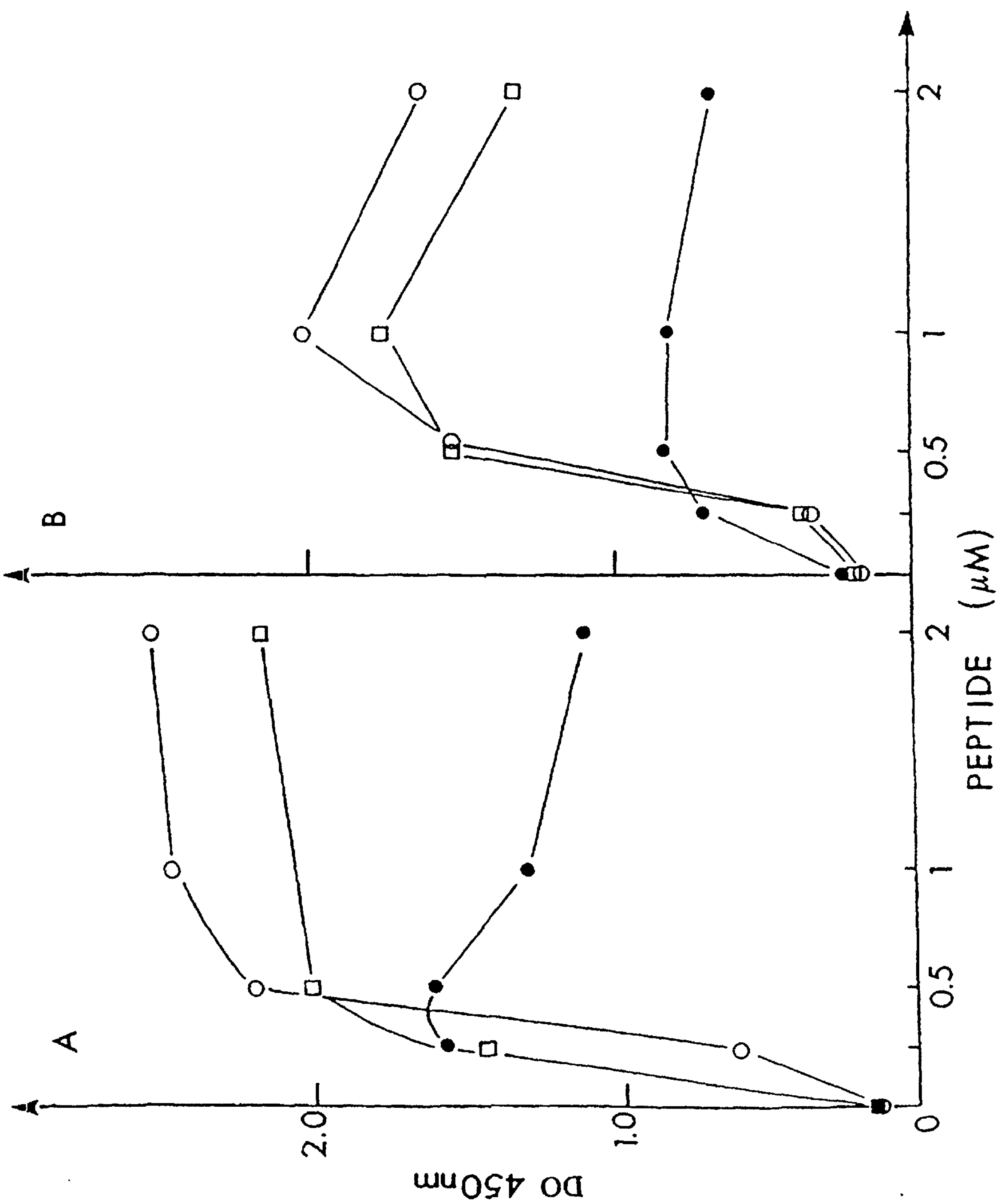


Figure 7

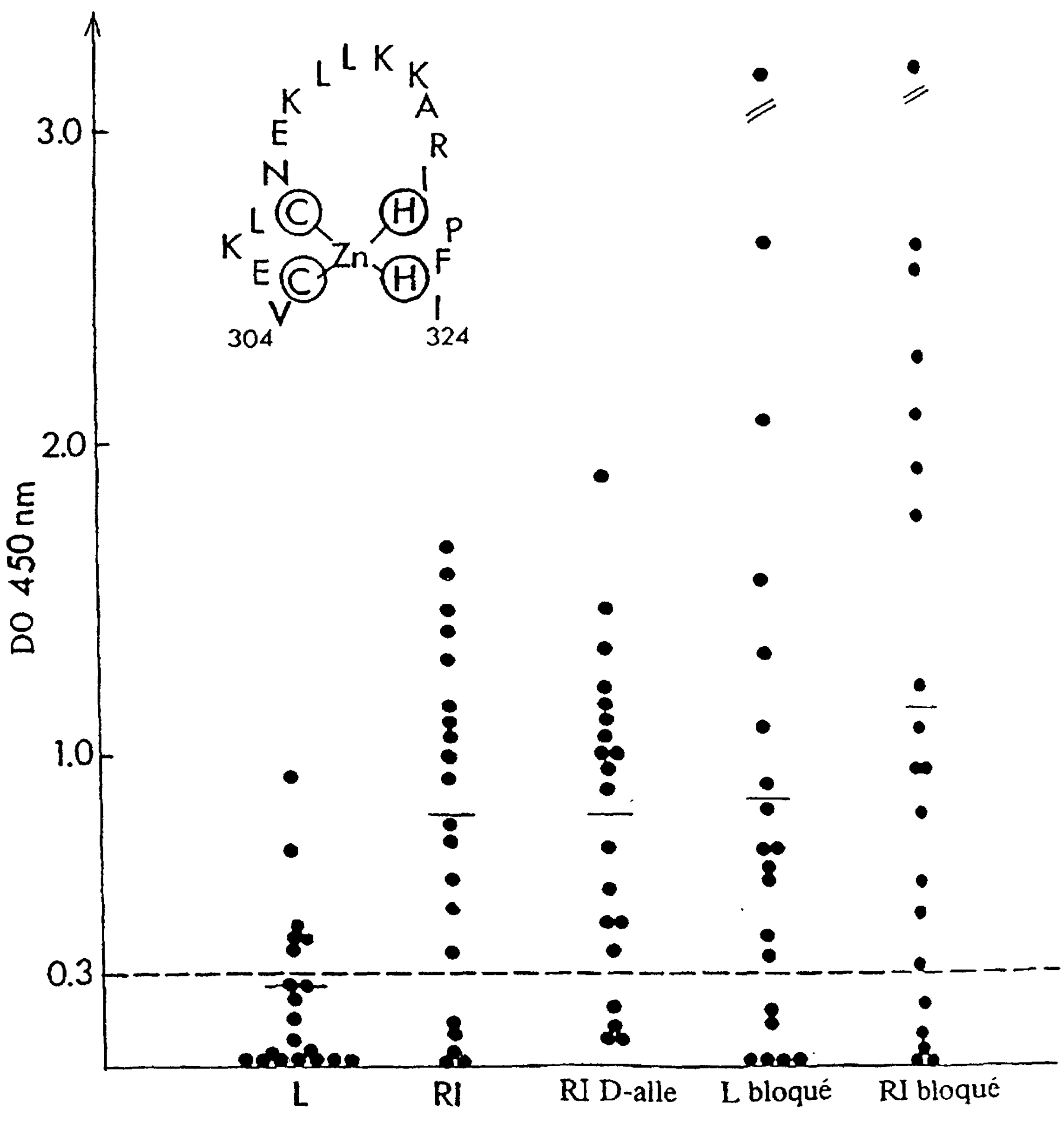
3.0
2.0
1.0
0.3
DO 450 nm
K
L
L
K
K
E
A
R
N
I
L
C
H
P
K
Zn
E
C
H
F
V
I
304
324
L
RI
RI D-alle
L bloqué
RI bloqué

Figure 8

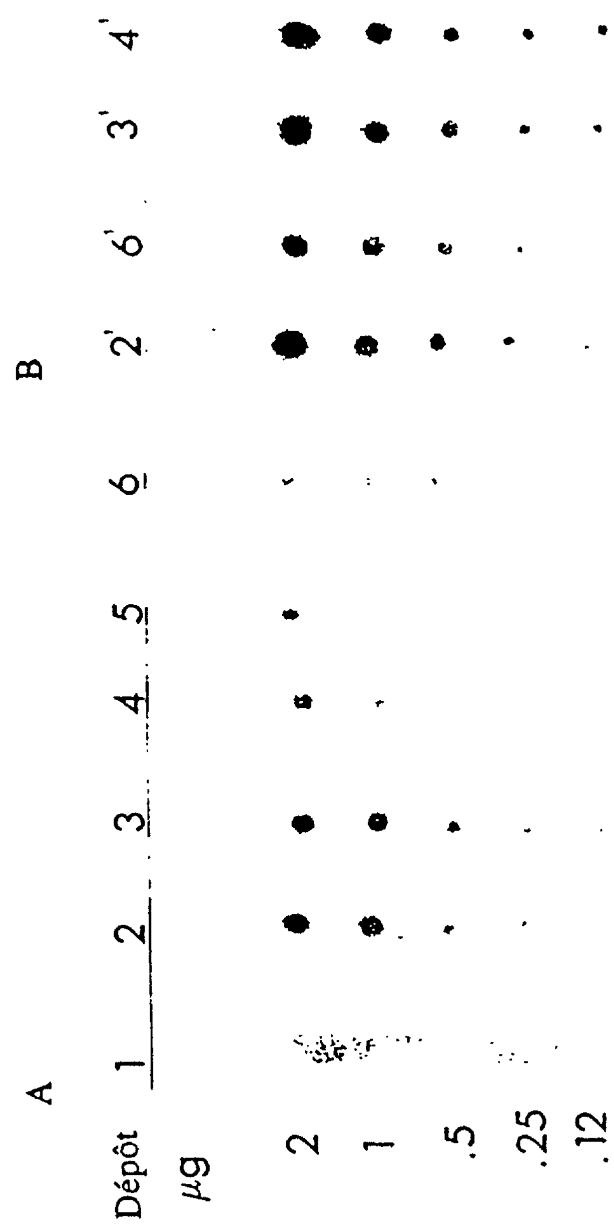


Figure 9

H-Gly-Ile-Leu-Gly-Phe-Val-Phe-Thr-Leu-OH — M 58-66

H-Gly-Leu$^{59}$-Leu-Gly-Phe-Val-Phe-Thr-Leu-OH — [Leu$^{59}$] M 58-66

H-Gly-Ile-Leu-Gly-Phe-Val-Phe-Ala$^{65}$-Leu-OH — [Ala$^{65}$] M 58-66

(GG0 a)
(GG0 b)

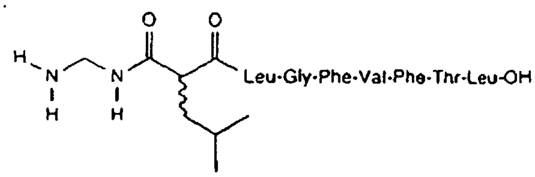

[gGly$^{58}$-mLeu$^{59}$] M58-66

(GG1 a)
(GG1 b)

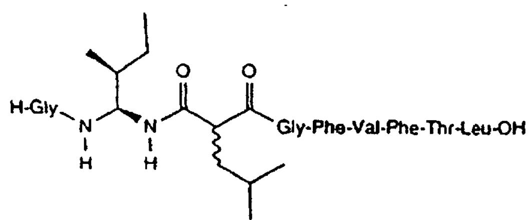

[gIle$^{59}$-mLeu$^{60}$] M58-66

(GG5)

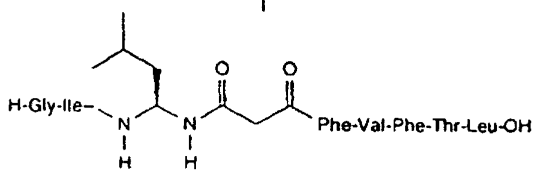

[gLeu$^{60}$-mGly$^{61}$] M58-66

(GG7)

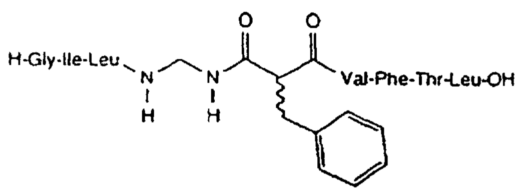

[gGly$^{61}$-mPhe$^{62}$] M58-66

**Figure 10**

(GG8 a)
(GG8 ab)

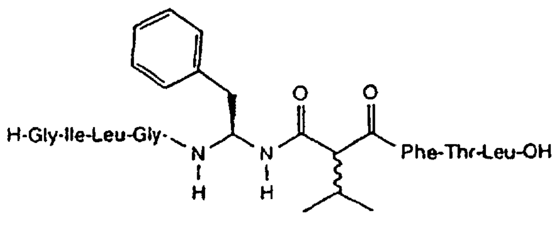

[gPhe$^{62}$-mVal$^{63}$] M58-66

(GG10)

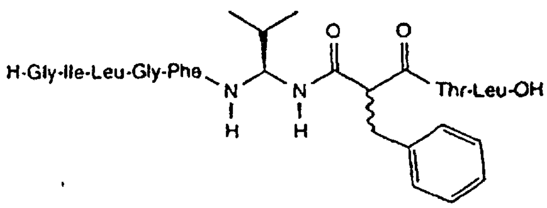

[gVal$^{63}$-mPhe$^{64}$] M58-66

(GG11)

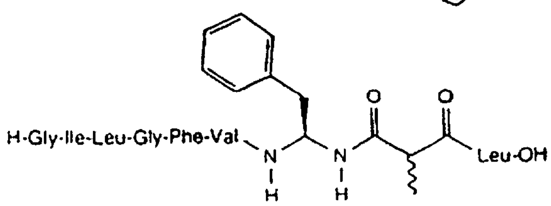

[gPhe$^{64}$-mAla$^{65}$] M58-66

(GG12)

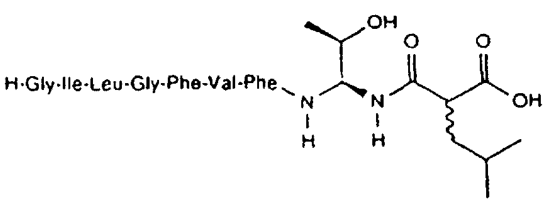

[gThr$^{65}$-mLeu$^{66}$] M58-66

**Figure 10 suite**

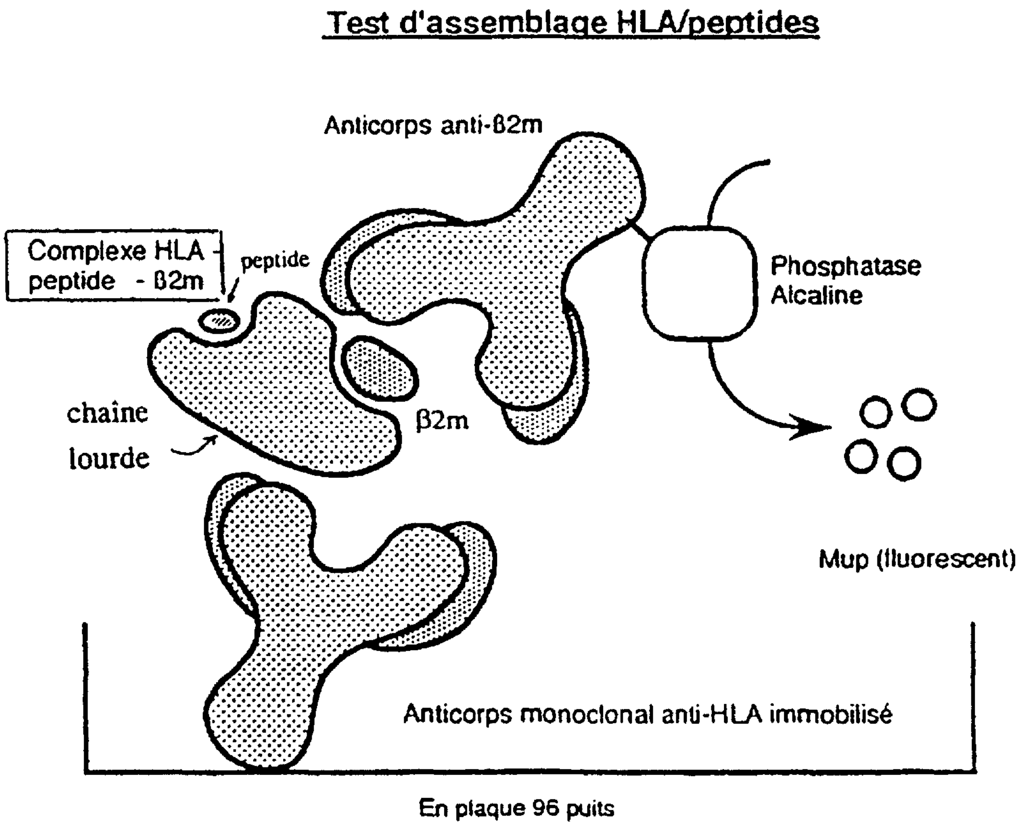
Test d'assemblage HLA/peptides
Anticorps anti-ß2m
Complexe HLA - peptide - ß2m
peptide
Phosphatase Alcaline
chaîne lourde
ß2m
Mup (fluorescent)
Anticorps monoclonal anti-HLA immobilisé
En plaque 96 puits

Figure 11

Fixation sur le HLA-A2 (lysat de cellules T2)

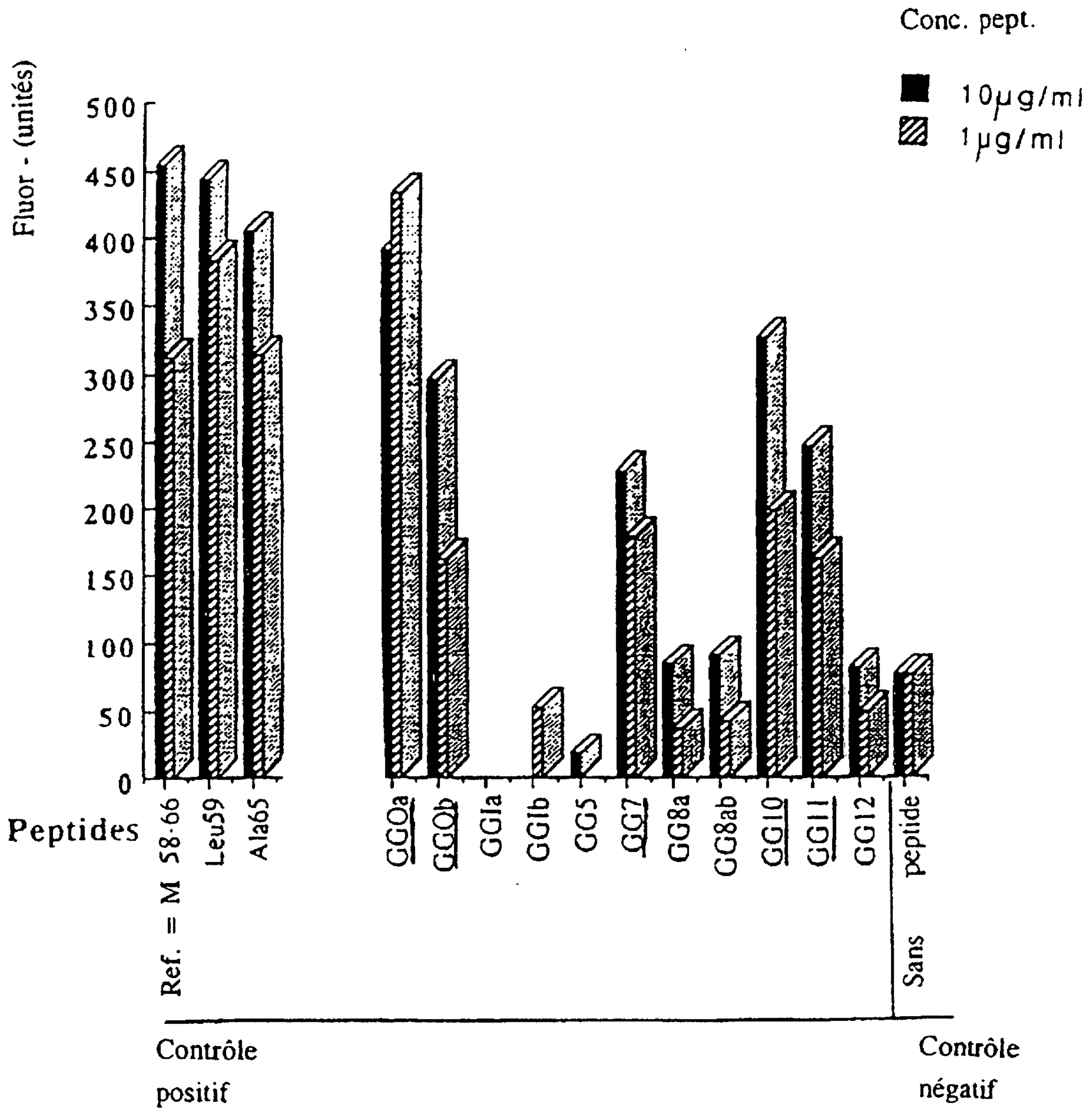


Figure 12

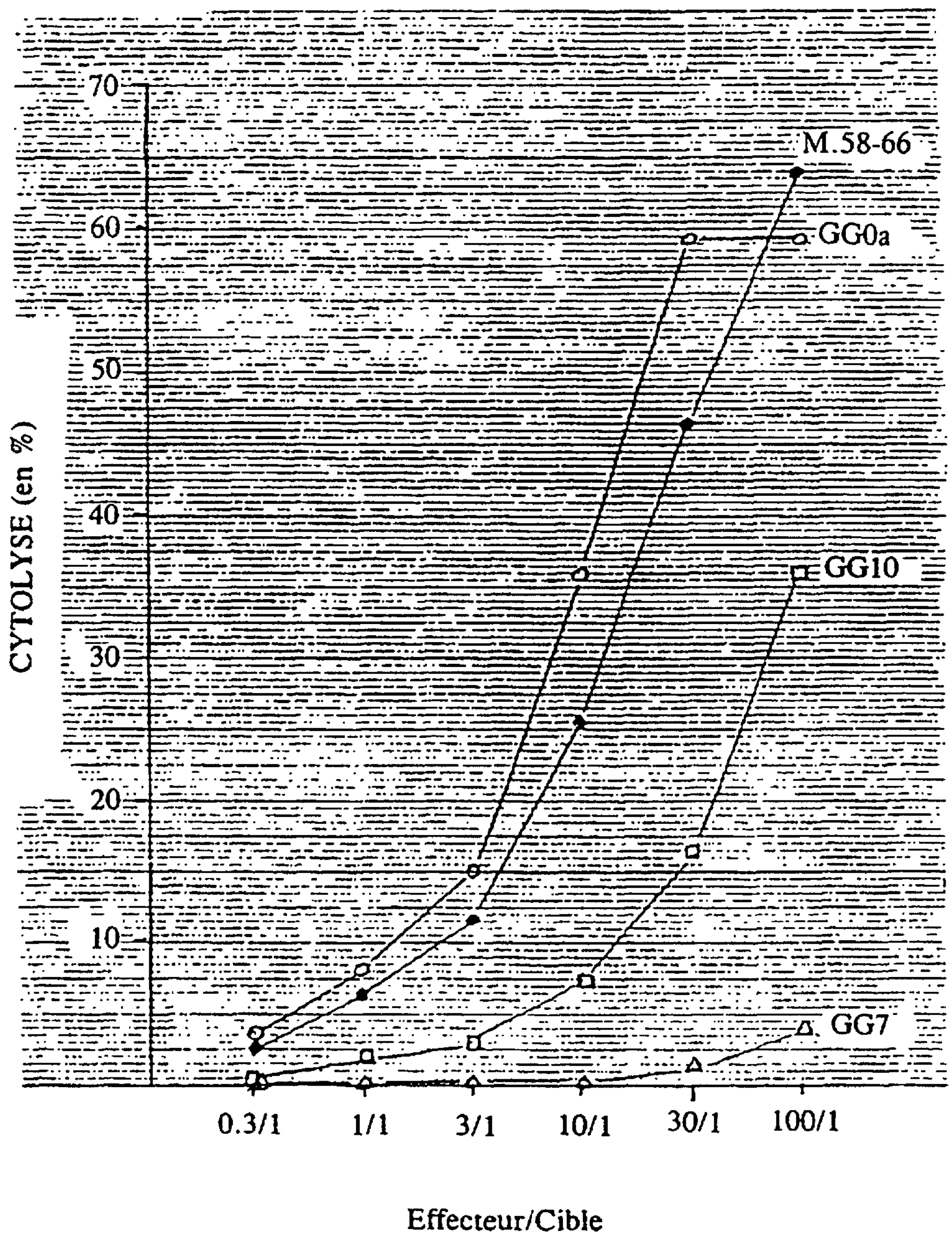
70
60
50
40
30
20
10
CYTOLYSE (en %)
M.58-66
GG0a
GG10
GG7
0.3/1
1/1
3/1
10/1
30/1
100/1
Effecteur/Cible

**Figure 13**